(19) Europäisches Patentamt / European Patent Office / Office européen des brevets

(11) **EP 2 243 790 A1**

(12) **EUROPEAN PATENT APPLICATION**

(43) Date of publication:
**27.10.2010 Bulletin 2010/43**

(51) Int Cl.:
***C07K 14/395*** *(2006.01)*

(21) Application number: **09161944.5**

(22) Date of filing: **04.06.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **24.04.2009 KR 20090036253**

(71) Applicant: **Samsung Electronics Co., Ltd.**
**Suwon-si, Gyeonggi-do 442-743 (KR)**

(72) Inventors:
- **Yu, Byung Jo**
  **Gyeonggi-do (KR)**
- **Park, Jae Chan**
  **Gyeonggi-do (KR)**
- **Park, Min Park**
  **Gyeonggi-do (KR)**
- **Kweon, Dae Hyoek**
  **Gyeonggi-do (KR)**
- **Hong, Min Eui**
  **Gyeonggi-do (KR)**

(74) Representative: **Grünecker, Kinkeldey,**
**Stockmair & Schwanhäusser**
**Anwaltssozietät**
**Leopoldstrasse 4**
**80802 München (DE)**

(54) **Isolated polynucleotide increasing alcohol tolerance of host cell, vector and host cell containing the same, and method of producing alcohol using the same**

(57) Provided are an isolated polynucleotide increasing alcohol tolerance of a host cell, a vector and a host cell which contain the same, and a method of producing bioalcohol using the same. When an isolated polynucleotide is overexpressed in a host cell, alcohol tolerance of the host cell is increased, resulting in an increase in alcohol volumetric productivity in fermentation.

FIG. 1

MSN2, Chromosome XIII, 343143-346374

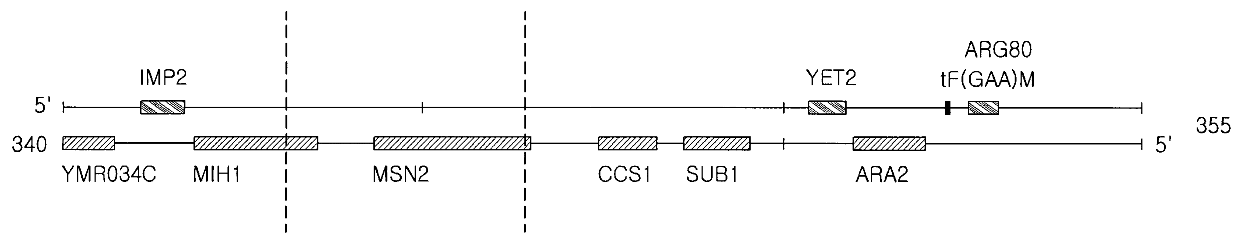


EP 2 243 790 A1

<110> Samsung Electronics Co., LTD.

<120> Isolated Polynucleotide Increasing Alcohol Tolerance of Host Cell, Vector and Host Cell Containing the same, and Method of Producing Alcohol using the same

<130> EP65499FZpau

<140> not yet assigned
<141> 2009-06-03

<150> KR 10-2009-036253
<151> 2009-04-24

<160> 31

<170> KopatentIn 1.71

<210> 1
<211> 377
<212> DNA
<213> Artificial Sequence

<220>
<223> truncated MIH1

<400> 1
atgaacaata tatttcatgg aactgaagat gaatgtgcca atgaagacgt tcttagtttc 60

caaaaaattt ccttgaaaag tccctttggt aagaagaaga acatatttag aaatgttcag 120

accttcttta agtcaaaaag caaacattcg aatgtcgacg atgatttaat caataaagag 180

aatcttgcct ttgataaatc tccattgtta acaaatcaca ggagtaagga aattgatggt 240

ccttcaccga atataaagca gcttggccat cgcgatgagt tagatgaaaa tgaaaatgaa 300

aatgatgata tagtcttaag catgcatttt gcttctcaaa ccttacaaag tccaacaaga 360

aactcatcaa gaagatc 377

<210> 2
<211> 1602
<212> DNA
<213> Artificial Sequence

<220>
<223> INO1

<400> 2
atgacagaag ataatattgc tccaatcacc tccgttaaag tagttaccga caagtgcacg 60

tacaaggaca acgagctgct caccaagtac agctacgaaa atgctgtagt tacgaagaca 120

gctagtggcc gcttcgatgt aacgcccact gttcaagact acgtgttcaa acttgacttg 180

aaaaagccgg aaaaactagg aattatgctc attgggttag gtggcaacaa tggctccact 240

ttagtggcct cggtattggc gaataagcac aatgtggagt ttcaaactaa ggaaggcgtt 300

aagcaaccaa actacttcgg ctccatgact caatgttcta ccttgaaact gggtatcgat 360

gcggaggggga atgacgttta tgctcctttt aactctctgt tgcccatggt tagcccaaac 420

gactttgtcg tctctggttg ggacatcaat aacgcagatc tatacgaagc tatgcagaga 480

agtcaagttc tcgaatatga tctgcaacaa cgcttgaagg cgaagatgtc cttggtgaag 540
cctcttcctt ccatttacta ccctgatttc attgcagcta atcaagatga gagagccaat 600
aactgcatca atttggatga aaaaggcaac gtaaccacga ggggtaagtg gacccatctg 660
caacgcatca gacgcgatat ccagaatttc aaagaagaaa acgcccttga taaagtaatc 720
gttctttgga ctgcaaatac tgagaggtac gtagaagtat ctcctggtgt taatgacacc 780
atggaaaacc tcttgcagtc tattaagaat gaccatgaag agattgctcc ttccacgatc 840
tttgcagcag catctatctt ggaaggtgtc ccctatatta atggttcacc gcagaatact 900
tttgttcccg gcttggttca gctggctgag catgagggta cattcattgc gggagacgat 960
ctcaagtcgg gacaaaccaa gttgaagtct gttctggccc agttcttagt ggatgcaggt 1020
attaaaccgg tctccattgc atcctataac catttaggca ataatgacgg ttataactta 1080
tctgctccaa aacaatttag gtctaaggag atttccaaaa gttctgtcat agatgacatc 1140
atcgcgtcta atgatatctt gtacaatgat aaactgggta aaaaagttga ccactgcatt 1200
gtcatcaaat atatgaagcc cgtcggggac tcaaaagtgg caatggacga gtattacagt 1260
gagttgatgt taggtggcca taaccggatt tccattcaca atgtttgcga agattcttta 1320
ctggctacgc ccttgatcat cgatctttta gtcatgactg agttttgtac aagagtgtcc 1380
tataagaagg tggacccagt taaagaagat gctggcaaat tcgagaactt ttatccagtt 1440
ttaaccttct tgagttactg gttaaaagct ccattaacaa gaccaggatt tcacccggtg 1500
aatggcttaa acaagcaaag aaccgcctta gaaaattttt taagattgtt gattggattg 1560
ccttctcaaa acgaactaag attcgaagag agattgttgt aa 1602

<210> 3
<211> 741
<212> DNA
<213> Artificial Sequence

<220>
<223> DOG1

<400> 3
atggcagaat tttcagctga tctatgtctt tttgacctag atggtaccat agtgagtaca 60
acagtggccg cagagaaagc atggaccaag ttgtgttacg aatacggtgt tgatccttcc 120
gagttattta agcattctca tggtgcaaga acacaagagg ttttgagaag gtttttccct 180
aaattggatg atacagacaa taaaggtgtt cttgctctag aaaaagatat tgcccatagt 240
tacttggaca cagtaagcct tattcctggt gcagagaact tactgttatc gttagatgta 300
gatactgaga ctcaaaaaaa gttacctgaa aggaaatggg ctatcgttac ctctggttct 360
ccatatttgg cattttcatg gttcgagaca atattgaaaa atgttggaaa gcccaaagtt 420
ttcattactg ggtttgacgt gaagaacggt aagcctgatc ccgagggtta ttcaagagct 480
cgtgatttat tgcgtcaaga tttgcaatta actggtaaac aggatctgaa gtatgttgtc 540

ttcgaagatg cacccgtggg cataaaggcc ggcaaagcaa tgggcgccat tactgtgggt 600

ataacatcct cgtatgacaa gagcgtttta tttgacgcag gagcagatta tgtagtctgt 660

gatttgacac aggtttccgt ggttaagaac aatgaaaacg gtattgtcat ccaggtaaac 720

aaccctttga caagggcctg a 741

<210> 4
<211> 885
<212> DNA
<213> Artificial Sequence

<220>
<223> HAL1

<400> 4
atgcatttca aagatttagg attgcatgac tacactctca aaaacttgat gtatgagaat 60

aattgctgta aattttatga tgccgtggat gaaaacaaca tctcatatgt tttaaaattt 120

gttccctcag atgtgacttc ggaaggggat actttcccat tcgtggatcg ctttcaagta 180

aaggaaggtg tttttttggt atattcctca aatgactttg gaaaagaagg tacggactac 240

tttacttata ctggtagtgg tggaaatgag gttcacatct cgggcacctc ttcagaagca 300

ggaataaaac cgcagtttat tgaaacttgc catccaaaac atcttaagcg gggaacaaaa 360

gagcaggaag atataaatag tagtacctca aagaaaagtg cagttatcaa caatttttcg 420

ggtgaaaaaa caccaaatcc aaggccacag agttccaaca tttcagaaag agagacgtat 480

gtcggaatat tgaacgtcaa atgtaaaaat aagaactcat cgaaaatacg aagtgaaaaa 540

ttggtaagct ccgtcatcga aacaaagcat acgccaggat tggcatctat tttatcgaaa 600

gaaggcacta catatccgaa taatgcggac gggaaacata tcagtatcgt gaatccatcc 660

tcaaaaatat atcattcatc ccataaacag attgttaaaa cgcctatccc taagagtggc 720

ctttctccaa ttgagagatg ccctttcaat ggtcaaaata ttaaatgcta ctcaccaaga 780

ccactagatc atgaaagtcc ccaacgtgat ttcaataata actttcagct gagaatactg 840

aagagctcgg tgttgcaaag gagacaatca acacagaata gttga 885

<210> 5
<211> 668
<212> DNA
<213> Artificial Sequence

<220>
<223> TRP1

<400> 5
atgtctgtta ttaatttcac aggtagttct ggtccattgg tgaaagtttg cggcttgcag 60

agcacagagg ccgcagaatg tgctctagat tccgatgctg acttgctggg tattatatgt 120

gtgcccaata gaaagagaac aattgacccg gttattgcaa ggaaaatttc aagtcttgta 180

aaagcatata aaaatagttc aggcactccg aaatacttgg ttggcgtgtt tcgtaatcaa 240

cctaaggagg atgttttggc tctggtcaat gattacggca ttgatatcgt ccaactgcat 300

ggagatgagt cgtggcaaga ataccaagag ttcctcggtt tgccagttat taaaagactc 360

gtatttccaa aagactgcaa catactactc agtgcagctt cacagaaacc tcattcgttt 420

attcccttgt ttgattcaga agcaggtggg acaggtgaac ttttggattg gaactcgatt 480

tctgactggg ttggaaggca agagagcccc gaaagcttac attttatgtt agctggtgga 540

ctgacgccag aaaatgttgg tgatgcgctt agattaaatg gcgttattgg tgttgatgta 600

agcggaggtg tggagacaaa tggtgtaaaa gactctaaca aaatagcaaa tttcgtcaaa 660

aatgctaa 668

<210> 6
<211> 790
<212> DNA
<213> Artificial Sequence

<220>
<223> truncated MRPL17

<400> 6
atgaaggtaa atttaatgtt gaaaagaggg cttgctactg caactgcaac tgccagttcc 60

gctcccccca agattaaagt cggagtacta ctgtcaagaa tccctataat taaatcagaa 120

ttaaatgaac tagagaaaaa atactatgag taccaatcag aactagaaaa gagactaatg 180

tggacgtttc cggcatattt ttatttcaaa aagggtactg tagcagaaca caaatttcta 240

tccctgcaga aaggacctat ctccaaaaaa aatggcattt ggtttcctag aggcataccg 300

gacattaaac atggcagaga aagaagtact aagcaagaag ttaaactttc tgatgacagt 360

acagtagcat ttagcaacaa tcaaaaagag caaagcaaag acgatgttaa taggcccgtg 420

attcccaacg acaggataac ggaagcagat aggtcaaatg atatgaagag ccttgaaaga 480

caattgagca ggaccttata tctttttggtt aaggataaaa gcggtacttg gaaattccct 540

aacttcgatc tttctgatga atctaagccg ttacacgtac acgcagagaa cgaattgaaa 600

ttgttgagcg gtgatcagat atacacttgg tctgtttctg ctacgcccat aggtgttttg 660

caggacgaga gaaataggac tgctgagttt attgtgaagt cacacatttt ggctggaaaa 720

tttgatttgg tggcgtcgaa aaatgatgca ttcgaggatt ttgcttggct gacaaaaggt 780

gagatcagtg 790

<210> 7
<211> 1468
<212> DNA
<213> Artificial Sequence

<220>
<223> Partial fragment YLR157C-B

<400> 7
attacaggag aaatctgagt gatgagaaga atgattctcg cagctatacg aatacaacca 60

aacccaaagt tatagctcgg aatcctcaaa aaacaaataa ttcgaaatcg aaaacagcca 120
gggctcacaa tgtatccaca tctaataact ctcccagcac ggacaacgat tccatcagta 180
aatcaactac tgaaccgatt caattgaaca ataagcacga ccttcacctt aggccaggaa 240
cttactgaat ctacggtaaa tcacactaat cattctgatg atgaactccc tggacacctc 300
cttctcgatt caggagcatc acgaaccctt ataagatctg ctcatcacat acactcagca 360
tcatctaatc ctgacataaa cgtagttgat gctcaaaaaa gaaatatacc aattaacgct 420
attggtgacc tacaatttca cttccaggac aacaccaaaa catcaataaa ggtattgcac 480
actcctaaca tagcctatga cttactcagt ttgaatgaat tggctgcagt agatatcaca 540
gcatgcttta ccaaaaacgt cttagaacga tctgacggca ctgtacttgc acctatcgta 600
aaatatggag acttttactg ggtatctaaa aagtacttgc ttccatcaaa tatctccgta 660
cccaccatca ataatgtcca tacaagtgaa agtacacgca aatatcctta tcctttcatt 720
catcgaatgc ttgcgcatgc caatgcaccg acaattcgat actcacttaa aaataacacc 780
atcacgtatt ttaacgaatc agatgtcgac tggtctagtg ctattgacta tcaatgtcct 840
gattgtttaa tcggcaaaag caccaaacac agacatatca aaggttcacg actaaaatac 900
caaaattcat acgaaccctt tcaataccta catactgaca tatttggtcc agttcacaac 960
ctaccaaata gtgcaccatc ctatttcatc tcatttactg atgagacaac aaaattccgt 1020
tgggtttatc cattacacga ccgtcgcgag gactctatcc tcgatgtttt tactacgata 1080
ctagctttta ttaagaacca gtttcaggcc agtgtcttgg ttatacaaat ggaccgtggt 1140
tctgagtata ctaacagaac tctccataaa ttccttgaaa aaaatggtat aactccatgc 1200
tatacaacca cagcggattc ccgagcacat ggagtcgctg aacggctcaa ccgtacctta 1260
ttagatgact gccgtactca actgcaatgt agtggtttac cgaaccattt atggttctct 1320
gcaatcgaat tttctactat tgtgagaaat tcactagctt cacctaaaag caaaaaatct 1380
gcaagacaac atgctggctt ggcaggactt gatatcagta ctttgttacc tttcggtcaa 1440
cctgttatcg tcaatgatca caacccta 1468

<210> 8
<211> 179
<212> DNA
<213> Artificial Sequence

<220>
<223> Putative SPG5 promoter

<400> 8
ccgtatatca gcttttagat caggctcgag tttcttgtta tatgtgcatt gcaaaagcat 60
aaacaaatcc tggcagccga agccgggcaa tccacttcga aacgcacggc tgaactatat 120
aaatataaag gacatgtgga gagaagcttc tcttccttca catttcgcat ttcatgatc 179

<210> 9
<211> 1943

<212> DNA
<213> Artificial Sequence

<220>
<223> truncated MSN2

<400> 9
aaaatcaaga aacttcactg aatttggggc ttcctccact atctttcgac tctccactgc 60
ccgtaacgga aacgatacca tccactaccg ataacagctt gcatttgaaa gctgatagca 120
acaaaaatcg cgatgcaaga actattgaaa atgatagtga aattaagagt actaataatg 180
ctagtggctc tggggcaaat caatacacaa ctcttacttc accttatcct atgaacgaca 240
ttttgtacaa catgaacaat ccgttacaat caccgtcacc ttcatcggta cctcaaaatc 300
cgactataaa tcctcccata aatacagcaa gtaacgaaac taatttatcg cctcaaactt 360
caaatggtaa tgaaactctt atatctcctc gagcccaaca acatacgtcc attaaagata 420
atcgtctgtc cttacctaat ggtgctaatt cgaatctttt cattgacact aacccaaaca 480
atttgaacga aaaactaaga aatcaattga actcagatac aaattcatat tctaactcca 540
tttctaattc aaactccaat tctacgggta atttaaattc cagttatttt aattcactga 600
acatagactc catgctagat gattacgttt ctagtgatct cttattgaat gatgatgatg 660
atgacactaa tttatcacgc cgaagattta gcgacgttat aacaaaccaa tttccgtcaa 720
tgacaaattc gaggaattct atttctcact ctttggacct ttggaaccat ccgaaaatta 780
atccaagcaa tagaaataca aatctcaata tcactactaa ttctacctca agttccaatg 840
caagtccgaa taccactact atgaacgcaa atgcagactc aaatattgct ggcaacccga 900
aaaacaatga cgctaccata gacaatgagt tgacacagat tcttaacgaa tataatatga 960
acttcaacga taatttgggc acatccactt ctggcaagaa caaatctgct tgcccaagtt 1020
cttttgatgc caatgctatg acaaagataa atccaagtca gcaattacag caacagctaa 1080
accgagttca acacaagcag ctcacctcgt cacataataa cagtagcact aacatgaaat 1140
ccttcaacag cgatctttat tcaagaaggc aaagagcttc tttacccata atcgatgatt 1200
cactaagcta cgacctggtt aataagcagg atgaagaccc caagaacgat atgctgccga 1260
attcaaattt gagttcatct caacaattta tcaaaccgtc tatgattctt tcagacaatg 1320
cgtccgttat tgcgaaagtg gcgactacag gcttgagtaa tgatatgcca tttttgacag 1380
aggaaggtga acaaaatgct aattctactc caaatttcga tctttccatc actcaaatga 1440
atatggctcc attatcgcct gcatcatcat cctccacgtc tcttgcaaca aatcatttct 1500
atcaccattt cccacagcag ggtcaccata ccatgaactc taaaatcggt tcttcccttc 1560
ggaggcggaa gtctgctgtg cctttgatgg gtacggtgcc gcttacaaat caacaaaata 1620
atataagcag tagtagtgtc aactcaactg gcaatggtgc tggggttacg aaggaaagaa 1680
ggccaagtta caggagaaaa tcaatgacac cgtccagaag atcaagtgtc gtaatagaat 1740
caacaaagga actcgaggag aaaccgttcc actgtcacat ttgtcccaag agctttaagc 1800

gcagcgaaca tttgaaaagg catgtgagat ctgttcactc taacgaacga ccatttgctt 1860

gtcacatatg cgataagaaa tttagtagaa gcgataattt gtcgcaacac atcaagactc 1920

ataaaaaaca tggagacatt taa 1943

<210> 10
<211> 855
<212> DNA
<213> Artificial Sequence

<220>
<223> truncated VPS35

<400> 10
atggcgtatg cggactcacc agaaaatgcg atcgctgtta tcaagcagcg aaccgcacta 60

atgaaccggt gtctatctca acacaaacta atggaatcat tacagcatac ttccataatg 120

ttgaccgaat tgagaaatcc aaacttatcg ccgaagaaat actacgaact ttatgtcatt 180

attttcgact cattgactaa tctatctacg tacctcatag aaaaccatcc tcaaaatcac 240

cacttagctg atctttatga gttggttcaa tataccggta acgtggtacc caggctttac 300

ttgatgatca cagttgggac cagctatctc actttcaatg aagcgcccaa gaaggaaatc 360

ttaaaggata tgattgagat gtgtcgtggt gtgcaaaacc caataagagg tttgttttta 420

cgctattatt tatcccagag aaccaaagaa ttacttccgg aggacgatcc gtcgtttaac 480

tctcaattta ttatgaataa tttcatcgag atgaacaagt tgtgggtgag attacaacac 540

caggggccac ttcgtgagag ggaaaccaga acacgtgaaa gaaaagagct gcaaatttta 600

gtggggtctc aactagtacg tctttcgcag attattgatg ataatttcca aatgtataag 660

caagatattc ttcccaccat tttggaacaa gtcatacaat gtagagattt agtatcccaa 720

gaatatcttt tggacgtcat ctgccaagtg ttcgcagacg agttccattt gaaaaccttg 780

gatactttac tgcaaactac tttgcatttg aaccctgatg tttcgataaa caagattgtt 840

ctcactttgg tcgat 855

<210> 11
<211> 168
<212> DNA
<213> Artificial Sequence

<220>
<223> truncated SNA3

<400> 11
cgatctttca cgcagacatg cgactgcgcc cgccgtagac cgtgacctgg aagctcaccc 60

tgcagaggaa tctcaagcac agcctccagc atatgatgaa gacgatgagg ccggtgccga 120

tgtgcccttg atggacaaca aacaacagct ctcttccggc cgtactta 168

<210> 12
<211> 636
<212> DNA

<213> Artificial Sequence

<220>
<223> truncated YHR045W

<400> 12
tgattcgata ggatcatcgc aaacaacaat aagcttgtac caatcctcgc tgccgtttag 60

catcttaaaa gacttccatg agtcgatgat caggacatca acatcttcta gtcgttgtct 120

aggaacagct ggcaagaaat gaggtattga acctgttctt atagaggtca tcatggaagc 180

caaggagaga gtaaaaccct ctacggtagc aatagaatta gctataccga tatttttgaa 240

attcttattg gaaagtttcg gaaatatgcg cttggccata ccattaagtt cactcaacga 300

ataacttttt tctcttcctg taaatttaat tatgtttttt cccttggaaa cttccattat 360

ggcattccaa acatcaccga aattgccatt tcttattttg tattttaaag atagccctaa 420

tcctgtcgtc aatgggaatc ctgtgggcac taaaatgctt cgataatatg ctgtttcatt 480

ttctttacga acactggata tgctagattg ctgtgacaaa gccacccctg ttaaatctct 540

cttaaaatca agtagaaaag agcttagcaa ccagttggcg cccagcacaa ttaacaatat 600

tgttattacc agctgtagta gaaaagacca attcat 636

<210> 13
<211> 938
<212> DNA
<213> Artificial Sequence

<220>
<223> truncated AIM45

<400> 13
atgtttaaat cattggctgc tgtcttgcct agagctagca aggcaaagtt cctccagaaa 60

aattacgcct ccactttagc tttcattgaa agctcaaaag atggctctgt ttcaaggtca 120

tcattgagtt tattggctgc tgcacaaaag ttgtctaacc ctatcacagc tgtaatcaca 180

ggtagcaaag ctgaaaaaac tgctgaggcg ctaaaatctt catattcatg cagcaattta 240

gaaaagcttg tcatatttga agattcaaaa ttagatacct gtcttcccga acaactaact 300

ccgttattag tgaaactatt aaaaggcggc gactattcac attttgttgt ctcaaactcc 360

tctgttggaa aaagtgtttt acctcgggtg ggtgcgctct tggacgtcca acctgtttgt 420

gaggttactg taatcaaaga tcctaagacc tttataaggc caatttatgc aggtaacatt 480

atttctacaa tagaatgcca ggcagaaaaa aaactgttga ttattagggc atcagctttt 540

ccaccaattg cagagggtag tatggattct gttaccattg agaagagaac tgatattcct 600

ccttgtgact taaatgttac ctgggttaaa actattctta ccaagagtga aaggcctgaa 660

cttacttctg cacagaacgt ggtaactggt ggaagggcac tcaaggataa ggagacattt 720

gagaagctat tatcgccgct agcagatgtt ttgcacgctg ctataggtgc cacaagagct 780

tctgttgata atggactatg tgataattct ctacaaatcg gtcagactgg taaggtagtc 840

gcaccaaatt tgtatatagc cattggcgtt tctggtgcag ttcagcattt agcgggaatg 900

aaggattcga aagttatcgt tgccattaac aatgatcc 938

<210> 14
<211> 125
<212> PRT
<213> Artificial Sequence

<220>
<223> truncated MIH1

<400> 14
Met Asn Asn Ile Phe His Gly Thr Glu Asp Glu Cys Ala Asn Glu Asp
1 5 10 15

Val Leu Ser Phe Gln Lys Ile Ser Leu Lys Ser Pro Phe Gly Lys Lys
20 25 30

Lys Asn Ile Phe Arg Asn Val Gln Thr Phe Phe Lys Ser Lys Ser Lys
35 40 45

His Ser Asn Val Asp Asp Asp Leu Ile Asn Lys Glu Asn Leu Ala Phe
50 55 60

Asp Lys Ser Pro Leu Leu Thr Asn His Arg Ser Lys Glu Ile Asp Gly
65 70 75 80

Pro Ser Pro Asn Ile Lys Gln Leu Gly His Arg Asp Glu Leu Asp Glu
85 90 95

Asn Glu Asn Glu Asn Asp Asp Ile Val Leu Ser Met His Phe Ala Ser
100 105 110

Gln Thr Leu Gln Ser Pro Thr Arg Asn Ser Ser Arg Arg
115 120 125

<210> 15
<211> 533
<212> PRT
<213> Artificial Sequence

<220>
<223> INO1

<400> 15
Met Thr Glu Asp Asn Ile Ala Pro Ile Thr Ser Val Lys Val Val Thr
1 5 10 15

Asp Lys Cys Thr Tyr Lys Asp Asn Glu Leu Leu Thr Lys Tyr Ser Tyr
20 25 30

Glu Asn Ala Val Val Thr Lys Thr Ala Ser Gly Arg Phe Asp Val Thr
35 40 45

Pro Thr Val Gln Asp Tyr Val Phe Lys Leu Asp Leu Lys Lys Pro Glu
50 55 60

Lys Leu Gly Ile Met Leu Ile Gly Leu Gly Gly Asn Asn Gly Ser Thr
65 70 75 80

Leu Val Ala Ser Val Leu Ala Asn Lys His Asn Val Glu Phe Gln Thr
85 90 95

```
Lys Glu Gly Val Lys Gln Pro Asn Tyr Phe Gly Ser Met Thr Gln Cys
            100                 105                 110

Ser Thr Leu Lys Leu Gly Ile Asp Ala Glu Gly Asn Asp Val Tyr Ala
        115                 120                 125

Pro Phe Asn Ser Leu Leu Pro Met Val Ser Pro Asn Asp Phe Val Val
    130                 135                 140

Ser Gly Trp Asp Ile Asn Asn Ala Asp Leu Tyr Glu Ala Met Gln Arg
145                 150                 155                 160

Ser Gln Val Leu Glu Tyr Asp Leu Gln Gln Arg Leu Lys Ala Lys Met
                165                 170                 175

Ser Leu Val Lys Pro Leu Pro Ser Ile Tyr Tyr Pro Asp Phe Ile Ala
            180                 185                 190

Ala Asn Gln Asp Glu Arg Ala Asn Asn Cys Ile Asn Leu Asp Glu Lys
        195                 200                 205

Gly Asn Val Thr Thr Arg Gly Lys Trp Thr His Leu Gln Arg Ile Arg
    210                 215                 220

Arg Asp Ile Gln Asn Phe Lys Glu Glu Asn Ala Leu Asp Lys Val Ile
225                 230                 235                 240

Val Leu Trp Thr Ala Asn Thr Glu Arg Tyr Val Glu Val Ser Pro Gly
                245                 250                 255

Val Asn Asp Thr Met Glu Asn Leu Leu Gln Ser Ile Lys Asn Asp His
            260                 265                 270

Glu Glu Ile Ala Pro Ser Thr Ile Phe Ala Ala Ala Ser Ile Leu Glu
        275                 280                 285

Gly Val Pro Tyr Ile Asn Gly Ser Pro Gln Asn Thr Phe Val Pro Gly
    290                 295                 300

Leu Val Gln Leu Ala Glu His Glu Gly Thr Phe Ile Ala Gly Asp Asp
305                 310                 315                 320

Leu Lys Ser Gly Gln Thr Lys Leu Lys Ser Val Leu Ala Gln Phe Leu
                325                 330                 335

Val Asp Ala Gly Ile Lys Pro Val Ser Ile Ala Ser Tyr Asn His Leu
            340                 345                 350

Gly Asn Asn Asp Gly Tyr Asn Leu Ser Ala Pro Lys Gln Phe Arg Ser
        355                 360                 365

Lys Glu Ile Ser Lys Ser Ser Val Ile Asp Asp Ile Ile Ala Ser Asn
    370                 375                 380

Asp Ile Leu Tyr Asn Asp Lys Leu Gly Lys Lys Val Asp His Cys Ile
385                 390                 395                 400

Val Ile Lys Tyr Met Lys Pro Val Gly Asp Ser Lys Val Ala Met Asp
                405                 410                 415

Glu Tyr Tyr Ser Glu Leu Met Leu Gly Gly His Asn Arg Ile Ser Ile
            420                 425                 430

His Asn Val Cys Glu Asp Ser Leu Leu Ala Thr Pro Leu Ile Ile Asp
        435                 440                 445

Leu Leu Val Met Thr Glu Phe Cys Thr Arg Val Ser Tyr Lys Lys Val
    450                 455                 460
```

Asp Pro Val Lys Glu Asp Ala Gly Lys Phe Glu Asn Phe Tyr Pro Val
465 470 475 480

Leu Thr Phe Leu Ser Tyr Trp Leu Lys Ala Pro Leu Thr Arg Pro Gly
485 490 495

Phe His Pro Val Asn Gly Leu Asn Lys Gln Arg Thr Ala Leu Glu Asn
500 505 510

Phe Leu Arg Leu Leu Ile Gly Leu Pro Ser Gln Asn Glu Leu Arg Phe
515 520 525

Glu Glu Arg Leu Leu
530

<210> 16
<211> 246
<212> PRT
<213> Artificial Sequence

<220>
<223> DOG1

<400> 16
Met Ala Glu Phe Ser Ala Asp Leu Cys Leu Phe Asp Leu Asp Gly Thr
1 5 10 15

Ile Val Ser Thr Thr Val Ala Ala Glu Lys Ala Trp Thr Lys Leu Cys
20 25 30

Tyr Glu Tyr Gly Val Asp Pro Ser Glu Leu Phe Lys His Ser His Gly
35 40 45

Ala Arg Thr Gln Glu Val Leu Arg Arg Phe Phe Pro Lys Leu Asp Asp
50 55 60

Thr Asp Asn Lys Gly Val Leu Ala Leu Glu Lys Asp Ile Ala His Ser
65 70 75 80

Tyr Leu Asp Thr Val Ser Leu Ile Pro Gly Ala Glu Asn Leu Leu Leu
85 90 95

Ser Leu Asp Val Asp Thr Glu Thr Gln Lys Lys Leu Pro Glu Arg Lys
100 105 110

Trp Ala Ile Val Thr Ser Gly Ser Pro Tyr Leu Ala Phe Ser Trp Phe
115 120 125

Glu Thr Ile Leu Lys Asn Val Gly Lys Pro Lys Val Phe Ile Thr Gly
130 135 140

Phe Asp Val Lys Asn Gly Lys Pro Asp Pro Glu Gly Tyr Ser Arg Ala
145 150 155 160

Arg Asp Leu Leu Arg Gln Asp Leu Gln Leu Thr Gly Lys Gln Asp Leu
165 170 175

Lys Tyr Val Val Phe Glu Asp Ala Pro Val Gly Ile Lys Ala Gly Lys
180 185 190

Ala Met Gly Ala Ile Thr Val Gly Ile Thr Ser Ser Tyr Asp Lys Ser
195 200 205

Val Leu Phe Asp Ala Gly Ala Asp Tyr Val Val Cys Asp Leu Thr Gln
210 215 220

Val Ser Val Val Lys Asn Asn Glu Asn Gly Ile Val Ile Gln Val Asn
225 230 235 240

Asn Pro Leu Thr Arg Ala
245

<210> 17
<211> 294
<212> PRT
<213> Artificial Sequence

<220>
<223> HAL1

<400> 17
Met His Phe Lys Asp Leu Gly Leu His Asp Tyr Thr Leu Lys Asn Leu
1 5 10 15

Met Tyr Glu Asn Asn Cys Cys Lys Phe Tyr Asp Ala Val Asp Glu Asn
20 25 30

Asn Ile Ser Tyr Val Leu Lys Phe Val Pro Ser Asp Val Thr Ser Glu
35 40 45

Gly Asp Thr Phe Pro Phe Val Asp Arg Phe Gln Val Lys Glu Gly Val
50 55 60

Phe Leu Val Tyr Ser Ser Asn Asp Phe Gly Lys Glu Gly Thr Asp Tyr
65 70 75 80

Phe Thr Tyr Thr Gly Ser Gly Gly Asn Glu Val His Ile Ser Gly Thr
85 90 95

Ser Ser Glu Ala Gly Ile Lys Pro Gln Phe Ile Glu Thr Cys His Pro
100 105 110

Lys His Leu Lys Arg Gly Thr Lys Glu Gln Glu Asp Ile Asn Ser Ser
115 120 125

Thr Ser Lys Lys Ser Ala Val Ile Asn Asn Phe Ser Gly Glu Lys Thr
130 135 140

Pro Asn Pro Arg Pro Gln Ser Ser Asn Ile Ser Glu Arg Glu Thr Tyr
145 150 155 160

Val Gly Ile Leu Asn Val Lys Cys Lys Asn Lys Asn Ser Ser Lys Ile
165 170 175

Arg Ser Glu Lys Leu Val Ser Ser Val Ile Glu Thr Lys His Thr Pro
180 185 190

Gly Leu Ala Ser Ile Leu Ser Lys Glu Gly Thr Thr Tyr Pro Asn Asn
195 200 205

Ala Asp Gly Lys His Ile Ser Ile Val Asn Pro Ser Ser Lys Ile Tyr
210 215 220

His Ser Ser His Lys Gln Ile Val Lys Thr Pro Ile Pro Lys Ser Gly
225 230 235 240

Leu Ser Pro Ile Glu Arg Cys Pro Phe Asn Gly Gln Asn Ile Lys Cys
245 250 255

Tyr Ser Pro Arg Pro Leu Asp His Glu Ser Pro Gln Arg Asp Phe Asn
260 265 270

```
Asn Asn Phe Gln Leu Arg Ile Leu Lys Ser Ser Val Leu Gln Arg Arg
        275                 280                 285

Gln Ser Thr Gln Asn Ser
    290


<210>    18
<211>    224
<212>    PRT
<213>    Artificial Sequence

<220>
<223>    TRP1


<400>    18
Met Ser Val Ile Asn Phe Thr Gly Ser Ser Gly Pro Leu Val Lys Val
1               5                   10                  15

Cys Gly Leu Gln Ser Thr Glu Ala Ala Glu Cys Ala Leu Asp Ser Asp
            20                  25                  30

Ala Asp Leu Leu Gly Ile Ile Cys Val Pro Asn Arg Lys Arg Thr Ile
        35                  40                  45

Asp Pro Val Ile Ala Arg Lys Ile Ser Ser Leu Val Lys Ala Tyr Lys
    50                  55                  60

Asn Ser Ser Gly Thr Pro Lys Tyr Leu Val Gly Val Phe Arg Asn Gln
65                  70                  75                  80

Pro Lys Glu Asp Val Leu Ala Leu Val Asn Asp Tyr Gly Ile Asp Ile
                85                  90                  95

Val Gln Leu His Gly Asp Glu Ser Trp Gln Glu Tyr Gln Glu Phe Leu
            100                 105                 110

Gly Leu Pro Val Ile Lys Arg Leu Val Phe Pro Lys Asp Cys Asn Ile
        115                 120                 125

Leu Leu Ser Ala Ala Ser Gln Lys Pro His Ser Phe Ile Pro Leu Phe
    130                 135                 140

Asp Ser Glu Ala Gly Gly Thr Gly Glu Leu Leu Asp Trp Asn Ser Ile
145                 150                 155                 160

Ser Asp Trp Val Gly Arg Gln Glu Ser Pro Glu Ser Leu His Phe Met
                165                 170                 175

Leu Ala Gly Gly Leu Thr Pro Glu Asn Val Gly Asp Ala Leu Arg Leu
            180                 185                 190

Asn Gly Val Ile Gly Val Asp Val Ser Gly Gly Val Glu Thr Asn Gly
        195                 200                 205

Val Lys Asp Ser Asn Lys Ile Ala Asn Phe Val Lys Asn Ala Lys Lys
    210                 215                 220



<210>    19
<211>    262
<212>    PRT
<213>    Artificial Sequence
```

<220>
<223> truncated MRPL17

<400> 19

```
Met Lys Val Asn Leu Met Leu Lys Arg Gly Leu Ala Thr Ala Thr Ala
1               5                   10                  15

Thr Ala Ser Ser Ala Pro Pro Lys Ile Lys Val Gly Val Leu Leu Ser
            20                  25                  30

Arg Ile Pro Ile Ile Lys Ser Glu Leu Asn Glu Leu Glu Lys Lys Tyr
        35                  40                  45

Tyr Glu Tyr Gln Ser Glu Leu Glu Lys Arg Leu Met Trp Thr Phe Pro
    50                  55                  60

Ala Tyr Phe Tyr Phe Lys Lys Gly Thr Val Ala Glu His Lys Phe Leu
65                  70                  75                  80

Ser Leu Gln Lys Gly Pro Ile Ser Lys Lys Asn Gly Ile Trp Phe Pro
                85                  90                  95

Arg Gly Ile Pro Asp Ile Lys His Gly Arg Glu Arg Ser Thr Lys Gln
            100                 105                 110

Glu Val Lys Leu Ser Asp Asp Ser Thr Val Ala Phe Ser Asn Asn Gln
        115                 120                 125

Lys Glu Gln Ser Lys Asp Asp Val Asn Arg Pro Val Ile Pro Asn Asp
    130                 135                 140

Arg Ile Thr Glu Ala Asp Arg Ser Asn Asp Met Lys Ser Leu Glu Arg
145                 150                 155                 160

Gln Leu Ser Arg Thr Leu Tyr Leu Leu Val Lys Asp Lys Ser Gly Thr
                165                 170                 175

Trp Lys Phe Pro Asn Phe Asp Leu Ser Asp Glu Ser Lys Pro Leu His
            180                 185                 190

Val His Ala Glu Asn Glu Leu Lys Leu Leu Ser Gly Asp Gln Ile Tyr
        195                 200                 205

Thr Trp Ser Val Ser Ala Thr Pro Ile Gly Val Leu Gln Asp Glu Arg
    210                 215                 220

Asn Arg Thr Ala Glu Phe Ile Val Lys Ser His Ile Leu Ala Gly Lys
225                 230                 235                 240

Phe Asp Leu Val Ala Ser Lys Asn Asp Ala Phe Glu Asp Phe Ala Trp
                245                 250                 255

Leu Thr Lys Gly Glu Ile
            260
```

<210> 20
<211> 489
<212> PRT
<213> Artificial Sequence

<220>
<223> Partial fragment YLR157C-B

<400> 20

```
Ile Thr Gly Glu Ile *** Val Met Arg Arg Met Ile Leu Ala Ala Ile
```

```
 1               5                   10                  15
Arg Ile Gln Pro Asn Pro Lys Leu *** Leu Gly Ile Leu Lys Lys Gln
            20                  25                  30
Ile Ile Arg Asn Arg Lys Gln Pro Gly Leu Thr Met Tyr Pro His Leu
        35                  40                  45
Ile Thr Leu Pro Ala Arg Thr Thr Ile Pro Ser Val Asn Gln Leu Leu
    50                  55                  60
Asn Arg Phe Asn *** Thr Ile Ser Thr Thr Phe Thr Leu Gly Gln Glu
65                  70                  75                  80
Leu Thr Glu Ser Thr Val Asn His Thr Asn His Ser Asp Asp Glu Leu
                85                  90                  95
Pro Gly His Leu Leu Leu Asp Ser Gly Ala Ser Arg Thr Leu Ile Arg
            100                 105                 110
Ser Ala His His Ile His Ser Ala Ser Ser Asn Pro Asp Ile Asn Val
        115                 120                 125
Val Asp Ala Gln Lys Arg Asn Ile Pro Ile Asn Ala Ile Gly Asp Leu
    130                 135                 140
Gln Phe His Phe Gln Asp Asn Thr Lys Thr Ser Ile Lys Val Leu His
145                 150                 155                 160
Thr Pro Asn Ile Ala Tyr Asp Leu Leu Ser Leu Asn Glu Leu Ala Ala
                165                 170                 175
Val Asp Ile Thr Ala Cys Phe Thr Lys Asn Val Leu Glu Arg Ser Asp
            180                 185                 190
Gly Thr Val Leu Ala Pro Ile Val Lys Tyr Gly Asp Phe Tyr Trp Val
        195                 200                 205
Ser Lys Lys Tyr Leu Leu Pro Ser Asn Ile Ser Val Pro Thr Ile Asn
    210                 215                 220
Asn Val His Thr Ser Glu Ser Thr Arg Lys Tyr Pro Tyr Pro Phe Ile
225                 230                 235                 240
His Arg Met Leu Ala His Ala Asn Ala Pro Thr Ile Arg Tyr Ser Leu
                245                 250                 255
Lys Asn Asn Thr Ile Thr Tyr Phe Asn Glu Ser Asp Val Asp Trp Ser
            260                 265                 270
Ser Ala Ile Asp Tyr Gln Cys Pro Asp Cys Leu Ile Gly Lys Ser Thr
        275                 280                 285
Lys His Arg His Ile Lys Gly Ser Arg Leu Lys Tyr Gln Asn Ser Tyr
    290                 295                 300
Glu Pro Phe Gln Tyr Leu His Thr Asp Ile Phe Gly Pro Val His Asn
305                 310                 315                 320
Leu Pro Asn Ser Ala Pro Ser Tyr Phe Ile Ser Phe Thr Asp Glu Thr
                325                 330                 335
Thr Lys Phe Arg Trp Val Tyr Pro Leu His Asp Arg Arg Glu Asp Ser
            340                 345                 350
Ile Leu Asp Val Phe Thr Thr Ile Leu Ala Phe Ile Lys Asn Gln Phe
        355                 360                 365
```

```
Gln Ala Ser Val Leu Val Ile Gln Met Asp Arg Gly Ser Glu Tyr Thr
    370                 375                 380

Asn Arg Thr Leu His Lys Phe Leu Glu Lys Asn Gly Ile Thr Pro Cys
385                 390                 395                 400

Tyr Thr Thr Thr Ala Asp Ser Arg Ala His Gly Val Ala Glu Arg Leu
                405                 410                 415

Asn Arg Thr Leu Leu Asp Asp Cys Arg Thr Gln Leu Gln Cys Ser Gly
            420                 425                 430

Leu Pro Asn His Leu Trp Phe Ser Ala Ile Glu Phe Ser Thr Ile Val
        435                 440                 445

Arg Asn Ser Leu Ala Ser Pro Lys Ser Lys Lys Ser Ala Arg Gln His
    450                 455                 460

Ala Gly Leu Ala Gly Leu Asp Ile Ser Thr Leu Leu Pro Phe Gly Gln
465                 470                 475                 480

Pro Val Ile Val Asn Asp His Asn Pro
                485


<210>   21
<211>   646
<212>   PRT
<213>   Artificial Sequence

<220>
<223>   truncated MSN2


<400>   21
Asn Gln Glu Thr Ser Leu Asn Leu Gly Leu Pro Pro Leu Ser Phe Asp
1               5                   10                  15

Ser Pro Leu Pro Val Thr Glu Thr Ile Pro Ser Thr Thr Asp Asn Ser
            20                  25                  30

Leu His Leu Lys Ala Asp Ser Asn Lys Asn Arg Asp Ala Arg Thr Ile
        35                  40                  45

Glu Asn Asp Ser Glu Ile Lys Ser Thr Asn Asn Ala Ser Gly Ser Gly
    50                  55                  60

Ala Asn Gln Tyr Thr Thr Leu Thr Ser Pro Tyr Pro Met Asn Asp Ile
65                  70                  75                  80

Leu Tyr Asn Met Asn Asn Pro Leu Gln Ser Pro Ser Pro Ser Ser Val
                85                  90                  95

Pro Gln Asn Pro Thr Ile Asn Pro Pro Ile Asn Thr Ala Ser Asn Glu
            100                 105                 110

Thr Asn Leu Ser Pro Gln Thr Ser Asn Gly Asn Glu Thr Leu Ile Ser
        115                 120                 125

Pro Arg Ala Gln Gln His Thr Ser Ile Lys Asp Asn Arg Leu Ser Leu
    130                 135                 140

Pro Asn Gly Ala Asn Ser Asn Leu Phe Ile Asp Thr Asn Pro Asn Asn
145                 150                 155                 160

Leu Asn Glu Lys Leu Arg Asn Gln Leu Asn Ser Asp Thr Asn Ser Tyr
                165                 170                 175
```

```
Ser Asn Ser Ile Ser Asn Ser Asn Ser Asn Ser Thr Gly Asn Leu Asn
            180                 185                 190

Ser Ser Tyr Phe Asn Ser Leu Asn Ile Asp Ser Met Leu Asp Asp Tyr
        195                 200                 205

Val Ser Ser Asp Leu Leu Leu Asn Asp Asp Asp Asp Asp Thr Asn Leu
    210                 215                 220

Ser Arg Arg Arg Phe Ser Asp Val Ile Thr Asn Gln Phe Pro Ser Met
225                 230                 235                 240

Thr Asn Ser Arg Asn Ser Ile Ser His Ser Leu Asp Leu Trp Asn His
                245                 250                 255

Pro Lys Ile Asn Pro Ser Asn Arg Asn Thr Asn Leu Asn Ile Thr Thr
            260                 265                 270

Asn Ser Thr Ser Ser Ser Asn Ala Ser Pro Asn Thr Thr Thr Met Asn
        275                 280                 285

Ala Asn Ala Asp Ser Asn Ile Ala Gly Asn Pro Lys Asn Asn Asp Ala
    290                 295                 300

Thr Ile Asp Asn Glu Leu Thr Gln Ile Leu Asn Glu Tyr Asn Met Asn
305                 310                 315                 320

Phe Asn Asp Asn Leu Gly Thr Ser Thr Ser Gly Lys Asn Lys Ser Ala
                325                 330                 335

Cys Pro Ser Ser Phe Asp Ala Asn Ala Met Thr Lys Ile Asn Pro Ser
            340                 345                 350

Gln Gln Leu Gln Gln Gln Leu Asn Arg Val Gln His Lys Gln Leu Thr
        355                 360                 365

Ser Ser His Asn Asn Ser Ser Thr Asn Met Lys Ser Phe Asn Ser Asp
    370                 375                 380

Leu Tyr Ser Arg Arg Gln Arg Ala Ser Leu Pro Ile Ile Asp Asp Ser
385                 390                 395                 400

Leu Ser Tyr Asp Leu Val Asn Lys Gln Asp Glu Asp Pro Lys Asn Asp
                405                 410                 415

Met Leu Pro Asn Ser Asn Leu Ser Ser Ser Gln Gln Phe Ile Lys Pro
            420                 425                 430

Ser Met Ile Leu Ser Asp Asn Ala Ser Val Ile Ala Lys Val Ala Thr
        435                 440                 445

Thr Gly Leu Ser Asn Asp Met Pro Phe Leu Thr Glu Glu Gly Glu Gln
    450                 455                 460

Asn Ala Asn Ser Thr Pro Asn Phe Asp Leu Ser Ile Thr Gln Met Asn
465                 470                 475                 480

Met Ala Pro Leu Ser Pro Ala Ser Ser Ser Ser Thr Ser Leu Ala Thr
                485                 490                 495

Asn His Phe Tyr His His Phe Pro Gln Gln Gly His His Thr Met Asn
            500                 505                 510

Ser Lys Ile Gly Ser Ser Leu Arg Arg Arg Lys Ser Ala Val Pro Leu
        515                 520                 525

Met Gly Thr Val Pro Leu Thr Asn Gln Gln Asn Asn Ile Ser Ser Ser
    530                 535                 540
```

Ser Val Asn Ser Thr Gly Asn Gly Ala Gly Val Thr Lys Glu Arg Arg
545 550 555 560

Pro Ser Tyr Arg Arg Lys Ser Met Thr Pro Ser Arg Arg Ser Ser Val
565 570 575

Val Ile Glu Ser Thr Lys Glu Leu Glu Glu Lys Pro Phe His Cys His
580 585 590

Ile Cys Pro Lys Ser Phe Lys Arg Ser Glu His Leu Lys Arg His Val
595 600 605

Arg Ser Val His Ser Asn Glu Arg Pro Phe Ala Cys His Ile Cys Asp
610 615 620

Lys Lys Phe Ser Arg Ser Asp Asn Leu Ser Gln His Ile Lys Thr His
625 630 635 640

Lys Lys His Gly Asp Ile
645

<210> 22
<211> 284
<212> PRT
<213> Artificial Sequence

<220>
<223> truncated VPS35

<400> 22
Met Ala Tyr Ala Asp Ser Pro Glu Asn Ala Ile Ala Val Ile Lys Gln
1 5 10 15

Arg Thr Ala Leu Met Asn Arg Cys Leu Ser Gln His Lys Leu Met Glu
20 25 30

Ser Leu Gln His Thr Ser Ile Met Leu Thr Glu Leu Arg Asn Pro Asn
35 40 45

Leu Ser Pro Lys Lys Tyr Tyr Glu Leu Tyr Val Ile Ile Phe Asp Ser
50 55 60

Leu Thr Asn Leu Ser Thr Tyr Leu Ile Glu Asn His Pro Gln Asn His
65 70 75 80

His Leu Ala Asp Leu Tyr Glu Leu Val Gln Tyr Thr Gly Asn Val Val
85 90 95

Pro Arg Leu Tyr Leu Met Ile Thr Val Gly Thr Ser Tyr Leu Thr Phe
100 105 110

Asn Glu Ala Pro Lys Lys Glu Ile Leu Lys Asp Met Ile Glu Met Cys
115 120 125

Arg Gly Val Gln Asn Pro Ile Arg Gly Leu Phe Leu Arg Tyr Tyr Leu
130 135 140

Ser Gln Arg Thr Lys Glu Leu Leu Pro Glu Asp Asp Pro Ser Phe Asn
145 150 155 160

Ser Gln Phe Ile Met Asn Asn Phe Ile Glu Met Asn Lys Leu Trp Val
165 170 175

Arg Leu Gln His Gln Gly Pro Leu Arg Glu Arg Glu Thr Arg Thr Arg
180 185 190

```
Glu Arg Lys Glu Leu Gln Ile Leu Val Gly Ser Gln Leu Val Arg Leu
        195                 200                 205

Ser Gln Ile Ile Asp Asp Asn Phe Gln Met Tyr Lys Gln Asp Ile Leu
    210                 215                 220

Pro Thr Ile Leu Glu Gln Val Ile Gln Cys Arg Asp Leu Val Ser Gln
225                 230                 235                 240

Glu Tyr Leu Leu Asp Val Ile Cys Gln Val Phe Ala Asp Glu Phe His
                245                 250                 255

Leu Lys Thr Leu Asp Thr Leu Leu Gln Thr Thr Leu His Leu Asn Pro
            260                 265                 270

Asp Val Ser Ile Asn Lys Ile Val Leu Thr Leu Val
        275                 280


<210>   23
<211>   55
<212>   PRT
<213>   Artificial Sequence

<220>
<223>   truncated SNA3


<400>   23
Asp Leu Ser Arg Arg His Ala Thr Ala Pro Ala Val Asp Arg Asp Leu
1               5                   10                  15

Glu Ala His Pro Ala Glu Glu Ser Gln Ala Gln Pro Pro Ala Tyr Asp
            20                  25                  30

Glu Asp Asp Glu Ala Gly Ala Asp Val Pro Leu Met Asp Asn Lys Gln
        35                  40                  45

Gln Leu Ser Ser Gly Arg Thr
    50                  55


<210>   24
<211>   212
<212>   PRT
<213>   Artificial Sequence

<220>
<223>   truncated YHR045W


<400>   24
Met Asn Trp Ser Phe Leu Leu Gln Leu Val Ile Thr Ile Leu Leu Ile
1               5                   10                  15

Val Leu Gly Ala Asn Trp Leu Leu Ser Ser Phe Leu Leu Asp Phe Lys
            20                  25                  30

Arg Asp Leu Thr Gly Val Ala Leu Ser Gln Gln Ser Ser Ile Ser Ser
        35                  40                  45

Val Arg Lys Glu Asn Glu Thr Ala Tyr Tyr Arg Ser Ile Leu Val Pro
    50                  55                  60

Thr Gly Phe Pro Leu Thr Thr Gly Leu Gly Leu Ser Leu Lys Tyr Lys
65                  70                  75                  80
```

Ile Arg Asn Gly Asn Phe Gly Asp Val Trp Asn Ala Ile Met Glu Val
85 90 95

Ser Lys Gly Lys Asn Ile Ile Lys Phe Thr Gly Arg Glu Lys Ser Tyr
100 105 110

Ser Leu Ser Glu Leu Asn Gly Met Ala Lys Arg Ile Phe Pro Lys Leu
115 120 125

Ser Asn Lys Asn Phe Lys Asn Ile Gly Ile Ala Asn Ser Ile Ala Thr
130 135 140

Val Glu Gly Phe Thr Leu Ser Leu Ala Ser Met Met Thr Ser Ile Arg
145 150 155 160

Thr Gly Ser Ile Pro His Phe Leu Pro Ala Val Pro Arg Gln Arg Leu
165 170 175

Glu Asp Val Asp Val Leu Ile Ile Asp Ser Trp Lys Ser Phe Lys Met
180 185 190

Leu Asn Gly Ser Glu Asp Trp Tyr Lys Leu Ile Val Val Cys Asp Asp
195 200 205

Pro Ile Glu Ser
210

<210> 25
<211> 312
<212> PRT
<213> Artificial Sequence

<220>
<223> truncated AIM45

<400> 25
Met Phe Lys Ser Leu Ala Ala Val Leu Pro Arg Ala Ser Lys Ala Lys
1 5 10 15

Phe Leu Gln Lys Asn Tyr Ala Ser Thr Leu Ala Phe Ile Glu Ser Ser
20 25 30

Lys Asp Gly Ser Val Ser Arg Ser Ser Leu Ser Leu Leu Ala Ala Ala
35 40 45

Gln Lys Leu Ser Asn Pro Ile Thr Ala Val Ile Thr Gly Ser Lys Ala
50 55 60

Glu Lys Thr Ala Glu Ala Leu Lys Ser Ser Tyr Ser Cys Ser Asn Leu
65 70 75 80

Glu Lys Leu Val Ile Phe Glu Asp Ser Lys Leu Asp Thr Cys Leu Pro
85 90 95

Glu Gln Leu Thr Pro Leu Leu Val Lys Leu Leu Lys Gly Gly Asp Tyr
100 105 110

Ser His Phe Val Val Ser Asn Ser Ser Val Gly Lys Ser Val Leu Pro
115 120 125

Arg Val Gly Ala Leu Leu Asp Val Gln Pro Val Cys Glu Val Thr Val
130 135 140

Ile Lys Asp Pro Lys Thr Phe Ile Arg Pro Ile Tyr Ala Gly Asn Ile
145 150 155 160

Ile Ser Thr Ile Glu Cys Gln Ala Glu Lys Lys Leu Leu Ile Ile Arg
165 170 175

Ala Ser Ala Phe Pro Pro Ile Ala Glu Gly Ser Met Asp Ser Val Thr
180 185 190

Ile Glu Lys Arg Thr Asp Ile Pro Pro Cys Asp Leu Asn Val Thr Trp
195 200 205

Val Lys Thr Ile Leu Thr Lys Ser Glu Arg Pro Glu Leu Thr Ser Ala
210 215 220

Gln Asn Val Val Thr Gly Gly Arg Ala Leu Lys Asp Lys Glu Thr Phe
225 230 235 240

Glu Lys Leu Leu Ser Pro Leu Ala Asp Val Leu His Ala Ala Ile Gly
245 250 255

Ala Thr Arg Ala Ser Val Asp Asn Gly Leu Cys Asp Asn Ser Leu Gln
260 265 270

Ile Gly Gln Thr Gly Lys Val Val Ala Pro Asn Leu Tyr Ile Ala Ile
275 280 285

Gly Val Ser Gly Ala Val Gln His Leu Ala Gly Met Lys Asp Ser Lys
290 295 300

Val Ile Val Ala Ile Asn Asn Asp
305 310

<210> 26
<211> 3202
<212> DNA
<213> Artificial Sequence

<220>
<223> truncated MSN2/MIH1

<400> 26
aaaatcaaga aacttcactg aatttggggc ttcctccact atctttcgac tctccactgc 60

ccgtaacgga aacgatacca tccactaccg ataacagctt gcatttgaaa gctgatagca 120

acaaaaatcg cgatgcaaga actattgaaa atgatagtga aattaagagt actaataatg 180

ctagtggctc tggggcaaat caatacacaa ctcttacttc accttatcct atgaacgaca 240

ttttgtacaa catgaacaat ccgttacaat caccgtcacc ttcatcggta cctcaaaatc 300

cgactataaa tcctcccata aatacagcaa gtaacgaaac taatttatcg cctcaaactt 360

caaatggtaa tgaaactctt atatctcctc gagcccaaca acatacgtcc attaaagata 420

atcgtctgtc cttacctaat ggtgctaatt cgaatctttt cattgacact aacccaaaca 480

atttgaacga aaaactaaga aatcaattga actcagatac aaattcatat tctaactcca 540

tttctaattc aaactccaat tctacgggta atttaaattc cagttatttt aattcactga 600

acatagactc catgctagat gattacgttt ctagtgatct cttattgaat gatgatgatg 660

atgacactaa tttatcacgc cgaagattta gcgacgttat aacaaaccaa tttccgtcaa 720

tgacaaattc gaggaattct atttctcact ctttggacct ttggaaccat ccgaaaatta 780

atccaagcaa tagaaataca aatctcaata tcactactaa ttctacctca agttccaatg 840

caagtccgaa taccactact atgaacgcaa atgcagactc aaatattgct ggcaacccga 900
aaaacaatga cgctaccata gacaatgagt tgacacagat tcttaacgaa tataatatga 960
acttcaacga taatttgggc acatccactt ctggcaagaa caaatctgct tgcccaagtt 1020
cttttgatgc caatgctatg acaaagataa atccaagtca gcaattacag caacagctaa 1080
accgagttca acacaagcag ctcacctcgt cacataataa cagtagcact aacatgaaat 1140
ccttcaacag cgatctttat tcaagaaggc aaagagcttc tttacccata atcgatgatt 1200
cactaagcta cgacctggtt aataagcagg atgaagaccc caagaacgat atgctgccga 1260
attcaaattt gagttcatct caacaattta tcaaaccgtc tatgattctt tcagacaatg 1320
cgtccgttat tgcgaaagtg gcgactacag gcttgagtaa tgatatgcca tttttgacag 1380
aggaaggtga acaaaatgct aattctactc caaatttcga tctttccatc actcaaatga 1440
atatggctcc attatcgcct gcatcatcat cctccacgtc tcttgcaaca aatcatttct 1500
atcaccattt cccacagcag ggtcaccata ccatgaactc taaaatcggt tcttcccttc 1560
ggaggcggaa gtctgctgtg cctttgatgg gtacggtgcc gcttacaaat caacaaaata 1620
atataagcag tagtagtgtc aactcaactg gcaatggtgc tggggttacg aaggaaagaa 1680
ggccaagtta caggagaaaa tcaatgacac cgtccagaag atcaagtgtc gtaatagaat 1740
caacaaagga actcgaggag aaaccgttcc actgtcacat ttgtcccaag agctttaagc 1800
gcagcgaaca tttgaaaagg catgtgagat ctgttcactc taacgaacga ccatttgctt 1860
gtcacatatg cgataagaaa tttagtagaa gcgataattt gtcgcaacac atcaagactc 1920
ataaaaaaca tggagacatt taatagaccc cattttttta attcgataga tctttcttca 1980
taagataatt ctgttcaatg acttatgaag cttacggctt attgttatta ttatcatgat 2040
tctttgccat ttttccggta ccttcacatt ttgaaggaat tcagttcccc cgcctgtaac 2100
aacttagata cacgtagata aaaattaata tagccaaaat atttggtatg atagggtata 2160
gtataatata attaatacaa tataatataa tatagtacag tacagtataa tattgtataa 2220
tatcatatgg caccgtaata tcatatatat atccgtacag tttaatgaat tccgtgcgca 2280
ttatgctgtg caatatgcct gtcagtttga aaatcgaaga taccacatcg tacaaaagca 2340
agaggggtgc ccctaatggg agaagattac cgtttttatt ttggggtagc taactgcgcc 2400
acaaatagta tctaccagtt tgctactttt agaactgtat tatgtatctt tgccgaatac 2460
ttaatttcct aggcctcatt ttcaggacta aggatgaaga ggggcttcaa tgttttatat 2520
ggcgcttttt ctgtccattg tatacgtcaa aaaagcatag atactccact tattagcggt 2580
aaagcccttt ttgatacatt gttccggcgt ttaaatattc tcattatgat atttccccaa 2640
cagttacact ctgttatttc cttctttttt atgctttatc tgtttttttcc ctgtttcgat 2700
gccgtatatc agaaaactta aagttacaag cgaaaacaaa gaaaagagag ttaacttgaa 2760
aggagacaaa agataaacag agcagtggac aaaccaggat tgaagtcagc gagggtgaag 2820
aaaccatgaa caatatattt catggaactg aagatgaatg tgccaatgaa gacgttctta 2880

gtttccaaaa aatttccttg aaaagtccct ttggtaagaa gaagaacata tttagaaatg 2940

ttcagacctt ctttaagtca aaaagcaaac attcgaatgt cgacgatgat ttaatcaata 3000

aagagaatct tgcctttgat aaatctccat tgttaacaaa tcacaggagt aaggaaattg 3060

atggtccttc accgaatata aagcagcttg gccatcgcga tgagttagat gaaaatgaaa 3120

atgaaaatga tgatatagtc ttaagcatgc attttgcttc tcaaacctta caaagtccaa 3180

caagaaactc atcaagaaga tc 3202

<210> 27
<211> 3460
<212> DNA
<213> Artificial Sequence

<220>
<223> INO1-truncated VPS35/truncated SNA3

<400> 27
cgatctttca cgcagacatg cgactgcgcc cgccgtagac cgtgacctgg aagctcaccc 60

tgcagaggaa tctcaagcac agcctccagc atatgatgaa gacgatgagg ccggtgccga 120

tgtgcccttg atggacaaca aacaacagct ctcttccggc cgtacttagt gatcggaacg 180

agctctttat caccgtagtt ctaaataaca catagagtaa attattgcct ttttcttcgt 240

tccttttgtt cttcacgtcc tttttatgaa atacgtgccg gtgttccggg gttggatgcg 300

gaatcgaaag tgttgaatgt gaaatatgcg gaggccaagt atgcgcttcg gcggctaaat 360

gcggcatgtg aaaagtattg tctattttat cttcatcctt ctttcccaga atattgaact 420

tatttaattc acatggagca gagaaagcgc acctctgcgt tggcggcaat gttaatttga 480

gacgtatata aattggagct ttcgtcacct ttttttggct tgttctgttg tcgggttcct 540

aatgttagtt ttatccttga tttattctgt ttcattccct tttttttcca gtgaaaaaga 600

agtaacaatg acagaagata atattgctcc aatcacctcc gttaaagtag ttaccgacaa 660

gtgcacgtac aaggacaacg agctgctcac caagtacagc tacgaaaatg ctgtagttac 720

gaagacagct agtggccgct tcgatgtaac gcccactgtt caagactacg tgttcaaact 780

tgacttgaaa aagccggaaa aactaggaat tatgctcatt gggttaggtg gcaacaatgg 840

ctccacttta gtggcctcgg tattggcgaa taagcacaat gtggagtttc aaactaagga 900

aggcgttaag caaccaaact acttcggctc catgactcaa tgttctacct tgaaactggg 960

tatcgatgcg gaggggaatg acgtttatgc tccttttaac tctctgttgc ccatggttag 1020

cccaaacgac tttgtcgtct ctggttggga catcaataac gcagatctat acgaagctat 1080

gcagagaagt caagttctcg aatatgatct gcaacaacgc ttgaaggcga agatgtcctt 1140

ggtgaagcct cttccttcca tttactaccc tgatttcatt gcagctaatc aagatgagag 1200

agccaataac tgcatcaatt tggatgaaaa aggcaacgta accacgaggg gtaagtggac 1260

ccatctgcaa cgcatcagac gcgatatcca gaatttcaaa gaagaaaacg cccttgataa 1320

agtaatcgtt ctttggactg caaatactga gaggtacgta gaagtatctc ctggtgttaa 1380
tgacaccatg gaaaacctct tgcagtctat taagaatgac catgaagaga ttgctccttc 1440
cacgatcttt gcagcagcat ctatcttgga aggtgtcccc tatattaatg gttcaccgca 1500
gaatactttt gttcccggct tggttcagct ggctgagcat gagggtacat tcattgcggg 1560
agacgatctc aagtcgggac aaaccaagtt gaagtctgtt ctggcccagt tcttagtgga 1620
tgcaggtatt aaaccggtct ccattgcatc ctataaccat ttaggcaata atgacggtta 1680
taacttatct gctccaaaac aatttaggtc taaggagatt tccaaaagtt ctgtcataga 1740
tgacatcatc gcgtctaatg atatcttgta caatgataaa ctgggtaaaa aagttgacca 1800
ctgcattgtc atcaaatata tgaagcccgt cggggactca aaagtggcaa tggacgagta 1860
ttacagtgag ttgatgttag gtggccataa ccggatttcc attcacaatg tttgcgaaga 1920
ttctttactg gctacgccct tgatcatcga tcttttagtc atgactgagt tttgtacaag 1980
agtgtcctat aagaaggtgg acccagttaa agaagatgct ggcaaattcg agaactttta 2040
tccagtttta accttcttga gttactggtt aaaagctcca ttaacaagac caggatttca 2100
cccggtgaat ggcttaaaca agcaaagaac cgccttagaa aattttttaa gattgttgat 2160
tggattgcct tctcaaaacg aactaagatt cgaagagaga ttgttgtaat ctcatttcaa 2220
cgactctctt tttctttttc cgcctaccta taaaaaaaca agacattcac cattatccta 2280
ttatcccttc catcaataca tatacttaac ataacgttta taaaaattca actatcaaca 2340
gtctttatat tttttttttt tcttttgaac tattgcctct ttgtcacttc gtcttaaagg 2400
ggcgtttttt attttttttt ttttttttca gttgaggtag atgcgagaaa gtgctgtatt 2460
tattcaaggg ccacctcagt aaagagaaga aaagagagaa aaaaaaaaag aaggtggtgt 2520
atgtgcgacc actcaacaag gcccagtgaa gttaatatat aacgataaaa ggaggaggac 2580
gagaaagaag aagctgaaaa acacaatggc gtatgcggac tcaccagaaa atgcgatcgc 2640
tgttatcaag cagcgaaccg cactaatgaa ccggtgtcta tctcaacaca aactaatgga 2700
atcattacag catacttcca taatgttgac cgaattgaga aatccaaact tatcgccgaa 2760
gaaatactac gaactttatg tcattatttt cgactcattg actaatctat ctacgtacct 2820
catagaaaac catcctcaaa atcaccactt agctgatctt tatgagttgg ttcaatatac 2880
cggtaacgtg gtacccaggc tttacttgat gatcacagtt gggaccagct atctcacttt 2940
caatgaagcg cccaagaagg aaatcttaaa ggatatgatt gagatgtgtc gtggtgtgca 3000
aaacccaata agaggtttgt ttttacgcta ttatttatcc cagagaacca aagaattact 3060
tccggaggac gatccgtcgt ttaactctca atttattatg aataatttca tcgagatgaa 3120
caagttgtgg gtgagattac aacaccaggg gccacttcgt gagagggaaa ccagaacacg 3180
tgaaagaaaa gagctgcaaa ttttagtggg gtctcaacta gtacgtcttt cgcagattat 3240
tgatgataat ttccaaatgt ataagcaaga tattcttccc accattttgg aacaagtcat 3300
acaatgtaga gatttagtat cccaagaata tcttttggac gtcatctgcc aagtgttcgc 3360

agacgagttc catttgaaaa ccttggatac tttactgcaa actactttgc atttgaaccc 3420

tgatgtttcg ataaacaaga ttgttctcac tttggtcgat 3460

<210> 28
<211> 2371
<212> DNA
<213> Artificial Sequence

<220>
<223> DOG1-truncated YHR045W

<400> 28
tgattcgata ggatcatcgc aaacaacaat aagcttgtac caatcctcgc tgccgtttag 60

catcttaaaa gacttccatg agtcgatgat caggacatca acatcttcta gtcgttgtct 120

aggaacagct ggcaagaaat gaggtattga acctgttctt atagaggtca tcatggaagc 180

caaggagaga gtaaaaccct ctacggtagc aatagaatta gctataccga tatttttgaa 240

attcttattg gaaagtttcg gaaatatgcg cttggccata ccattaagtt cactcaacga 300

ataacttttt tctcttcctg taaatttaat tatgtttttt cccttggaaa cttccattat 360

ggcattccaa acatcaccga aattgccatt tcttattttg tattttaaag atagccctaa 420

tcctgtcgtc aatgggaatc ctgtgggcac taaaatgctt cgataatatg ctgtttcatt 480

ttctttacga acactggata tgctagattg ctgtgacaaa gccacccctg ttaaatctct 540

cttaaaatca agtagaaaag agcttagcaa ccagttggcg cccagcacaa ttaacaatat 600

tgttattacc agctgtagta gaaaagacca attcattgct catcaacttt tgccgcttgt 660

ttaactgtct ttctttctaa cgaaagaact ttctatagct taattgaaaa gcacttgaaa 720

tgcttaaaac ttaacctatg ttgaagctat aaaggtgttg cagaatgatt tccattcaat 780

gtacttctcg aagttgctgt cgtttatgat acgatacata ttagaacttt ttcattctta 840

acgacgcgca aagaaaaacg aaaagctaat gaccaaacca agggtcctag gattctcatt 900

aacatcaatt caaggcaatc agttacaata tttataataa agtaattcgt gctatgtatc 960

cttttgccaa gtaacttgcc agaaattccg tttatctact tcatcttatc ggtgaaaaaa 1020

tatacactgg gctcttctac acacgccttc taccgcatat cctttagagt cttggcttct 1080

tgtaattttc aatcacggtt atgttccgtg atacagacac gtcagagtat tcctatatat 1140

tccgacatta accccgacat ttcaaacaac cgttgttcag ctatagaagg aagcgtatat 1200

ctatgagatt ataggctctg gtaccatcta atgatataaa tttcatggaa ctttttttatt 1260

gcaagtattt tatttaattt tattttcatt tttaatgatc gcgattattc tgttggaaat 1320

aacgttctga tggagattgt tggttacgtt gccactcacg taagaagttc aaaggataat 1380

ggcagaattt tcagctgatc tatgtctttt tgacctagat ggtaccatag tgagtacaac 1440

agtggccgca gagaaagcat ggaccaagtt gtgttacgaa tacggtgttg atccttccga 1500

gttatttaag cattctcatg gtgcaagaac acaagaggtt ttgagaaggt tttccctaa 1560

attggatgat acagacaata aaggtgttct tgctctagaa aaagatattg cccatagtta 1620

cttggacaca gtaagcctta ttcctggtgc agagaactta ctgttatcgt tagatgtaga 1680

tactgagact caaaaaaagt tacctgaaag gaaatgggct atcgttacct ctggttctcc 1740

atatttggca ttttcatggt tcgagacaat attgaaaaat gttggaaagc ccaaagtttt 1800

cattactggg tttgacgtga agaacggtaa gcctgatccc gagggttatt caagagctcg 1860

tgatttattg cgtcaagatt tgcaattaac tggtaaacag gatctgaagt atgttgtctt 1920

cgaagatgca cccgtgggca taaaggccgg caaagcaatg ggcgccatta ctgtgggtat 1980

aacatcctcg tatgacaaga gcgttttatt tgacgcagga gcagattatg tagtctgtga 2040

tttgacacag gtttccgtgg ttaagaacaa tgaaaacggt attgtcatcc aggtaaacaa 2100

ccctttgaca agggcctgag taaacaaaaa tgtgacaaaa gaacgaatat atatagatgt 2160

aaaacatatg gacaagcaaa aagtcgaatt atgtatgtca ttttaggtac tgaagaggta 2220

agattttttt tgagtttttc ttcgaagatg gttgtgtggt tatatgttaa tcttccttag 2280

cgcaaaacac ttccatcaac tgtatttcgt tggaatgctt tgtattcagt tttgtatcat 2340

tatctttaat cacaattgcg tcaggatgta a 2371

<210> 29
<211> 3061
<212> DNA
<213> Artificial Sequence

<220>
<223> HAL1-truncated AIM45

<400> 29
gatcactata tttagttcga aagttagtgt ttgttttgcc aagatatcct cgcagaaaca 60

gttttactta aagtttcagg tgttgcgtgg aatgcccctg ctaagctgtc gtgtcgcgct 120

cttccccgcg tttgtttcac attatatatc atatggcgta tgacggtatg ggtgaaaata 180

agcgtaggct ggttgtgtgt atttctctcg cactttgaaa gggaaaaata aaaatagagt 240

ctattaggaa gctcacatat gccgggaaaa attacgtaaa gcatcaaaag ggaaagaaaa 300

tatagggaaa gataaaacaa aaagcagaac ggtatcaggc attcttgttt tagcctttat 360

gtacgatatg gcgtttatta tggagaatat tgaggtattc aactatttaa tacttgctat 420

aaattctgcc aaactactaa tcattccgtt tatataactc aagaaaagag aaatacagaa 480

acaaatagat cagatgcatt tcaaagattt aggattgcat gactacactc tcaaaaactt 540

gatgtatgag aataattgct gtaaatttta tgatgccgtg gatgaaaaca acatctcata 600

tgttttaaaa tttgttccct cagatgtgac ttcggaaggg gatactttcc cattcgtgga 660

tcgctttcaa gtaaaggaag gtgttttttt ggtatattcc tcaaatgact ttggaaaaga 720

aggtacggac tactttactt atactggtag tggtggaaat gaggttcaca tctcgggcac 780

ctcttcagaa gcaggaataa aaccgcagtt tattgaaact tgccatccaa aacatcttaa 840

gcggggaaca aaagagcagg aagatataaa tagtagtacc tcaaagaaaa gtgcagttat 900

caacaatttt tcgggtgaaa aaacaccaaa tccaaggcca cagagttcca acatttcaga 960
aagagagacg tatgtcggaa tattgaacgt caaatgtaaa aataagaact catcgaaaat 1020
acgaagtgaa aaattggtaa gctccgtcat cgaaacaaag catacgccag gattggcatc 1080
tattttatcg aaagaaggca ctacatatcc gaataatgcg gacgggaaac atatcagtat 1140
cgtgaatcca tcctcaaaaa tatatcattc atcccataaa cagattgtta aaacgcctat 1200
ccctaagagt ggcctttctc caattgagag atgccctttc aatggtcaaa atattaaatg 1260
ctactcacca agaccactag atcatgaaag tccccaacgt gatttcaata ataactttca 1320
gctgagaata ctgaagagct cggtgttgca aaggagacaa tcaacacaga atagttgaaa 1380
aattcgttgg tactagcttc gggtcggtta gctgcgctgc tatgcatttg ttaatatctc 1440
catcgaattt ttgttgtttc gttcaataac tttattactt ccccccttgga ctctttcgtt 1500
ctatcgttct acaagtccag ccaaaatttt tcccctcttc cttttctttt gttcacttct 1560
tagctcactt atataattat atactgatat ttggattctt ttgttgcaaa tatgctctcc 1620
cagatttttc tgttgagatg attcatgctt tacatggatt gagcattaga gagtaactat 1680
atccaatttc gtaagacgag tatctacttt cccttgtccc cagtaacctc aggaacgtga 1740
caactacttt tcttaaactg tcaacagcca atgataccgt attcaatgca tgtcttggga 1800
ttaagccact ttgattgagt tccgtattag tagtgagaat taaatcttgc aagatataag 1860
aattactcaa cagagacgag atactttcct ttttttggtt cttttgttgt tacttggctt 1920
aatggacaaa gtctgctcgc atattgtata tgttactctt acgatgtgac tccgcccgtt 1980
tattatgact tttcggtaca tttttagggc ctcgaacgaa aatctactaa ctaaaaatta 2040
aaaacaatta aaataatcaa caaacaaaat ctagtgatat aatttactac cattaacggt 2100
aaagcagcta attgttaatt tctatgttta aatcattggc tgctgtcttg cctagagcta 2160
gcaaggcaaa gttcctccag aaaaattacg cctccacttt agctttcatt gaaagctcaa 2220
aagatggctc tgtttcaagg tcatcattga gtttattggc tgctgcacaa aagttgtcta 2280
accctatcac agctgtaatc acaggtagca aagctgaaaa aactgctgag gcgctaaaat 2340
cttcatattc atgcagcaat ttagaaaagc ttgtcatatt tgaagattca aaattagata 2400
cctgtcttcc cgaacaacta actccgttat tagtgaaact attaaaaggc ggcgactatt 2460
cacattttgt tgtctcaaac tcctctgttg gaaaaagtgt tttacctcgg gtgggtgcgc 2520
tcttggacgt ccaacctgtt tgtgaggtta ctgtaatcaa agatcctaag acctttataa 2580
ggccaattta tgcaggtaac attatttcta caatagaatg ccaggcagaa aaaaaactgt 2640
tgattattag ggcatcagct tttccaccaa ttgcagaggg tagtatggat tctgttacca 2700
ttgagaagag aactgatatt cctccttgtg acttaaatgt tacctgggtt aaaactattc 2760
ttaccaagag tgaaaggcct gaacttactt ctgcacagaa cgtggtaact ggtggaaggg 2820
cactcaagga taaggagaca tttgagaagc tattatcgcc gctagcagat gttttgcacg 2880
ctgctatagg tgccacaaga gcttctgttg ataatggact atgtgataat tctctacaaa 2940

tcggtcagac tggtaaggta gtcgcaccaa atttgtatat agccattggc gtttctggtg 3000

cagttcagca tttagcggga atgaaggatt cgaaagttat cgttgccatt aacaatgatc 3060

c 3061

<210> 30
<211> 903
<212> DNA
<213> Artificial Sequence

<220>
<223> TRP1 variant

<400> 30
caccttacgt acaatcttga tccggagctt ttcttttttt gccgattaag aattcggtcg 60

aaaaaagaaa aggagagggc caagagggag ggcattggtg actattgagc acgtgagtat 120

acgtgattaa gcacacaaag gcagcttgga gtatgtctgt tattaatttc acaggtagtt 180

ctggtccatt ggtgaaagtt tgcggcttgc agagcacaga ggccgcagaa tgtgctctag 240

attccgatgc tgacttgctg ggtattatat gtgtgcccaa tagaaagaga acaattgacc 300

cggttattgc aaggaaaatt tcaagtcttg taaaagcata taaaaatagt tcaggcactc 360

cgaaatactt ggttggcgtg tttcgtaatc aacctaagga ggatgttttg gctctggtca 420

atgattacgg cattgatatc gtccaactgc atggagatga gtcgtggcaa gaataccaag 480

agttcctcgg tttgccagtt attaaaagac tcgtatttcc aaaagactgc aacatactac 540

tcagtgcagc ttcacagaaa cctcattcgt ttattccctt gtttgattca gaagcaggtg 600

ggacaggtga acttttggat tggaactcga tttctgactg ggttggaagg caagagagcc 660

ccgaaagctt acattttatg ttagctggtg gactgacgcc agaaaatgtt ggtgatgcgc 720

ttagattaaa tggcgttatt ggtgttgatg taagcggagg tgtggagaca aatggtgtaa 780

aagactctaa caaaatagca aatttcgtca aaaatgctaa gaaataggtt attactgagt 840

agtatttatt taagtattgt ttgtgcactt gcctgcaggc cttttgaaaa gcaagcataa 900

aag 903

<210> 31
<211> 1091
<212> DNA
<213> Artificial Sequence

<220>
<223> truncated MRPL17 variant

<400> 31
gatctttact ccacccttta tttgctcata gtacataaaa ctaatctacc tctatatata 60

atttctgttt tttcctatgt tccttttttt tttttgttaa atggacatcc ttatgtattc 120

acataactac tgtgtttcaa atgtagcatt atcctattct ttattctctt tttttaccgt 180

attcctggtt ttttttcaag gtttagatga aaatgaaaat gaaaaaaaaa aaaggaaaga 240

```
aaacagttaa acaatcaata aactttatat tctacgtaat cataagcgct tatccagtac        300
catgaaggta aatttaatgt tgaaaagagg gcttgctact gcaactgcaa ctgccagttc        360
cgctcccccc aagattaaag tcggagtact actgtcaaga atccctataa ttaaatcaga        420
attaaatgaa ctagagaaaa aatactatga gtaccaatca gaactagaaa agagactaat        480
gtggacgttt ccggcatatt tttatttcaa aaagggtact gtagcagaac acaaatttct        540
atccctgcag aaaggaccta tctccaaaaa aaatggcatt tggtttccta gaggcatacc        600
ggacattaaa catggcagag aaagaagtac taagcaagaa gttaaacttt ctgatgacag        660
tacagtagca tttagcaaca atcaaaaaga gcaaagcaaa gacgatgtta ataggcccgt        720
gattcccaac gacaggataa cggaagcaga taggtcaaat gatatgaaga gccttgaaag        780
acaattgagc aggaccttat atcttttggt taaggataaa agcggtactt ggaaattccc        840
taacttcgat ctttctgatg aatctaagcc gttacacgta cacgcagaga acgaattgaa        900
attgttgagc ggtgatcaga tatacacttg gtctgtttct gctacgccca taggtgtttt        960
gcaggacgag agaaatagga ctgctgagtt tattgtgaag tcacacattt tggctggaaa       1020
atttgatttg gtggcgtcga aaaatgatgc attcgaggat tttgcttggc tgacaaaagg       1080
tgagatcagt g                                                            1091
```

## Description

BACKGROUND

1. Field

Example embodiments relate to an isolated nucleotide increasing alcohol tolerance of a host cell, a vector and a host cell which contain the same, and a method of producing alcohol using the same.

2. Description of Related Art

With globally increasing concern about the exhaustion of resources and pollution of the environment by overuse of fossil fuels, the development of novel and renewable alternative energy sources that stably and continuously produce energy is being considered. As an example of this development of alternative energy, technology for producing bio alcohol from biomass has been attracting considerable attention.

Today, first generation biofuels using saccharides such as a sugar cane or using starches such as a corn are being produced. In addition, second generation biofuels using wood sources, lignocelluloses, which are considered the most abundant, rich and renewable sources in the world, are being developed. In recent times, development of biofuels using algae has also been progressing.

Processes of producing these biofuels include pretreating biomass to facilitate to be applied to saccharification, saccharifying the pretreated biomass to convert the pretreated biomass into monosaccharides, and fermenting the monosaccharides to produce bioalcohol.

The fermentation uses biological oxidation of an organic compound using fermentation bacteria such as yeast, etc. Bacterial metabolism can occur through various different mechanisms depending on the bacterial species and environmental conditions. All heterotropic bacteria generate energy through oxidation of organic compounds such as carbohydrates (e.g., glucoses), lipids and proteins.

The general process by which bacteria metabolize suitable substrates is glycolysis, which is a sequence of reactions that converts glucose into pyruvate to generate ATP. In production of metabolic energy, the fate of pyruvate varies depending on the bacterial species and environmental conditions. There are three principle reactions of pyruvate. First, under aerobic conditions, many microorganisms will produce energy the citric acid cycle and the conversion of pyruvate into acetyl coenzyme A, catalysed by pyruvate dehydrogenase (PDH).

Second, under anaerobic conditions, certain ethanologenic organisms can carry out alcoholic fermentation by the decarboxylation of pyruvate into acetaldehyde, catalysed by pyruvate decarboxylase (PDC) and the subsequent reduction of acetaldehyde into ethanol by NADH, catalysed by alcohol dehydrogenase (ADH).

Third, pyruvate is converted into lactate through catalysis by lactate dehydrogenase (LDH).

There has been much interest in producing ethanol using either microorganisms that undergo anaerobic fermentation naturally or through the use of host cells which incorporate the pyruvate decarboxylase and alcohol dehydrogenase genes.

However, since microorganisms generally have low alcohol tolerance, the microorganisms may be damaged due to alcohol that is produced by the microorganisms if the alcohol concentration becomes high, and may die if the alcohol concentration exceeds 15%.

For this reason, conventionally, research into improving volumetric productivity of alcohol through an optimized fermentation process and improved strains of microorganisms has been conducted by industries associated with alcohol fermentation and production, to obtain economical advantages.

In recent times, technology for increasing alcohol tolerance using spt-modified strains of yeast has been developed by G. Stephanopoulos

However, as far as concerns, discovery of various gene groups involved in ethanol tolerance and discovery of a variety of novel gene groups further involved in alcohol tolerance are needed in consideration of production of second generation energy, isobutanol.

SUMMARY

Example embodiments provide strains that exhibit high viability and homeostasis by increasing alcohol tolerance of a microorganism, and thus are widely applied to alcohol fermentation through various genetic disturbances. Other example embodiments provide a method of producing bioalcohol with high volumetric productivity using strains having excellent fermenting ability and excellent fermentation maintaining ability.

In one aspect, an isolated polynucleotide encoding a polypeptide increasing alcohol tolerance and/or volumetric productivity of alcohol of a host cell is provided.

In one example, the isolated polynucleotide may be one selected from the group consisting of: a polynucleotide consisting of a base sequence having at least 90% identity to a base sequence selected from SEQ ID NOs: 1 to 8; a polynucleotide encoding a polypeptide consisting of an amino acid sequence having at least 90% identity to an amino acid sequence selected from SEQ ID NOs: 14 to 20; a polynucleotide consisting of a base sequence hybridized to a base sequence selected from SEQ IL3 NOs: 1 to 8 under stringent conditions; and a polynucleotide encoding a polypeptide consisting of an amino acid sequence hybridized to an amino acid sequence selected from SEQ ID NOs: 14 to 20 under stringent conditions.

In another aspect, a vector containing the isolated polynucleotide is provided.

In another aspect, a host cell having increased alcohol tolerance and/or volumetric productivity of alcohol when incubated in a monosaccharide-containing nutrient media is provided.

The host cell may encode a polypeptide increasing alcohol tolerance of a host cell, and exhibit overexpression of at least one isolated polynucleotide including a polynucleotide selected from the group consisting of: a polynucleotide consisting of a base sequence having at least 90% identity to a base sequence selected from SEQ ID NOs: 1 to 8; a polynucleotide encoding a polypeptide consisting of an amino acid sequence having at least 90% identity to an amino acid sequence selected from SEQ ID NOs: 14 to 20; a polynucleotide consisting of a base sequence hybridized to a base sequence selected from SEQ ID NOs: 1 to 8 under stringent conditions; and a polynucleotide encoding a polypeptide consisting of an amino acid sequence hybridized to an amino acid sequence selected from SEQ ID NOs: 14 to 20 under stringent conditions.

In another aspect, a method of producing bioalcohol using the host cell is provided. The method may include a fermentation process including incubating a host cell in a monosaccharide-containing nutrient media and producing bioalcohol.

For example, the method of producing bioalcohol may be performed by engineering a host cell to overexpress one or more isolated polynucleotides encoding a polypeptide increasing alcohol tolerance of the host cell and including one selected from the group consisting of a polynucleotide consisting of a base sequence having at least 90% identity to a base sequence selected from SEQ ID NOs: 1 to 8, a polynucleotide encoding a polypeptide consisting of an amino acid sequence having at lease 90% identity to an amino acid sequence selected from SEQ ID NOs: 14 to 20, a polynucleotide consisting of a base sequence hybridized to a base sequence selected from SEQ ID NOs: 1 to 8 under stringent conditions, and a polynucleotide encoding a polypeptide consisting of an amino acid sequence hybridized to an amino acid sequence selected from SEQ ID NOs: 14 to 20 under stringent conditions; and incubating the host cell in a monosaccharide-containing nutrient source under suitable conditions for a predetermined period of time to produce alcohol through fermentation.

## BRIEF DESCRIPTION OF THE DRAWINGS

Example embodiments are described in further detail below with reference to the accompanying drawings. It should be understood that various aspects of the drawings may have been exaggerated for clarity:

FIG. 1 shows an open reading frame (ORF) map of a 1st polynucleotide according to an example embodiment;

FIG. 2 shows an ORF map of a 2nd polynucleotide according to an example embodiment;

FIG. 3 shows an ORF map of a 3rd polynucleotide according to an example embodiment;

FIG. 4 shows an ORF map of a 4th polynucleotide according to an example embodiment;

FIG. 5 shows an ORF map of a 5th polynucleotide according to an example embodiment;

FIG. 6 shows an ORF map of a 6th polynucleotide according to an example embodiment;

FIG. 7 shows an ORF map of a 7th polynucleotide according to an example embodiment;

FIG. 8 shows an ORF map of an 8th polynucleotide according to an example embodiment;

FIG. 9 shows a result of a 5% (w/v) ethanol tolerance test in a liquid medium according to Experimental Example 1;

FIG. 10 shows a result of a 1% (w/v) isobutanol tolerance test in a liquid medium according to Experimental Example 2;

FIG. 11 shows the volumetric productivity of ethanol in a liquid medium according to Experimental Example 1;

FIG. 12 shows a result of an ethanol tolerance test in a solid medium according to Experimental Example 3;

FIG. 13 shows a result of an isobutanol tolerance test in a solid medium according to Experimental Example 4;

FIGS. 14 to 17 show results of fermentation tests in 5% ethanol and 10% glucose according to Experimental Example 5 (FIG. 14: Example 1. FIG. 15: Example 2, FIG. 16: Example 3 and FIG. 17: Example 4);

FIGS. 18 to 21 show results of fermentation tests in 5% ethanol and 20% glucose according to Experimental Example 6 (FIG. 18: Example 1; FIG. 19: Example 2; FIG. 20: Example 3; and FIG. 21; Example 4);

FIGS. 22 to 27 show results of fermentation tests according to glucose concentration gradients in Experimental Example 7 (FIGS. 22 and 23: 10% (w/v) glucose; FIGS. 24 and 25: 20% (w/v) glucose; and FIGS. 26 and 27: 30% (w/v) glucose);

FIGS. 28 to 30 show results of fermentation tests in mixed media with various glucose/galactose concentration gradients according to Experimental Example 8 (FIG. 28: 2% (w/v) glucose/2% (w/v) galactose; FIG. 29: 2% (w/v) glucose/6% (w/v) galactose; and FIG. 30:2% (w/v) glucose/8% (w/v) galactose); and

FIGS, 31 and 32 show results of ethanol tolerance tests in 15% (w/v) ethanol media using colony forming units (CFUS) according to Experimental Example 9 (FIG. 31: relative viable cell count; and FIG. 32: cell death rate).

## DETAILED DESCRIPTION OF THE EMBODIMENTS

Hereinafter, advantages, features and methods for embodying the inventive concept will be described more fully with reference to the detailed descriptions of the following example embodiments and the accompanying drawings. However, it should be understood that the inventive concept is not limited to the described example embodiments, and thus may be embodied in various forms.

In addition, it would be understood that all the numbers representing contents and conditions used in the specification and claims may be changed. Thus, unless indicated otherwise, a numeral parameter shown in the specification and accompanying claims is an approximation that may be changed according to the purpose of the inventive concept.

1. Isolated Polynucleotide

According to an example embodiment, an isolated polynucleotide encoding a protein having increased alcohol tolerance is provided.

In one example, the isolated polynucleotide may include a polynucleotide consisting of a base sequence selected from SEQ ID NOs: 1 to 8, which encodes a protein having increased alcohol tolerance. Alternatively, the isolated polynucleotide may include a polynucleotide which includes a base sequence having at least 70, 75, 80, 85, 90, 95 or 99% identity to the above-mentioned base sequences and has the above-mentioned activity. The isolated polynucleotide may include a fragment or variant of the polynucleotide or a polynucleotide hybridized to the polynucleotide under stringent conditions.

In another example, the isolated polynucleotide may be a polynucleotide encoding a polypeptide consisting of an amino acid sequence selected from SEQ ID NOs: 14 to 20 and increasing alcohol tolerance of a host cell. Alternatively, the isolated polynucleotide may include a polynucleotide encoding a polypeptide consisting of at least 70, 75, 80, 85, 90, 95 or 99% identity to the above-mentioned amino acid sequence and having the above-mentioned activity, or may include a fragment or variant of the polynucleotide or a polynucleotide encoding a polypeptide consisting of an amino acid sequence hybridized to the above-mentioned polypeptide under stringent conditions.

Therefore, examples of the isolated polynucleotide may be selected from the following:

(a) a polynucleotide consisting of a base sequence having at least 90% identity to a base sequence selected from SEQ ID NOs: 1 to 8;

(b) a polynucleotide encoding a polypeptide consisting of an amino acid sequence having at least 90% identity to an amino acid sequence selected from SEQ ID NOs: 14 to 20;

(c) a polynucleotide consisting of a base sequence hybridized to a base sequence selected from SEQ ID NOs: 1 to 8 under stringent conditions; and

(d) a polynucleotide encoding a polypeptide consisting of an amino acid sequence hybridized to an amino acid sequence selected from SEQ ID NOs: 14 to 20 under stringent conditions.

The isolated polynucleotide may be derived from yeast, for example, *Saccharomyces cerevisiae.*

Since the isolated polynucleotide encodes a protein increasing alcohol tolerance, a host cell containing the same exhibits excellent viability in high-concentration alcohol and excellent homeostasis during fermentation. Thus, when it is used in industrial alcohol fermentation, alcohol volumetric productivity may be increased.

The terms used herein have the following meanings unless there are specific descriptions. In addition, all technical and scientific terms used herein have meanings conventionally understood by those skilled in the art unless there are specific descriptions.

The term "polynucleotide" generally refers to a non-modified or modified polyribonucleotide (e.g. RNA) or polydeoxyribonucleotide (e.g. DNA). Examples of the "polynucleotide" include, but are not limited to, single- or double-stranded DNA, DNA that is a mixture of single- and double-stranded regions, single- or double-stranded RNA, RNA that is a mixture of single- and double-stranded regions, hybrid molecules including single- or double stranded DNA or RNA, or DNA or RNA that is a mixture of single- and double-stranded regions. In addition, the "polynucleotide" may include a triple-stranded region having RNA or DNA or both RNA and DNA, or may include a relatively short polynucleotide, often referred to as an oligonucleotide.

The term "isolated" means altered by a human from the natural state, that is, changed and/or removed from its original environment. For example, a polynucleotide or polypeptide originally present in a living organism is not "isolated," but the same polynucleotide or polypeptide isolated from a natural co-existing material is "isolated." Further, a polynucleotide or polypeptide introduced into a living organism by transformation, genetic engineering or other recombination techniques is "isolated," although it is present in a living organism.

The term "polypeptide" refers to a peptide or protein containing two or more amino acids linked to each other by peptide bonds or modified peptide bonds. The "polypeptide" includes short chains such as peptides, oligopeptides or oligomers, and to long chains such as proteins. The "polypeptide" may include amino acids other than the 20 gene-encoded amino acids. The "polypeptide" includes amino acid sequences modified by natural processes or chemical modification techniques known in the art. The modifications include acetylation, acylation, ADP-ribosylation, amidation, biontinylation, covalent attachment of flavin, covalent attachment of a heme moiety, covalent attachment of a nucleotide or nucleotide derivative, covalent attachment of a lipid or lipid derivative, covalent attachment of phosphotidylinositol, crosslinking, cyclization, disulfide bond formation, demethylation, formation of covalent crosslinks, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination.

The term "fragment" of a polynucleotide sequence refers to a polynucleotide sequence shorter than a reference sequence in sequence listing. The "fragment" of the polypeptide sequence is a polypeptide sequence that is shorter than the reference sequence but has substantially the same biological function or activity as a reference polypeptide.

The term "variant" refers to a polynucleotide or polypeptide that differs from, but has the same essential properties as, a reference polynucleotide or polypeptide. A typical variant of a polynucleotide differs in nucleotide sequence from the reference polynucleotide. Changes in the nucleotide sequence of the variant may or may not alter the amino acid sequence of a polypeptide encoded by the reference polynucleotide. The nucleotide changes may result in amino acid substitutions, additions, deletions, fusions and truncations in the polypeptide encoded by the reference sequence. A typical variant of a polypeptide differs in amino acid sequence from the reference polypeptide. Generally, alterations are limited so that the sequences of the reference polypeptide and the variant are closely similar overall and, in many regions, identical. A variant and a reference polypeptide may differ in amino acid sequence by one or more substitutions, insertions, or deletions in any combination. A substituted or inserted amino acid residue may or may not be one encoded by a genetic code. Typical conservative substitutions include Gly, Ala; Val, Ile, Leu; Asp, Glu; Asn, Grn; Ser, Thr; Lys, Arg; and Phe and Tyr. A variant of a polynucleotide or polypeptide may be naturally occurring such as an allele, or it may be a variant that is not known to occur naturally. Non -naturally occurring variants of polynucleotides and polypeptides may be made by mutagenesis techniques or by direct synthesis. A variant of a polypeptide may be a polypeptide having one or more post-translational modification such as glycosylation, phosphorylation, methylation and ADP ribosylation. The variant of the polynucleotide may include a splice variant, an allelic variant or a polynucleotide having single nucleotide polymorphism (SNP).

The term "stringent conditions" refers to conditions under which overnight incubation is conducted for about 2.5 hours in 6 x SSC/0.1% SDS at 42 °C, and then a filter is washed in 1.0 x SSC/0.1% SDS at 65 °C.

The term "identity" reflects a relationship between two or more polypeptide or polynucleotide sequences, determined by comparing the sequences. Generally, the "identity" refers to an exact nucleotide to nucleotide or amino acid to amino acid correspondence of the two polynucleotide or two polypeptide sequences, respectively, over the length of the sequences being compared. Methods of comparing identity and similarity of two sequences are known in the art. For example, % identity between two polynucleotides, and % identity and % similarity between two polypeptide sequences may be determined using the Wisconsin Sequence Analysis Package, version 9.1 (Devereux J. et al., Nucleic Acids Res, 12, 387-395 (1984); available from Genetics Computer Group, Madison Wisconsin, USA) such as the programs BESTFIT and GAP.

BESTFIT finds the best single region of similarity between two sequences using the "local homology" algorithm of Smith and. BESTFIT is more suitable for comparing two polynucleotides or two polypeptide sequences that are not similar in length, and assumes that a shorter sequence represents a longer portion.

In comparison, GAP aligns two sequences to find a "maximum similarity", according to the Needleman-Wunsch algorithm. GAP is more suitable for comparing sequences having approximately the same length, and expects that alignment will be made over the entire length. Preferably, the parameters "gap weight" and "length weight" used in each program are 50 and 3 for polynucleotide sequences and 12 and 4 for polypeptide sequences, respectively.

Other programs for determining identity and/or similarity between sequences include the BLAST family of programs (Altschul S. F. et al., Nucleic Acids Res., 25:389-3402 (1997); available from National Center for Biotechnology Information (NCBI) and FASTA (Pearson W.R., Methods in Enzymology, 183, 63-99 (1990)).

The term "increase in alcohol tolerance (or resistance)" means an improvement in resistance of a host to alcohol. The increase in alcohol tolerance may be observed by comparing the wild-type cell and the control cell (transformed with an empty vector) in cell growth rate in minimum inhibitory concentration (MIC), final cell density and decreased lag time.

The polynucleotide consisting of a base sequence selected from SEQ ID NOs: 1 to 8, or the polynucleotide encoding a polypeptide consisting of an amino acid sequence selected from SEQ ID NOs: 14 to 20 includes all or partial genes listed in Table 1 below.

[Table 1]

| No. of Base Sequence | No. of Amino Acid Sequence | Gene | Name |
|---|---|---|---|
| 1 | 14 | truncated MIH1 | 1st polynucleotide |
| 2 | 15 | INO1 | 2nd polynucleotide |
| 3 | 16 | DOG1 | 3rd polynucleotide |
| 4 | 17 | HAL1 | 4th polynucleotide |
| 5 | 18 | TRP1 | 5th polynucleotide |
| 6 | 19 | truncated MRPL17 | 6th polynucleotide |
| 7 | 20 | Partial fragment YLR157C-B | 7th polynucleotide |
| 8 | - | putative SPG5 promoter | 8th polynucleotide |

Hereinafter, each polynucleotide will be described in detail.

In one example, the isolated polynucleotide encoding a polypeptide increasing alcohol tolerance of a host cell may include a polynucleotide (hereinafter, referred to as a "1st polynucleotide") selected from the following:

(i) a polynucleotide consisting of a base sequence having at least 90% identity to a base sequence of SEQ ID NO: 1;

(ii) a polynucleotide encoding a polypeptide consisting of an amino acid sequence having at least 90% identity to an amino acid sequence of SEQ ID NO: 14;

(iii) a polynucleotide consisting of a base sequence hybridized to a base sequence of SEQ ID NO: 1 under stringent conditions; and

(iv) a polynucleotide encoding a polypeptide consisting of an amino acid sequence hybridized to an amino acid sequence of SEQ ID NO: 14 under stringent conditions.

In the 1st polynucleotide, the base sequence set forth in SEQ ID NO: 1 or the amino acid sequence set forth in SEQ ID NO: 14 encodes partial MIH 1 gene. That is, the 1st polynucleotide encodes partial MIH1 gene (hereinafter, referred to as "truncated MIH1") from which 126th to 555th amino acids are deleted, resulting in containing 1st to 125th amino acids.

The MIH1 gene is a cell cycle regulator, which serves as a tyrosine phosphatase involved in extension of G2 phase in a Yeast cell cycle. However, since the 126th to 555th amino acids are deleted, it is estimated that the truncated MIH1 gene will not be able to control the cell cycle, resulting in continuous cell growth.

The 1st polynucleotide may be an isolated polynucleotide selected from the following:

(i) an isolated polynucleotide consisting of base sequences having at least 90% identity to base sequences of SEQ ID NOs: 1 and 9;

(ii) an isolated polynucleotide encoding a polypeptide consisting of amino acid sequences having at least 90% identity to amino acid sequences of SEQ ID NOs: 14 and 21;

(iii) an isolated polynucleotide hybridized to the isolated polynucleotide of (i) under stringent conditions; and

(iv) an isolated polynucleotide hybridized to the isolated polynucleotide of (ii) under stringent conditions.

The isolated polynucleotide of (i) may be an isolated polynucleotide (hereinafter, referred to as a "1-1st polynucleotide) having a base sequence set forth in SEQ ID NO: 26.

The 1-1st polynucleotide has a genetic map shown in FIG. 1. Referring to FIG. 1, the 1-1st polynucleotide is derived from the 13th chromosome of *S. cerevisiae,* which includes partial MSN2 gene and partial MIH1 gene.

In the 1-1st polynucleotide, the base sequence set forth in SEQ ID NO: 9 or the amino acids sequence set forth in SEQ ID NO: 21 encodes partial MSN2 gene.

The MSN2 gene is a transcription factor expressed by cell in response to various stresses received from an external environment. The expressed MSN2 binds to a promoter region of various gene groups in a nucleus having stress response elements (STREs), which are specific recognition sites, expressing the STREs.

However, the 1-1st polynucleotide encodes partial MSN2 gene (hereinafter, referred to as "truncated MSN2") from which 1st to 48th amino acids are deleted, resulting in encoding only 656 amino acids (49th to 705th amino acids). The deleted site is an activation domain site that binds to and thus activates YAK1, resulting in stopping cell growth. When this truncated MSN2 is expressed, it is estimated that it will compete with intact MSN2 and thus inhibit an activity of the YAK1, resulting in cell growth under a stress condition (e.g. high concentration ethanol).

In another example, the isolated polynucleotide encoding a polypeptide increasing alcohol tolerance of the host cell may include a polynucleotide (hereinafter, referred to as a "2$^{nd}$ polynucleotide") selected from the following:

(i) a polynucleotide consisting of a base sequence having at least 90% identity to a base sequence of SEQ ID NO: 2;

(ii) a polynucleotide encoding a polypeptide consisting of an amino acid sequence having at least 90% identity to an amino acid sequence of SEQ ID NO: 15;

(iii) a polynucleotide consisting of a base sequence hybridized to a base sequence of SEQ ID NO: 2 under stringent conditions; and

(iv) a polynucleotide encoding a polypeptide consisting of an amino acid sequence hybridized to an amino acid sequence of SEQ ID NO: 15 under stringent conditions.

In the 2$^{nd}$ polynucleotide, the base sequence set forth in SEQ ID NO: 2 or the amino acid sequence set forth in SEQ ID NO: 15 encodes IN01 gene.

The IN01 gene is a gene encoding an inositol-1-phosphate synthase involved in syntheses of inositol phosphate and inositol-containing phospholipid. Inositol is known as an essential material for growth of a microorganism, stimulating development of the microorganism. It is also known that when IN01 is deleted, ethanol tolerance is rapidly decreased, and when inositol is excessive, ethanol tolerance is increased.

The 2$^{nd}$ polynucleotide may be an isolated polynucleotide selected from the following:

(i) an isolated polynucleotide consisting of base sequences having at least 90% identity to base sequences of SEQ ID NOs: 2, 10 any 11;

(ii) an isolated polynucleotide encoding a polypeptide consisting of amino acid sequences having at least 90% identity to amino acid sequences of SEQ ID NOs: 15,22 and 23;

(iii) an isolated polynucleotide hybridized to the isolated polynucleotide of (i) under stringent conditions; and

(iv) an isolated polynucleotide hybridized to the isolated polynucleotide of (ii) under stringent conditions.

The isolated polynucleotide of (i) may be an isolated polynucleotide (hereinafter, referred to as a "2-1$^{st}$ polynucleotide") having a base sequence set forth in SEQ ID NO: 27 having a genetic map shown in FIG. 2.

Referring to FIG. 2, the 2-1$^{st}$ polynucleotide is derived from the 10$^{th}$ chromosome of *S. cerevisiae,* which encodes IN01 gene as described above.

Further, in the 2-1$^{st}$ polynucleotide, the base sequence set forth in SEQ ID NO: 10 or the amino acid sequence set forth in SEQ ID NO: 22 encodes genes (hereinafter, referred to as "truncated VPS35"), from which 285$^{th}$ to 945$^{th}$ amino acids are deleted, resulting in encoding only 1$^{st}$ to 284$^{th}$ amino acids. The VPS35 gene serves to transport foreign proteins.

Further more, in the 2-1$^{st}$ polynucleotide, the base sequence set forth in SEQ ID NO: I 1 or the amino acid sequence set forth in SEQ ID NO: 23 encodes partial SNA3 gene (referred to as "truncated SNA3") from which 1$^{st}$ to 77$^{th}$ amino acids are deleted, encoding only the 78$^{th}$ to 134$^{th}$ amino acids. A function of the SNA3 gene is not known yet.

In another example, the isolated polynucleotide may include a polynucleotide (referred to as a "3$^{rd}$ polynucleotide") selected from the following:

(i) a polynucleotide consisting of a base sequence having at least 90% identity to a base sequence of SEQ ID NO: 3;

(ii) a polynucleotide encoding a polypeptide consisting of an amino acid sequence having at least 90% identity to an amino acid sequence of SEQ ID NO: 16;

(iii) a polynucleotide consisting of a base sequence hybridized to a base sequence of SEQ ID NO: 3 under stringent conditions; and

(iv) a polynucleotide encoding a polypeptide consisting of an amino acid sequence hybridized to an amino acid sequence of SEQ ID NO: 16 under stringent conditions.

In the 3$^{rd}$ polynucleotide, the base sequence set forth in SEQ ID NO: 3 or the amino acid sequence set forth in SEQ ID NO: 16 encodes DOG1 gene.

It is known that the DOG1 gene encodes 2-deoxyglucose-6-phophatase and confers tolerance with respect to 2-deoxyglucose when overexpressed.

The 3$^{rd}$ polynucleotide may be an isolated polynucleotide selected from the following:

(i) an isolated polynucleotide consisting of base sequences having at least 90% identity to base sequences of SEQ ID NOs: 3 and 12;

(ii) an isolated polynucleotide consisting of amino acid sequences having at least 90% identity to amino acid sequences of SEQ ID NOs: 16 and 24;

(iii) an isolated polynucleotide hybridized to the isolated polynucleotide of (i) under stringent conditions; and

(iv) an isolated polynucleotide hybridized to the isolated polynucleotide of (ii) under stringent conditions.

The isolated polynucleotide of (i) may be a polynucleotide (referred to as a "3-1$^{st}$ polynucleotide") having a base sequence set forth in SEQ ID NO: 28 having a genetic map shown in FIG. 3.

Referring to FIG. 3, the 3-1$^{st}$ polynucleotide, derived from the 8$^{th}$ chromosome of *S. cerevisiae,* encodes the DOG1 gene as described above. In the 3-1$^{st}$ polynucleotide, the base sequence set forth in SEQ ID NO: 12 or the amino acid sequence set forth in SEQ ID NO: 24 encodes partial YHRO45W gene (referred to as "truncated YHRO45W") from which 213th to 561st amino acids are deleted, encoding only 1st to 212th amino acids.

A function of the YHRO45W gene is not known yet, but the YHRO45W gene is known to encode a green fluorescent protein (GFP)-fusion protein located in a vesicle [Huh WK, et al. (2003) Global analysis of protein localization in budding yeast. Mature 425(6959):686-91].

In one example, the isolated polynucleotide may include a polynucleotide (referred to as a "4th polynucleotide") selected from the following:

(i) a polynucleotide consisting of a base sequence having at least 90% identity to a base sequence of SEQ ID NO: 4;

(ii) a polynucleotide encoding a polypeptide consisting of an amino acid sequence having at least 90% identity to an amino acid sequence of SEQ ID NO: 17;

(iii) a polynucleotide consisting of a base sequence hybridized to a base sequence of SEQ ID NO: 4 under stringent conditions; and

(iv) a polynucleotide encoding a polypeptide consisting of an amino acid sequence hybridized to an amino acid sequence of SEQ ID NO: 17 under stringent conditions.

In the 4th polynucleotide, the polynucleotide consisting of the base sequence set forth in SEQ ID NO: 4 or encoding a polypeptide consisting of the amino acid sequence set forth in SEQ ID NO: 17 encodes HAL 1 gene.

The HAL1 gene encodes cytoplasmic protein involved in halotolerance. Expression of the HAL1 gene is inhibited by Ssn6p-Tup1p and Skolp, or induced by NaC1, KCl and sorbitol via Gcn4p [refer to Marquez JA, el al. (1998) The Ssn6-Tup1 repressor complex of Saccharomyces cerevisiae is involved in the osmotic induction of HOG-dependent and -independent genes. EMBO J 17(9):2543-53; Pascual-Ahuir A, et al. (2001) The Sko1p repressor and Gcn4p activator antagonistically modulate stress-regulated transcription in Saccharomyces cerevisiae. Mol Cell Biol 21(1): 16-25].

The 4th polynucleotide may be an isolated polynucleotide selected from the following:

(i) an isolated polynucleotide consisting of base sequences having at least 90% identity to base sequences of SEQ ID NOs: 4 and 13;

(ii) an isolated polynucleotide consisting of amino acid sequences having at least 90% identity to amino acid sequences of SEQ ID NOs: 17 and 25;

(iii) an isolated polynucleotide hybridized to the isolated polynucleotide of (i) under stringent conditions; and

(iv) an isolated polynucleotide hybridized to the isolated polynucleotide of (ii) under stringent conditions.

The isolated polynucleotide of (i) may be an isolated polynucleotide (referred to as a "4-1st polynucleotide") having the base sequence set forth in SEQ ID NO: 29 having a genetic map shown in FIG. 4.

Referring to FIG. 4, the 4-1st polynucleotide, derived from the 16th chromosome of *S. cerevisiae,* encodes the HAL1 gene as described above. In the 4-1st polynucleotide, the base sequence set forth in SEQ ID NO: 13 or the amino acid sequence set forth in SEQ ID NO: 25 encodes partial AIM45 gene (referred to as "truncated AIM45") from which 313th to 345th amino acids are deleted, encoding only 1st to 31th amino acids. A function of the AIM45 gene is not clearly known yet.

In one example, the isolated polynucleotide may include a polynucleotide (referred to as a "5th polynucleotide") selected from the following:

(i) a polynucleotide consisting of a base sequence having at least 90% identity to a base sequence of SEQ ID NO: 5;

(ii) a polynucleotide encoding a polypeptide consisting of an amino acid sequence having at lease 90% identity to an amino acid sequence of SEQ ID NO: 18;

(iii) a polynucleotide consisting of a base sequence hybridized to a base sequence of SEQ ID NO: 5 under stringent conditions; and

(iv) a polynucleotide encoding a polypeptide consisting of an amino acid sequence hybridized to an amino acid sequence of SEQ ID NO: 18 under stringent conditions.

In the 5th polynucleotide, the base sequence set forth in SEQ ID NO: 5 or the amino acid sequence set forth in SEQ ID NO: 18 encodes TRP1 gene.

The TRP1 gene encodes phophoribosylanthranilate isomerase catalyzing the third step of tryptophan biosynthesis.

The 5th polynucleotide may be an isolated polynucleotide (referred to as a "5-1st polynucleotide") having the base sequence set forth in SEQ ID NO: 30 having a genetic map shown in FIG. 5.

Referring to FIG. 5, the 5-1st polynucleotide, derived from the 4th chromosome of *S. cerevisiae,* encodes the TRP 1 gene as described above.

In another example, the isolated polynucleotide may include a polynucleotide (referred to as a "6th polynucleotide") selected from the following:

(i) a polynucleotide consisting of a base sequence having at least 90% identity to a base sequence of SEQ ID NO: 6;

(ii) a polynucleotide encoding a polypeptide consisting of an amino acid sequence having at least 90% identity to an amino acid sequence of SEQ ID NO 19;

(iii) a polynucleotide consisting of a base sequence hybridized to a base sequence of SEQ ID NO 6 under stringent conditions; and

(iv) a polynucleotide encoding a polypeptide consisting of an amino acid sequence hybridized to an amino acid sequence of SEQ ID NO 19 under stringent conditions.

In the 6$^{th}$ polynucleotide, the base sequence set forth in SEQ ID NO: 6 or the amino acid sequence set forth in SEQ ID NO: 19 encodes partial MRPL17 gene (referred to as "truncated MRPL17"), encoding only 262 amino acids by deletion of the 263$^{rd}$ amino acid.

The MRPL 17 gene encodes a mitochondrial ribosomal protein.

The 6$^{th}$ polynucleotide may be an isolated polynucleotide (referred to as a "6-1$^{st}$ polynucleotide) having the base sequence set forth in SEQ ID NO: 31 having a genetic map shown in FIG. 6.

Referring to FIG. 6, the 6-1$^{st}$ polynucleotide, derived from the 14$^{th}$ chromosome of *S. cerevisiae,* encodes the truncated MRPL 17.

In one example, the isolated polynucleotide may include a polynucleotide (referred to as a "7$^{th}$ polynucleotide") selected from the following:

(i) a polynucleotide consisting of a base sequence having at least 90% identity to a base sequence of SEQ ID NO: 7;

(ii) a polynucleotide encoding a polypeptide consisting of an amino acid sequence having at least 90% identity to an amino acid sequence of SEQ ID NO: 20;

(iii) a polynucleotide consisting of a base sequence hybridized to set forth in SEQ ID NO: 7 under stringent conditions; and

(iv) a polynucleotide encoding a polypeptide consisting of an amino acid sequence hybridized to an amino acid sequence of SEQ ID NO: 20 under stringent conditions.

In the 7$^{th}$ polynucleotide, the base sequence set forth in SEQ ID NO: 7 or the amino acid sequence set forth in SEQ ID NO: 20 encodes partial YLR157C-B or YLRCTyl-1 gene. The 7$^{th}$ polynucleotide is referred to as "partial fragment YLR157C-B."

The YLR157C-B gene is a transposable element gene, which includes retrotransposon TYA Gag and TYB Pol genes. The YLRCTy1-1 gene is a long terminal repeat (LTR) retrotransposon, which includes co-transcribed TYA Gag and TYB Pol genes and encodes a protein involved in the structure and function of a virus-like particle [refer to Kim JM, et al, (1998) Transposable elements and genome organization: a comprehensive survey of retrotransposons revealed by the complete Saccharomyces cerevisiae genome sequence. Genome Res 8(5):464-78]. These genes are involved in DNA-directed DNA polymerase activity, peptidase activity, protein binding, ribonuclease activity, RNA binding, and RNA-directed DNA polymerase activity.

The 7$^{th}$ polynucleotide has a genetic map shown in FIG. 7. Referring to FIG. 7, the 7$^{th}$ polynucleotide, derived from the 12$^{th}$ chromosome of *S. cerevisiae,* encodes YLR157C-B and YLRCTy1-1.

In one example, the isolated polynucleotide may include a polynucleotide (referred to as an "8$^{th}$ polynucleotide") selected from the following:

(i) a polynucleotide consisting of a base sequence having at least 90% identity to a base sequence of SEQ ID NO: 8; and

(ii) a polynucleotide consisting of a base sequence hybridized to a base sequence of SEQ ID NO: 8 under stringent conditions.

The 8$^{th}$ polynucleotide has a genetic map shown in FIG. 8. Referring to FIG. 8, the 8$^{th}$ polynucleotide is derived from the 13$^{th}$ chromosome of *S. cerevisiae,* and the base sequence of SEQ ID NO: 8 encodes a promoter site of SPG5 gene. The 8$^{th}$ polynucleotide is referred to as a "putative SPG5 promoter."

The SPG5 gene encodes a protein necessary for growing a microorganism at a high temperature in a stationary phase, and does not require a non-fermentable carbon source for growth.

The sequences and names of the isolated 1$^{st}$ to 8$^{th}$ polynucleotides and their examples, the 1-1$^{st}$ to 6-1$^{st}$ polynucleotides, which are described above, are shown in Table 2.

[Table 2]

| Name | Base Sequence | Gene | Name | Base Sequence | A. A. Sequence |
|---|---|---|---|---|---|
| 1-1$^{st}$ polynucleotide | 26 | truncated M1H1 | 1$^{st}$ polynucleotide | 1 | 14 |
| | | truncated MSN2 | - | 9 | 21 |

(continued)

| Name | Base Sequence | Gene | Name | Base Sequence | A. A. Sequence |
|---|---|---|---|---|---|
| 2-1st polynucleotide | 27 | INO1 | 2nd polynucleotide | 2 | 15 |
| | | truncated VPS35 | - | 10 | 22 |
| | | truncated SNA3 | - | 11 | 23 |
| 3-1st polynucleotide | 28 | DOG1 | 3rd polynucleotide | 3 | 16 |
| | | truncated YHR045W | - | 12 | 24 |
| 4-1st polynucleotide | 29 | HAL1 | 4th polynucleotide | 4 | 17 |
| | | truncated AIM45 | - | 13 | 25 |
| 5-1st polynucleotide | 30 | TRP1 | 5th polynucleotide | 5 | 18 |
| 6-1st polynucleotide | 31 | truncated MRPL17 | 6th polynucleotide | 6 | 19 |
| 7th polynucleotide | 7 | Partial fragment YLR157C-B | - | 7 | 20 |
| 8th polynucleotide | 8 | putative SPG5 promoter | - | 8 | - |

The isolated polynucleotides encode proteins increasing alcohol tolerance of host cells.

The alcohol tolerance may be determined by a specific growth rate in minimum inhibitory concentration (MIC) of a host cell, a colony forming unit (CFU), a final cell density, or a decreased rate of lag time.

In one example, the alcohol tolerance may be expressed as the specific growth rate in the MIC, where the "specific growth rate" may be expressed by the following Equation (1), representing a cell growth rate per unit time.

$$\text{specific growth rate } (h^{-1}) = \frac{1}{x}\,\frac{d[X]}{dt} \qquad (1)$$

In Equation (1), x is a cell concentration (g/L) and t is time.

Here, the MIC means a minimum concentration of a material inhibiting growth and survival of at least 99% of existing microbial colonies, that is, a minimum concentration causing the induction of apoptosis. For example, for wild-type *S. cerevisiae,* the MIC may be about 5% for ethanol or about 1% for isobutanol.

Accordingly, when the polynucleotide is overexpressed in a host cell for alcohol fermentation, usually in fermentation yeast, the alcohol tolerance is increased.

Examples of the alcohol may include ethylene glycol, propylene glycol, polyethylene glycol, polypropylene glycol, poly(ethylene glycopropylene glycol), 1,3-propanediol, 1,2-butanediol, 2,3-butanediol, 1,4-butanediol, 1,6-hexanediol, pinacol, glycerol, neopentylglycol, pentaerythritol, mezo-hydrobenzoin, 1,2-cyclopentanediol, 1,2-cyclohexanediol, methanol, ethanol, isopropanol, n-propanol, n-butanol, isobutanol, sec-butanol, tert-butanol, n-pentanol, isopentanol, tert-pentanol, cyclopentanol, cyclohexanol, n-hexanol, n-heptanol, n-octanol, n-nonanol, n-decanol, phenoxyethanol, benzylalcohol, diphenyl carbinol, tetraphenylcarbinol, and mixtures thereof.

The alcohol may also be ethanol or isobutanol.

The various polynucleotides described herein may be recombinant polynucleotides. The recombinant polynucleotides may be synthetic polynucleotides or other polynucleotides engineered *in vitro.* The recombinant polynucleotide may be used to produce gene products encoded thereby in cells or other biological systems. For example, a cloned polynucleotide may be inserted into a suitable expression vector (e.g., a plasmid), and then the plasmid may be used to transform the suitable host cell. The host cell containing the recombinant polynucleotide is referred to as a "recombinant host cell." When a gene is expressed in the recombinant host cell, a "recombinant protein" may be produced. The recombinant polynucleotide may have non-coding regions (or sequences) (e.g., a promoter, a replication origin, a ribosome-binding site, etc.).

2. Vector

According to another example embodiment, a vector containing the isolated polynucleotide is provided.

The term "vector" refers to a construct having a polynucleotide sequence operably linked to an expression regulatory sequence. The term "operably linked" refers to a functional association between two parts, so that the function of one part (e.g., capability of regulating transcription) is affected by the action of the other part (e.g., transcription of a sequence).

Accordingly, when a polynucleotide expression regulatory sequence (e.g., a promoter or other transcription regulatory sequences) is linked to a desired polynucleotide sequence (e.g., natural or recombinant polynucleotide) by functional connection, the polynucleotide is operably linked to the expression regulatory sequence, which directs the transcription of the polynucleotide.

The expression regulatory sequence or promoter is an expression regulatory sequence directing the transcription of the polynucleotide, which may be an extrinsic or intrinsic polynucleotide. The promoter has a nucleic acid sequence adjacent to a transcription start site such as a polymerase-binding site. In addition, the promoter may include a terminal enhancer or repressor element.

Available vectors include, but are not limited to, bacteria, plasmids, phages, cosmids, episomes, viruses and insertable DNA fragments. The plasmid is a circular double-stranded DNA loop, which is capable of inserting an additional DNA fragment into a host genome.

The vector may produce a protein or peptide encoded by a polynucleotide described herein by introduction into a host cell.

Promoters suitable for yeast include, but are not limited to, GAPDH, PGK, ADH, PHO5, GAL1 and GAL10. The vector may also include an additional regulatory sequence. Examples of the suitable regulatory sequences may include a Shine-Dalgarno sequence found in the replicase gene of phage MS-2, and a Shine-Dalgarno sequence found in cII of bacteriophage λ. Moreover, the expression vector may include a suitable marker that may be used to select transfected host cells.

Examples of the vectors capable of being expressed in fermentation microorganisms, usually yeast, and of genetic recombination include, but are not limited to, 2 micron, pBM272, pBR322-6, pBR322-8, pCS19, pDW227, pDW229, pDW232, pEMBLYe23, pEMBLYe24, pEMBLYi21, pEMBLYi22, pEMBLYi32, pEMBLYr25, pFL2, pFL26, pFL34, pFL35, pFL36, pFL38, pFL39, pFL40, pFL44L, pFL44S, pFL45L, pFL45S, pFL46L, pFL46S, pFL59, pFL59+, pFL64-, pFL64+, pG6, pG63, pGAD10, pGAD424, pGBT9, pGK12, pJRD171, pKD1, pNKY2003, pNKY3, pNN414, pON 163, pON3, pPM668, pRAJ275, pRS200, pRS303, pRS304, pRS305, pRS306, pRS313, pRS314, pRS315, pRS316, pRS403, pRS404, pRS405, pRS406, pRS413, pRS414, pRS415, pRS416, pRS423, pRS424, pRS425, pRS426, pRSS56, pSG424, pSKS104, pSKS105, pSKS106, pSZ62, pSZ62, pUC-URA3, pUT332, pYAC2, pYAC3, pYAC4, pYAC5, pYAC55, pYACneo, pYAC-RC, pYES2, pYESHisA, pYESHisB, pYESHisC, pYEUra3, rpSE937, YCp50, YCpGAL0, YCpGAL1, YCplac111, YCplac22, YCplac33, YDp-H, YDp-K, YDp-L, YDp-U, YDp-W, YEp13, YEp213, YEp24, YEp351, YEp352, YEp353, YEp354, YEp355, YEp356, YEp356R, YEp357, YEp357R, YEp358, YEp358R, YEplac112, YEplac181, YEplac195, YIp30, YIp31, YIp351, YIp352, YIp353, YIp354, YIp355, YIp356, YIp356R, YIp357, YIp357R, YIp358, YIp358R, YIp5, YIplac128, YIplac204, YIplac211, YRp12, YRp17, YRp7, pAL19, paR3, pBG1, pDBlet, pDB248X, pEA500, pFL20, pIRT2, pIRT2U, pIRT2-CAN1, pJK148, pJK210, pON163, pNPT/ADE1-3, pSP1, pSP2, pSP3, pSP4, pUR18, pUR19, pZA57, pWH5, pAPT1, pCHY21, pEVP11, REP1 ,REP3, REP4, REP41, REP42, REP81, REP82, RIP, REP3X, REP4X, REP41X, REP81X, REP42X, REP82X, RIP3X/s, RIP4X/s, pYZ1N, pYZ41N, pYZ81N, pSLF101, pSLF102, pSLF104, pSM1/2, p2UG, pART1/N795, and pYGT. In one example, the vector may be plasmid pRS424.

3. Host Cell

In still anther example embodiment, a host cell capable of producing alcohol when incubated in a monosaccharide-containing nutrient source, and having one or more kinds of overexpressed polypeptides increasing alcohol tolerance of the host cell is provided. Alternatively, the host cell may be increased in monosaccharide uptake rate when incubated in a monosaccharide-containing nutrient source, and capable of being grown in a minimal medium.

Due to high alcohol tolerance, this host cell can survive at a high rate even in high concentration alcohol when incubated in a monosaccharide-containing nutrient source such as glucose or galactose. Thus, the host cell can have tolerance to various inhibitors inhibiting production of fermentation products in high-capacity industrial fermentation processes, and prevent a cell growth inhibition phenomenon in high concentration ethanol. As a result, it is very economical since alcohol production yield may be increased and availability of the host cell may be increased.

The polypeptide may be encoded by an isolated polynucleotide including one selected from the group consisting of (a) a polynucleotide consisting of a base sequence having at least 90% identity to a base sequence selected from SEQ ID NOs: 1 to 8; (b) a polynucleotide encoding a polypeptide consisting of an amino acid sequence having at least 90% identity to an amino acid sequence selected from SEQ ID NOs: 14 to 20; (c) a poly-nucleotide consisting of a base sequence hybridized to a base sequence selected from SEQ ID NOs: 1 to 8 under stringent conditions; and (d) a polynucleotide encoding a polypeptide consisting of an amino acid sequence hybridized to an amino acid sequence selected from SEQ ID NOs: 14 to 20 under stringent conditions.

In one example, the polypeptide may include an amino acid sequence having at least 90% identity to an amino acid sequence of SEQ ID NO: 14, or an amino acid sequence hybridized to an amino acid sequence of SEQ ID NO: 14 under stringent conditions.

In another example, the polypeptide may include an amino acid sequence having at least 90% identity to an amino acid sequence of SEQ ID NO: 15, or an amino acid sequence hybridized to an amino acid sequence of SEQ ID NO: 15 under stringent conditions.

In another aspect, the polypeptide may be encoded by a polynucleotide consisting of a base sequence having at least 90% identity to a base sequence of SEQ ID NO: 26 or 27.

The host cells are capable of producing alcohol when incubated in a monosaccharide-containing nutrient source, and may be, but are not limited to, bacteria, fungi or yeasts. The host cell may also provide a suitable cell environment for expressing the polynucleotide described herein.

Examples of the host cells may include, but are not limited to, *Saccharomyces cerevisiae, Klebsiella oxytoca P2, Brettanomyces curstersii, Saccharomyces uvzrun, Candida brassicae, Sarcina ventriculi, Zymomonas mobilis, Kluyveromyces marxianus* IMB3, *Clostridium acetobutylicum, Clostridium beijerinckii, Kluyveromyces fragilis, Brettanomyces custersii, Clostriduim aurantibutylicum* and *Clostridium tetanomorphum.*

The host cell may also be yeast, which may be selected from the group consisting of the genera *Saccharomyces, Pachysolen, Clavispora, Kluyveromyces, Debaryomyces, Schwanniomyces, Candida, Pichia,* and *Dekkera,* but the inventive concept is not limited thereto.

The host cell described herein may exhibit at least 1%, 2%, 5%, 8%, 10%, 12%, 15% or 20% increase in the specific growth rate ($h^{-1}$), compared to the wild-type *S. cerevisiae* in MIC. In this case, the MIC may be 5% for ethanol or 1% for isobutanol.

In another aspect, the host cell may exhibit at least 5%, 10%, 15%, 20%, 25%, 30%, 35%, 40%, 45% or 50% increase in volumetric productivity of ethanol (g/L/h), compared to the wild-type *S. cerevisiae* under the same conditions for incubation. In this case, the ethanol volumetric productivity refers to time for producing the maximum concentration of ethanol by consuming given substrates.

Alternatively, the host cell may exhibit at least 5%, 10%, 15%, 20%, 25%, 30%, 35%, 40%, 45% or 50% increase in specific ethanol production rate (g ethanol/g dry cell/h), compared to the wild-type *S. cerevisiae* under the same conditions for incubation. In this case, the specific ethanol production rate refers to a rate of consuming given substrates to convert into ethanol for a unit time per unit cell.

The exemplary host cell is an overexpressed strain exhibiting an enhancement in expression of a predetermined polypeptide. Here, "enhancement" means an increase in intracellular activity or concentration of a protein encoded by a polynucleotide.

By overexpression, the activity or concentration of a polypeptide is increased by 10%, 25%, 50%, 75%, 100%, 150%, 200%, 300%, 400% or 500% and as much as 1000% or 2000%, compared to that of a polypeptide present in the wild-type strain, which may be the strain of *S. cerevisiae.*

In one example, the overexpression of the polypeptide may be achieved by increasing the number of copies of a gene encoding a corresponding protein. A gene or gene construct may be present in a plasmid replicable in multi-copies, or integrated into a chromosome to amplify

In another example, overexpression may be achieved by regulating the gene encoding the corresponding protein through control of a gene regulatory sequence, which is not naturally present in the gene such that it is recombinantly inserted into the gene. For example, overexpression may be brought about through transformation of a promoter, a regulatory region or a ribosome-binding site in a constructive gene. Overexpression may also be achieved using a gene encoding a transformed protein having a specific activity which is higher than a wild-type protein of the host cell, or an allele thereof, or by changing a composition of media and an incubation process.

In addition, overexpression may be achieved by methods well known in the art [refer to Eikmanns et al. (Gene 102, 93-98 (1991)); EP 0 472 869; US 4,601,893; LaBarre et al. (Journal of Bacteriology 175, 1001-1007 (1993)); WO 96/15246, the teachings of which are incorporated herein in its entirety.]

Meanwhile, various methods of introducing the polynucleotide or vector into the host cell are well known in the art [refer to Molecular Cloning: A Laboratory Manual, 2nd Edition, Sambrook et al. Cold Spring Harbor Laboratory Press, (1989)].

For example, the methods include calcium phosphate transfection, DEAE-dextran-mediated transfection, transvection, microinjection, cationic lipid-mediated transfection, electrophoration, transduction, scrape loading, ballistic transduction or transfection. Further, for these methods, various expression systems including chromosomes, episomes and virus-derived systems, bacterial plasmids, bacteriophages, transposons, enzyme episomes, insertion elements, enzyme chromosome elements and virus-derived vectors may be used.

The expression systems may regulate expression and also include a regulatory region. In general, to produce a polypeptide in a host cell, all systems or vectors which can maintain, multiply or express the polynucleotide, may be used. The exemplary overexpression method is a method of using a plasmid vector.

In another aspect, the host cells according to the inventive concept may be strains exhibiting excellent alcohol tolerance, which was deposited in Genebank of Korea Research Institute of Bioscience and biotechnology (KRIBB; Yuseong-gu, Daejeon, Korea) on 16 March, 2009. Accession numbers, names and names of isolated polynucleotides included in respective host cells are shown in Table 3.

[Table 3]

| Accession No. | Name | Isolated Polynucleotide |
|---|---|---|
| KCTC11476BP | *S.cerevisiae* CEN.PK2-1D/pRS424-MSN2/MIH1 | 1-1$^{st}$ polynucleotide |
| KCTC11477BP | *S.cerevisiae* CEN.PK2-1D/pRS424-INO1 | 2-1$^{st}$ polynucleotide |
| KCTC11478BP | *S.cerevisiae* CEN.PK2-1D/pRS424-DOG1 | 3-1$^{st}$ polynucleotide |
| KCTC11479BP | *S.cerevisiae* CEN.PK2-1D/pRS424-HAL1 | 4-1$^{st}$ polynucleotide |
| KCTC11480BP | *S.cerevisiae* CEN.PK2-1D/pRS424-TRP1 | 5-1$^{st}$ polynucleotide |
| KCTC11481BP | *S.cerevisiae* CEN.PK2-1D/pRS424-MRPL17 | 6-1$^{st}$ polynucleotide |
| KCTC11482BP | *S.cerevisiae* CEN.PK2-1D/pRS424-YLR157C-B | 7$^{th}$ polynucleotide |
| KCTC11483BP | *S.cerevisiae* CEN.PK2-1D/pRS424-SPG5p | 8$^{th}$ polynucleotide |

4. Method of Producing Bioalcohol

According to another example embodiment, a method of producing bioalcohol by incubating the host cell in a monosaccharide-containing nutrient source and producing alcohol through fermentation is provided.

Fermentation, as expressed by the following reaction formulae, is the conversion of monosaccharides produced by saccharification into alcohol through fermentation by microorganisms.

$$C_6H_{12}O_6 \rightarrow 2C_2H_5OH + 2CO_2 \qquad (2)$$

$$3C_5H_{10}O_5 \rightarrow 5C_2H_5OH + 5CO_2 \qquad (3)$$

In one example, the method of producing bioalcohol may include: engineering a host cell to overexpress one or more isolated polynucleotides encoding a polypeptide increasing alcohol tolerance of the host cell and including one selected from the group consisting of (a) a polynucleotide consisting of a base sequence having at least 90% identity to a base sequence selected from SEQ ID NOs: 1 to 8, (b) a polynucleotide encoding a polypeptide consisting of an amino acid sequence having at least 90% identity to an amino acid sequence selected from SEQ ID NOs: 14 to 20, (c) a polynucleotide consisting of a base sequence hybridized to a base sequence selected from SEQ ID NOs: 1 to 8 under stringent conditions, and (d) a polynucleotide encoding a polypeptide consisting of an amino acid sequence hybridized to an amino acid sequence selected from SEQ ID NOs: 14 to 20 under stringent conditions; and incubating the host cell in a monosaccharide-containing nutrient source under conditions suitable for producing alcohol, and producing alcohol through fermentation.

The engineering may be performed by any method of overexpressing one or more polypeptides listed above, for example, a method of using an expression vector such as a plasmid. For example, the engineering may be performed by inserting a polynucleotide encoding the polypeptide into a vector, amplifying the vector, and inserting the vector into the host cell.

The host cell may be a yeast cell, which is derived from *S. cerevisiae.*

The monosaccharide may include at least one selected from the group consisting of glucose, galactose, galactose derivatives, 3,6-anhydrogalactose, fucose, rhamnose, xylose, glucuronic acid, arabinose and mannose, or a mixture of glucose and galactose.

The monosaccharide may be a hydrolyte of sugar biomass, woody biomass or algae biomass.

The bioalcohol is a fuel produced from biomass, which may be, but is not limited to, ethanol, propanol, isopropanol, butanol, isobutanol, acetone, ethylene, propylene, fatty acid methyl ester, or a mixture thereof.

In one example, before fermentation, saccharification may be required to produce a monosaccharide-containing nutrient source. The saccharification is a hydrolysis operation of a biomass or polysaccharide into monosaccharides using a hydrolysis catalyst such as sulfuric acid, or a hydrolase.

Meanwhile, the saccharification and fermentation may be performed in separate reaction vessels through separate hydrolysis and fermentation (SHF) processes, or in one reaction vessel through a simultaneous saccharification and fermentation (SSF) process.

The SHF process may be performed under optimized conditions for the respective saccharification and fermentation, but may create inhibition of enzymatic hydrolysis between an intermediate product and a final product. Thus, more enzymes are needed to overcome this problem, which is uneconomical. For example, as an intermediate product, cellobiose, is converted into a final product, glucose, in the saccharification of cellulose, glucoses are accumulated, thereby inducing inhibition of the hydrolysis between the intermediate produce and the final product, resulting in terminating the reaction.

In comparison, in the SSF process, as soon as glucose is produced in saccharification, yeast consumes the glucose through fermentation and thus glucose accumulation in a reaction vessel can be minimized. As a result, inhibition driven by a final product, which can occur in the SHF process, can be prevented, and hydrolysis mediated by a hydrolase (enzyme) can be enhanced. Further, the SSF process can reduce production costs due to low equipment costs and low input of enzyme, and also lessen a risk of contamination due to ethanol present in the reaction vessel.

Conditions for the fermentation are not particularly limited, and the fermentation may be performed by stirring under conditions including: an initial glucose concentration of about 2 to about 30% (w/v), a temperature of about 25 to about 37°C, pH of about 5.0 to about 8.0, and a stirring rate of about 100 to about 250 rpm.

Additional operations and/or other processes may be selected by those skilled in the art as occasion demands. For example, the operations or processes may include pretreatment of biomass through grinding or hydrolysis to be suitable for saccharification, or purification of a fermented solution yielded by the fermentation according to the method known in the art.

5. Method of selecting gene exhibiting alcohol tolerance

According to another example embodiment, a method of selecting a gene exhibiting an increase in alcohol tolerance of a yeast cell when overexpressed is provided, which includes the following operations.

**Operation A:** A yeast genomic library (e.g., *S. cerevisiae*) is constructed using a multi-copy plasmid.

In operation A, the method of constructing the genomic library is not limited, and may be performed by (i) digesting genomic DNA of the wild-type strain of *S. cerevisiae* using restriction enzymes, (ii) introducing a digested DNA fragment into a multi-copy plasmid, and (iii) amplifying the plasmid. In operation A, the yeast may be *S. cerevisiae* CEN. PK2-1D, and the multi-copy plasmid may be pRS424.

**Operation B:** The genomic library constructed in operation A is transformed into a yeast cell, thereby constructing a library of the transformed yeast (referred to as the "test strain") in which all genes are overexpressed. The transformation operation may be performed by a conventional method [refer to Ito, H., Y. Fukuoka, K. Murata, A. Kimura (1983) Transformation of intact yeast cells treated with alkali cations, J. Bacteriol. 153, 163-168, the teachings of which are incorporated herein in its entirety].

**Operation C:** After confirming MIC of the test strain, the cells of the test strain are plated and incubated on agar plates containing various isobutanol gradients. Subsequently, cells grown in a relatively high concentration are selected, resulting in a library stock. The alcohol MIC of the test strain may be about 5% for ethanol or about 1% for isobutanol.

**Operation D:** A liquid SC minimal medium containing isobutanol in the MIC is inoculated with the library stock, and followed by serial subculture in a fresh medium having the same isobutanol concentration to enrich the culture with a lower concentration of inoculation. The serial subculture may be repeated 5 to 10 times. The minimal medium may contain 100 to 300 g/L of glucose, and/or 20 to 80 g/L of galactose. After the enrichment, a predetermined amount of the culture is diluted and plated on an agar plate containing isobutanol in the MIC for incubation. Then, big colonies are selected from the plate.

**Operation E:** Alcohol tolerance tests are performed on the selected big colonies, so that a strain exhibiting excellent alcohol tolerance is selected. Plasmids are isolated from the selected transformed yeast cells, and then a yeast gene sequence introduced into the isolated plasmid is confirmed using a known gene sequence at both sides of restriction enzymes used for cloning. In this case, the gene sequence may be confirmed using Gel documentation (gel doc) or Hydra.

In one example, the selection method may further include the following operations after operation E.

**Operation F:** Both terminal sequences of the insert introduced into the plasmid are compared to the yeast gene sequence to confirm an exact gene introduced into the multi-copy plasmid.

**Operation G:** The plasmid containing the confirmed gene is transformed into a yeast cell again to confirm if an alcohol tolerance effect is caused by disturbance of genes contained in the isolated plasmid.

Herein, by the above-described method, a total of 8 genes involved in alcohol tolerance were discovered.

The inventive concept will be described in more detail below with reference to various examples.

**[Construction Example 1]**

1-1. Construction of Genomic library

To construct a genomic library of CEN.PK2-1D of *S. cerevisiae* (*MATalpha; ura3-52; trp1-289; leu2-3_112;*

*his3 D1; MAL2-8C; SUC2*), genomic DNA is fragmented by sonication. Then, fragments having sizes of 2 to 4 kb are selected on an agarose gel.

Subsequently, a multi-copy plasmid (pRS424) is digested with restriction enzymes, BamHI, and followed by a fill-in reaction to create blunt ends.

The selected fragment is inserted into the blunt-ended pTS424 by ligation using T4 DNA ligase. The plasmid containing the isolated polynucleotide constructed as described above is transformed into *E. coli* and then amplified to construct the genomic DNA library.

1-2. Construction of Transformed Yeast Library

CEN.PK2-1D strains of *S. cerevisiae* are incubated in YPD (containing 10g of Yeast extract/L, 20g of Peptone/L, 20g of Dextrose/L) liquid media. Subsequently, the plasmid constructed in Construction Example 1-1 is transformed into the CEN.PK2-1D strain using an Alkali-Cation Yeast Kit (MP Biomedicals), resulting in a transformed yeast library.

1-3. Preparation of Library Stock

The transformed strains (referred to as the "test strain") are plated on solid media for incubation for about 48 hours at about 30 °C. After that, colonies grown on the plates are harvested, resulting in a library stock. The solid minimal media contains 6.7 g/L of YNB, 20 g/L of glucose, 6.2 mg/L of CSM-Leu, 0.01%(w/v) Leucine, and 0.2%(w/v) Uracil without Tryptophan, respectively, sterilizing the media with 2%(w/v) agar at high temperature, and pouring them into big agar plates (SPL Co.).

1-4. Sequence Analysis and Preparation of Host cell

Strains exhibiting excellent alcohol tolerance are selected from the selected colonies through an ethanol tolerance test according to the following protocol. Plasmids are isolated from the selected 8 strains using a Zymoprep kit (Zymo research). To analyze a sequence of the isolated polynucleotide contained in the plasmid, a sequence of the cloned gene is analyzed using a sequencing primer prepared based on a sequence of the cloned plasmid gene.

[Protocol for Ethanol Tolerance Test]

Strains are incubated in a 4-baffle flask (250ml) containing a minimal medium in the final volume of 50 ml for 2 days at 30 °C. 1 ml each of the culture for 150 g/L ethanol viability test and 2 ml of the culture for 170 g/L ethanol viability test are centrifuged, and then pellets are washed with distilled water. The pellets are re-centrifuged, and then suspended in respective 15 ml conical tubes (SPL) containing 20 g/L glucose minimal medium (SC-Trp) in the final volume of 5 ml for incubation. From each culture, samples, taken by at least 100 μl up to 500 μl every 3 hours, are streaked on solid minimal media diluted in moderation for 2-day incubation at a stirring rate of 200 rpm at 30 °C.

[Examples 1-8]

8 strains are selected by the selection according to Construction Example 1, and 8 plasmids are successfully isolated from each strain. A sequence of an isolated polynucleotide contained in each plasmid is confirmed. To confirm effects of overexpression of these sequences, the isolated plasmids are re-introduced into *S. cerevisiae* CEN.PK2-1D parent strain using an EZ-Yeast Transformation Kit (MP Biomedicals), resulting in host cells of Examples 1 to 8. SEQ ID NOs and names of the isolated polynucleotides included in the respective plasmids introduced into the host cells of Examples 1 to 8 are shown in Table 4.

[Table 4]

| Example | SEQ ID NO: | Name |
|---|---|---|
| 1 | 26 | 1-1st polynucleotide |
| 2 | 27 | 2-1st polynucleotide |
| 3 | 28 | 3-1st polynucleotide |
| 4 | 29 | 4-1st polynucleotide |
| 5 | 30 | 5-1st polynucleotide |
| 6 | 31 | 6-1st polynucleotide |
| 7 | 7 | 7th polynucleotide |
| 8 | 8 | 8th polynucleotide |

[Experimental Example 1: Cell Growth Rate in 5%(w/v) Ethanol Liquid Medium]

5% (w/v) ethanol-containing minimal medium is inoculated with strains of Examples 1 to 8 for stationary culture at 30 °C. For the stationary culture, a 15 ml falcon tube and a minimal medium (SC medium) are used. Initial inoculation is carried out using an overnight culture at low cell density ($OD_{600}$: about 0.05) in the total volume of about 5 ml.

Every 12 hours, samples are taken from each culture, and cells are isolated and washed for measurement of optical density to analyze a cell growth rate. The optical density (OD) is measured using a UV spectrophotometer (A600).

The results are shown in FIG. 9. It can be seen from FIG. 9 that all host cells of Examples 1 to 8 show higher cell growth rates than the control group, wild-type yeast.

[Experimental Example 2: Cell Growth Rate and Ethanol Volumetric Productivity in 1% (w/v) Isobutanol-Containing Liquid Medium]

A cell growth rate is analyzed by the same method described in Experimental Example 1, except for using a culture medium containing 1% (w/v) isobutanol instead of 5%(w/v) ethanol, and the results are shown in FIG. 10.

It can be seen from FIG. 10 that all host cells of Examples 1 to 8 show higher cell growth rates than the control group, wild-type yeast.

In addition, ethanol volumetric productivity is analyzed by gas chromatography (GC), and the results are shown in FIG. 11. It can be seen that all host cells of Examples 1 to 8 show significant increases in ethanol volumetric productivity, compared to the control group, the wild-type yeast.

[Experimental Example 3: Viability according to Ethanol Concentration in Solid Medium]

The strains of Examples 1 to 8 are incubated in minimal solid media (SC media) containing ethanol in a concentration of 0, 1, 2, 3, 4 and 5%(w/v) and 2%(w/v) glucose, respectively. Cells are diluted three times by a fifth and then patched on a plate from left (standard: OD600=1) to right sides. The results are shown in FIG. 12.

It can be seen from FIG. 12 that all host cells of Examples 1 to 8 show increases in viability, compared to the control group, the wild-type yeast. Particularly, the yeast groups into which the isolated polynucleotides of Examples 1, 2, 4, 7 and 8 are introduced show strong tolerance to ethanol.

[Experimental Example 4: Viability according to Isobutanol Concentration in Solid Medium]

Viabilities are analyzed by the same method described in Experimental Example 3, except for using plates containing isobutanol in a concentration of 0, 0.2, 0.4 and 0.6% instead of ethanol, and the results are shown in FIG. 13.

It can be seen that all host cells of Examples 1 to 8 show increases in viability, compared to the control group, the wild-type yeast. Particularly, the yeast groups into which the polynucleotides of Examples 1, 2, 4, 7 and 8 are introduced show strong tolerance to isobutanol.

[Experimental Example 5: Fermentation Test in 5% Ethanol and 10% Glucose]

The yeast strains of Examples 1 to 4 are fermented in mixed liquid media containing 5%(w/v) ethanol and 10% (w/v) glucose. Each yeast strain is incubated in a 250ml glass flask containing a minimal medium (SG media) in the total volume of 50 ml, and grown to a high cell density.

Viability is analyzed by a spectrophotometer (OD600), and ethanol volumetric productivity is analyzed by gas chromatography (GC). To analyze the ethanol volumetric productivity, a standard solution is prepared by mixing 100 g/L of 1-propanol and 100 g/L of ethanol in the ratio of 1:1 (v/v), and then a value obtained using 100 g/L of ethanol and 100 g/L of 1-propanol is set as an internal standard. Then, all samples are mixed with 100 g/L of 1-propanol in the ratio of 1:1 (v/v), respectively, and injected for GC. The ethanol volumetric productivity per unit sample is calculated in g/L. The results are shown in FIGS. 14 to 17.

In addition, to confirm whether a specific growth rate ($h^{-1}$) and ethanol volumetric productivity (g/L/h) are increased or not, data obtained by the fermentation test are calculated using parameters, and the results are shown in Table 5.

[Table 5: 5% ethanol, 10% glucose test]

| | Control | Example 1 | Example 2 | Example 3 | Example 4 |
|---|---|---|---|---|---|
| Specific growth rate ($h^{-1}$) 0-12h | 0.020 | 0.028 | 0.038 | 0.044 | 0.029 |
| Volumetric productivity (g/L/h) 0-16h | 1.783 | 2.108 | 2.435 | 2.364 | 2.358 |

Referring to FIGS. 14 to 17 and Table 5, it can be seen that the strains of Examples 1 to 4 show increases in viability, compared to the control group, the wild-type strain, resulting in an increase in alcohol tolerance. It can be also seen that time for producing 90g/L ethanol is shorter in the strains of Examples 1 to 4 (20 to 24h) than in the control strain (28 to 32h), resulting in an increase in alcohol volumetric productivity.

Particularly, it can be seen that the strain of Example 2 shows about 90% increase in specific growth rate and about 37% increase in ethanol volumetric productivity, compared to the control strain. It can be also seen that the strain of Example 3 shows about 100% or more increase in specific growth rate, which is 0.044 ($h^{-1}$), compared to the control group (0.02).

[Experimental Example 6: Fermentation Test in 5% ethanol and 20% glucose]

A fermentation test is performed by the same method described in Experimental Example 5 except that the yeast strains of Examples 1 to 4 are fermented in mixed liquid media containing 5%(w/v) ethanol and 20%(w/v) glucose. Viabilities and ethanol volumetric productivities are shown in FIGS. 18 to 21. In addition, specific growth rates (h-1) and ethanol volumetric productivities (g/L/h) are shown in Table 6.

[Table 6: 5% Ethanol and 20% Glucose Test]

| Parameter | Control | Example 1 | Example 2 | Example 3 | Example 4 |
|---|---|---|---|---|---|
| Specific growth rate($h^{-1}$) 0-12h | 0.017 | 0.030 | 0.032 | 0.034 | 0.019 |
| Volumetric productivity(g/L/h) 0-32h | 1.294 | 1.427 | 2.025 | 1.858 | 1.457 |

Referring to FIGS. 18 to 21 and Table 6, it can be seen that the strains of Examples 1 to 4 show increases in alcohol tolerance and alcohol volumetric productivity, compared to the control strain (wild-type). Particularly, it can be seen that the strain of Example 2 shows about 90% increase in specific growth rate and about 57% increase in ethanol volumetric productivity, compared to the control group. It can be also seen that the strain of Example 3 shows about 100% or more increase in the specific growth rate (0.034), compared to the control strain (0.017).

[Experimental Example 7: Fermentation Test according to Concentration Gradient of Glucose]

A fermentation test is performed using the yeast strain of Example 2 by the same method described in Experimental Example 5, except that 5%(w/v) ethanol is not contained, and glucose in a concentration 10%, 20% and 30% (w/v) is used. The fermentation tests are respectively performed at low cell density (OD = about 0.05) and high cell density (OD = about 10) under an oxygen-limited condition.

Viabilities and ethanol volumetric productivities are shown in FIGS. 22 to 27.

FIGS. 22, 24 and 26 show the results obtained at low cell density, and FIGS. 23, 25 and 27 show the results obtained at high cell density. Specific growth rates ($h^{-1}$) and ethanol volumetric productivities (g/L/h) obtained at low and high cell densities are shown in Tables 7 and 8, respectively.

[Table 7: Low Inoculums]

| Parameter | 10% Glucose | | 20% Glucose | | 30% Glucose | |
|---|---|---|---|---|---|---|
| | Control | Example 2 | Control | Example 2 | Control | Example 2 |
| Specific growth rate ($h^{-1}$) 0h-32h | 0.122 | 0.130 | 0.119 | 0.124 | 0.115 | 0.121 |
| Volumetric productivity (g/Lh$^{-1}$) 0h-48h | 0.625 | 0.750 | 0.685 | 0.761 | 0.694 | 0.725 |
| Specific productivity (g/DCWh$^{-1}$) 0h-48h | 0.286 | 0.343 | 0.329 | 0.344 | 0.433 | 0.418 |

[Table 8: High Inoculums]

| Parameter | 10% Glucose | | 20% Glucose | | 30% Glucose | |
|---|---|---|---|---|---|---|
| | Control | Example 2 | Control | Example 2 | Control | Example 2 |
| Specific growth rate($h^{-1}$) 0h-32h | - | - | 2.701 | 3.135 | 2.514 | 3.004 |
| Volumetric productivity (g/Lh$^{-1}$) 0h-48h | - | - | 1.173 | 1.280 | 0.961 | 1.292 |
| Specific productivity (g/DCWh$^{-1}$) 0h-48h | - | - | 0.256 | 0.235 | 0.254 | 0.224 |

Referring to FIGS. 22 to 27 and Tables 7 and 8, it can be seen that once yeast INO1 gene (set forth in SEQ ID NO: 2) is amplified and transformed, the transformed cell is rapidly grown in glucose and efficiently converts glucose into ethanol compared to the control strain. Specifically, it can be seen that in the case of the low cell inoculums, the transformed strain exhibits a higher volumetric productivity than the control parent strain in both 10% glucose and 20% glucose fermentation tests. It can be also seen that in the case of high cell inoculums, the transformed strain shows higher specific growth rate (0 to 24h) and ethanol volumetric productivity (0 to 60h) in both 20% and 30% glucose fermentation tests than the control strain.

[Experimental Example 8: Fermentation Test in Glucose/Galactose Mixed Medium]

A fermentation test is performed by the same method described in Experimental Example 5, except that the yeast strains of Examples 1 to 3 are fermented in mixed media containing glucose and galactose in various ratios (glucose:galactose = 2:2%(w/v), 2:6%(w/v) and 2:8%(w/v)), instead of the mixed media containing 5%(w/v) ethanol and 10%(w/v) glucose. Viability and ethanol volumetric productivity for the yeast strains of Examples 1 to 3 are shown in FIGS. 28 to 30. The ethanol volumetric productivity (g/L/h) is also shown in Table 9 below.

Referring to FIGS. 28 to 30 and Table 9, it can be seen that the strains of Examples 1 to 3 show increases in alcohol tolerance and alcohol volumetric productivity, compared to the control strain (wild-type).

[Table 9]

| Parameter | Glucose: Galactose %(w/v) | Control | Example 1 | Example 2 | Example 3 |
|---|---|---|---|---|---|
| Volumetric productivity (g/Lh$^{-1}$) 0h-56h | 2: 2 | 0.638 | 0.633 | 0.844 | 0.670 |
| | 2: 6 | 0.673 | 0.547 | 0.834 | 0.620 |
| | 2: 8 | 0.638 | 0.633 | 0.844 | 0.670 |

[Experimental Example 9: Viability Test using Colony Forming Unit (CFU)]

The strains of Examples 1 to 3 are incubated in liquid media containing 15%(w/v) ethanol and 2%(w/v) glucose. After 2, 4, 6, 8, 10 and 12 hours, viability is measured for each strain, and the viability is expressed in relative number according to time versus the number of initial colonies, and shown in FIG. 31. After 2, 4 and 6 hours, a cell death rate is measured, and shown in FIG. 32. The measurement of the viability and cell death rate is performed according to the method disclosed in "Engineering Yeast Transcription Machinery for Improved Ethanol Tolerance and Production" by Hal Alper et al., published in 8 December 2006, Science 314, 1565 (2006).

Referring to FIGS. 31 and 32, it can be seen that overexpressed strains of Examples 1 to 3 show significant increases in ethanol tolerance, compared to the control strain (wild-type). Specifically, it can be seen that, in the case of 15% ethanol, the strain of Example 3 (DOG1) shows 70% increase in ethanol tolerance and the strains of Examples 1 and 2 also show increases in ethanol tolerance, compared to the control group.

According example embodiments, an isolated polynucleotide encodes a protein enhancing alcohol tolerance, so that a host cell containing the isolated polynucleotide can exhibit excellent viability even in high-concentration alcohol and excellent homeostasis during fermentation. Thus, when the isolated polynucleotide is applied to industrial alcohol fermentation, an enhancement in alcohol volumetric productivity can be achieved, which is very efficient for industrial use.

## Claims

1. An isolated polynucleotide comprising a polynucleotide selected from the group consisting of:

a polynucleotide consisting of a base sequence having at least 90% identity to a base sequence selected from SEQ ID NOs: 1 to 8;
a polynucleotide encoding a polypeptide consisting of an amino acid sequence having at least 90% identity to an amino acid sequence selected from SEQ ID NOs: 14 to 20;
a polynucleotide consisting of a base sequence hybridized to a base sequence selected from SEQ ID NOs: 1 to 8 under stringent conditions; and
a polynucleotide encoding a polypeptide consisting of an amino acid sequence hybridized to an amino acid sequence selected from SEQ ID NOs: 14 to 20 under stringent conditions,
wherein the polynucleotide encodes a polypeptide increasing alcohol tolerance of a host cell.

2. The isolated polynucleotide according to claim 1, wherein the isolated polynucleotide is selected from the group consisting of:

(i) an isolated polynucleotide consisting of base sequences having at least 90% identity to base sequences of SEQ ID NOs: 1 and 9;
(ii) an isolated polynucleotide encoding a polypeptide consisting of amino acid sequences having at least 90% identity to amino acid sequences of SEQ ID NOs: 14 and 21;
(iii) an isolated polynucleotide hybridized to the isolated polynucleotide of (i) under stringent conditions; and
(iv) an isolated polynucleotide hybridized to the isolated polynucleotide of (ii) under stringent conditions.

3. The isolated polynucleotide according to claim 2, wherein the isolated polynucleotide has a base sequence set forth in SEQ ID NO: 26.

4. The isolated polynucleotide according to claim 1, wherein the isolated polynucleotide is selected from the group consisting of:

(i) an isolated polynucleotide consisting of base sequences having at least 90% identity to base sequences of SEQ ID NOs: 2, 10 and 11;
(ii) an isolated polynucleotide encoding a polypeptide consisting of amino acids sequences having at least 90% identity to amino acid sequences of SEQ ID NOs: 15, 22 and 23;
(iii) an isolated polynucleotide hybridized to the isolated polynucleotide of (i) under stringent conditions; and
(iv) an isolated polynucleotide hybridized to the isolated polynucleotide of (ii) under stringent conditions.

**5.** The isolated polynucleotide according to claim 4, wherein the isolated polynucleotide has a base sequence set forth in SEQ ID NO: 27.

**6.** The isolated polynucleotide according to claim 1, wherein the isolated polynucleotide is a polynucleotide derived from *Saccharomyces cerevisiae.*

**7.** The isolated polynucleotide according to claim 1, wherein the alcohol tolerance is expressed as a specific cell growth rate ($h^{-1}$) in minimum inhibition concentration (MIC).

**8.** The isolated polynucleotide according to claim 7, wherein the MIC is about 5% for ethanol or about 1% for isobutanol.

**9.** The isolated polynucleotide according to claim 1, wherein the isolated polynucleotide is selected from the group consisting of:

an isolated polynucleotide consisting of a base sequence having at least 90% identity to a base sequence selected from SEQ ID NOs: 28 to 31; and
an isolated polynucleotide consisting of a base sequence hybridized to a base sequence selected from SEQ ID NOs: 28 to 31 under stringent conditions.

**10.** A vector comprising an isolated polynucleotide according to any one of claims 1 to 9.

**11.** The vector according to claim 10, wherein the vector is a plasmid.

**12.** A host cell capable of producing alcohol when incubated in a monosaccharide-containing nutrient source, and exhibiting overexpression of one or more isolated polynucleotides that encode a polypeptide increasing alcohol tolerance of the host cell and comprise a polynucleotide selected from the group consisting of:

a polynucleotide consisting of a base sequence having at least 90% identity to a base sequence selected from SEQ ID NOs: 1 to 8;
a polynucleotide encoding a polypeptide consisting of an amino acid sequence having at least 90% identity to an amino acid sequence selected from SEQ ID NOs: 14 to 20;
a polynucleotide consisting of a base sequence hybridized to a base sequence selected from SEQ ID NOs: 1 to 8 under stringent conditions; and
a polynucleotide encoding a polypeptide consisting of an amino acid sequence hybridized to an amino acid sequence selected from SEQ ID NOs: 14 to 20 under stringent conditions.

**13.** The host cell according to claim 12, wherein the host cell is a species of the genus *Saccharomyces*.

**14.** The host cell according to claim 12, wherein the monosaccharide is glucose, galactose or a combination thereof.

**15.** The host cell according to claim 12, wherein the host cells exhibits at least 30% increase in specific growth rate ($h^{-1}$) in MIC, compared to wild-type *S. cerevisiae*.

**16.** The host cell according to claim 12, wherein the MIC is about 5% for ethanol or about 1% for isobutanol.

**17.** The host cell according to claim 12, wherein the host cell exhibits at least 10% increase in volumetric productivity of ethanol (g/L/h) compared to wild-type *S. cerevisiae* under the same incubation conditions.

**18.** The host cell according to claim 12, wherein the host cell exhibits overexpression of a polynucleotide consisting of a base sequence having at least 90% identity to a base sequence of SEQ ID NO: 26 or 27.

**19.** The host cell according to claim 12, wherein the host cell is selected from the group consisting of:

a host cell derived from *S. cerevisiae* CEN.PK2-1D/pRS424-MSN2/MIH1 deposited under Accession No. KCTC11476BP;
a host cell derived from *S. cerevisiae* CEN.PK2-1D/pRS424-INO1 deposited under Accession No. KCTC11477BP;
a host cell derived from *S. cerevisiae* CEN.PK2-1D/pRS424-DOG1 deposited under Accession No. KCTC11478BP;
a host cell derived from *S. cerevisiae* CEN.PK2-1D/pRS424-HAL1 deposited under Accession No. KCTC11479BP
a host cell derived from *S. cerevisiae* CEN.PK2-1D/pRS424-TRP1 deposited under Accession No. KCTC11480BP
a host cell derived from *S. cerevisiae* CEN.PK2-1D/pRS424-MRPL17 deposited under Accession No. KCTC 11481 BP;
a host cell derived from *S. cerevisiae* CEN.PK2-1D/pRS424-YLR157C-B deposited under Accession No. KCTC11482BP; and
a host cell derived from *S. cerevisiae* CEN.PK2-1D/pRS424-SPG5p deposited under Accession No. KCTC11483BP.

**20.** The host cell according to claim 12, wherein the overexpression is achieved by increasing the number of copies of the polynucleotide.

**21.** A method of producing bioalcohol comprising incubating the host cell according to any one of claims 12 to 20 in a monosaccharide-containing nutrient source and producing alcohol through fermentation.

**22.** The method according to claims 21, further comprising:

engineering a host cell to overexpress one or more isolated polynucleotides encoding a polypeptide increasing alcohol tolerance of the host cell and including one selected from the group consisting of (a) a polynucleotide consisting of a base sequence having at least 90% identity to a base sequence selected from SEQ ID NOs: 1 to 8, (b) a polynucleotide encoding a polypeptide consisting of an amino acid sequence having at least 90% identity to an amino acid sequence selected from SEQ ID NOs: 14 to 20, (c) a polynucleotide consisting of a base sequence hybridized to a base sequence selected from SEQ ID NOs: 1 to 8 under stringent conditions, and (d) a polynucleotide encoding a polypeptide consisting of an amino acid sequence hybridized to an amino acid sequence selected from SEQ ID NOs: 14 to 20 under stringent conditions; and
incubating the host cell in a monosaccharide-containing nutrient source under conditions suitable for producing alcohol, and producing alcohol through fermentation.

**23.** The method according to claim 22, wherein the engineering of the host cell includes inserting the polynucleotide encoding a polypeptide into a vector, amplifying the vector, and inserting the vector into the host cell.

**24.** The method according to claim 21, wherein the host cell is a yeast cell.

**25.** The method according to claim 21, wherein the incubating of the host cell is performed by stirring at a rate of about 100 to about 250rpm under conditions including an initial glucose concentration of about 2 to about 30% (w/v), a temperature of about 25 to about 37°C, and pH of about 5.0 to about 8.0.

FIG. 1

MSN2, Chromosome XIII, 343143-346374

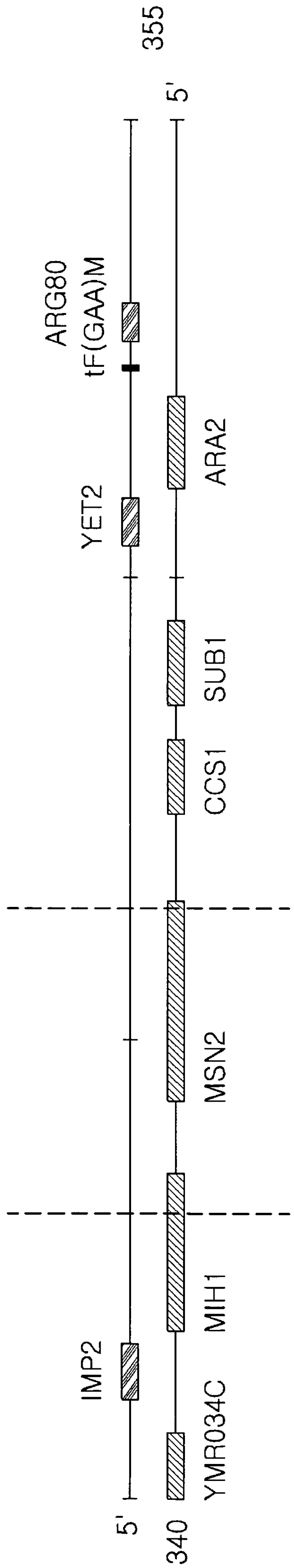

FIG. 2

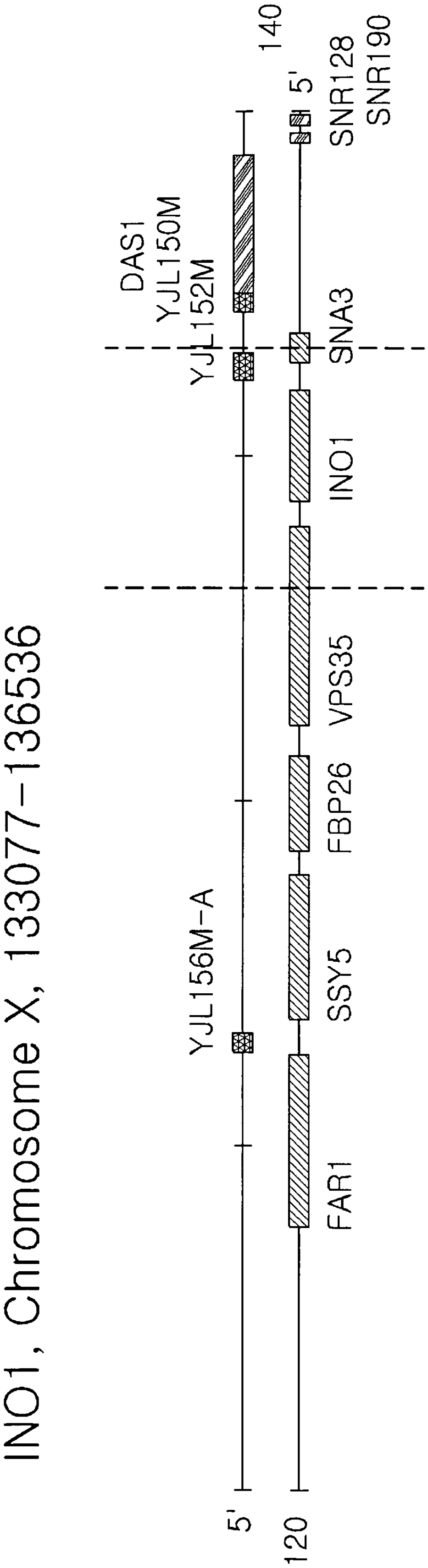
INO1, Chromosome X, 133077−136536
5'
120
140
5'
YJL156M-A
YJL152M
YJL150M
DAS1
FAR1
SSY5
FBP26
VPS35
INO1
SNA3
SNR128
SNR190

FIG. 3

DOG1, Chromosome VIII, 193809-196179

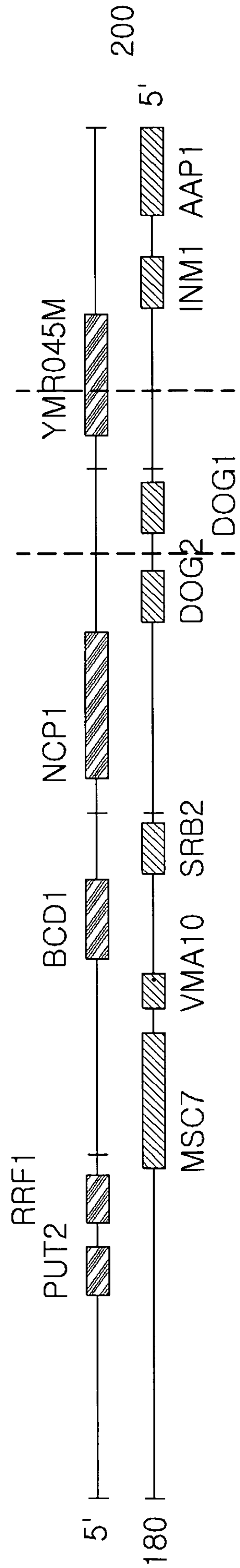

FIG. 4

HAL1, Chromosome XVI, 564101-567161

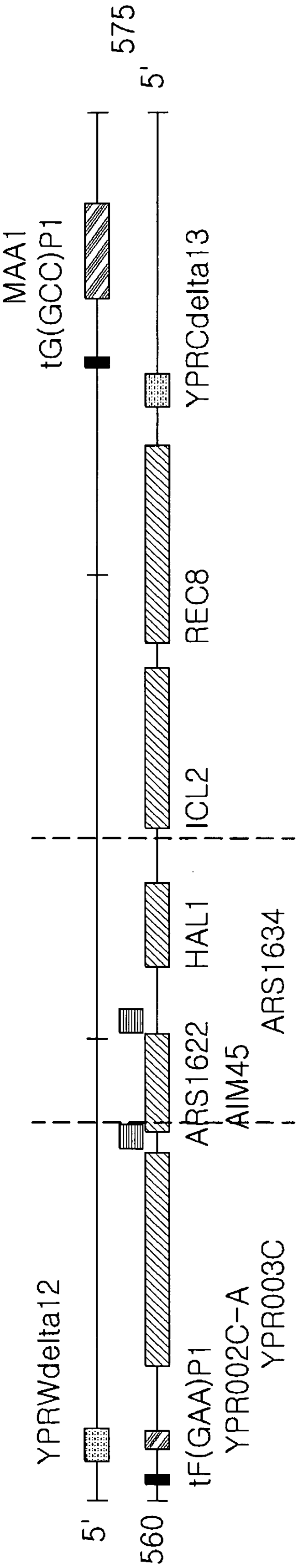

FIG. 5

TRP1, Chromosome IV, 461688-462590

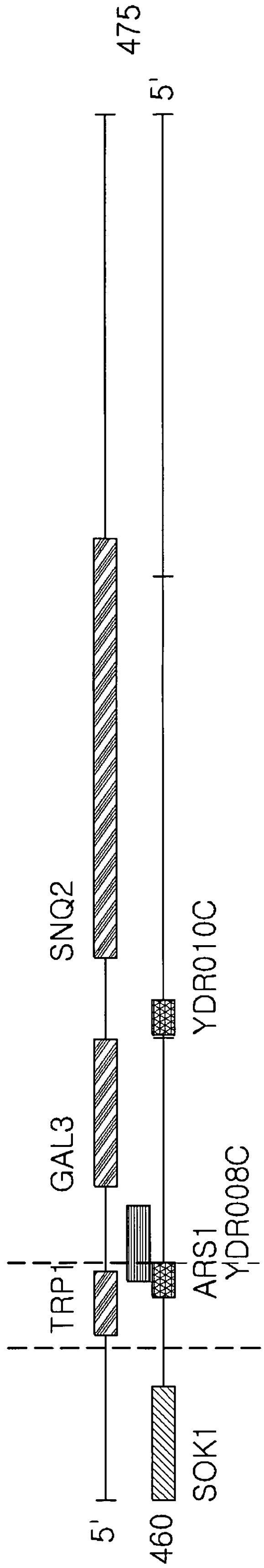

FIG. 6

MRPL17, Chromosome XIV, 171502-172588

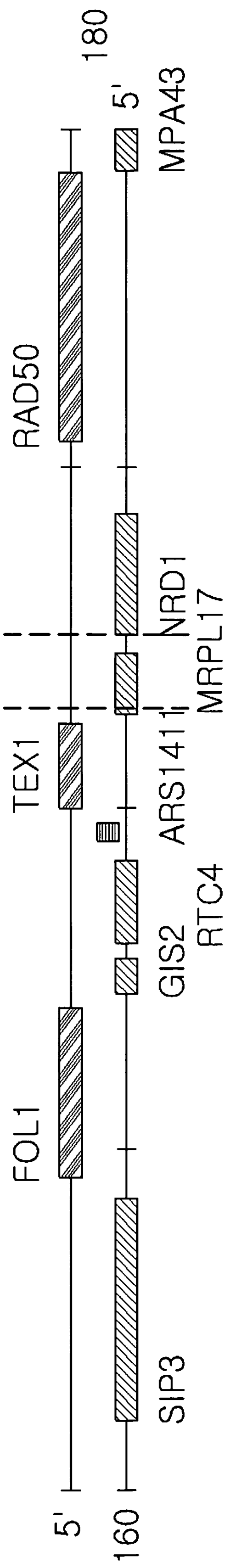

FIG. 7

YLR157C-B/YLRCTy1-1, Chromosome XII, 479060-480527

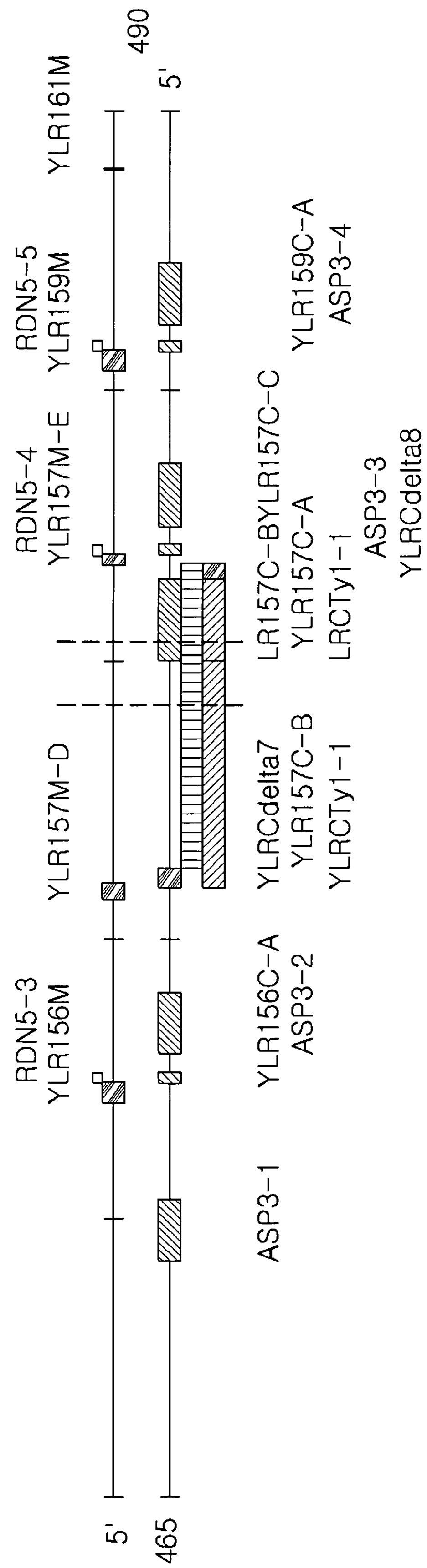

FIG. 8

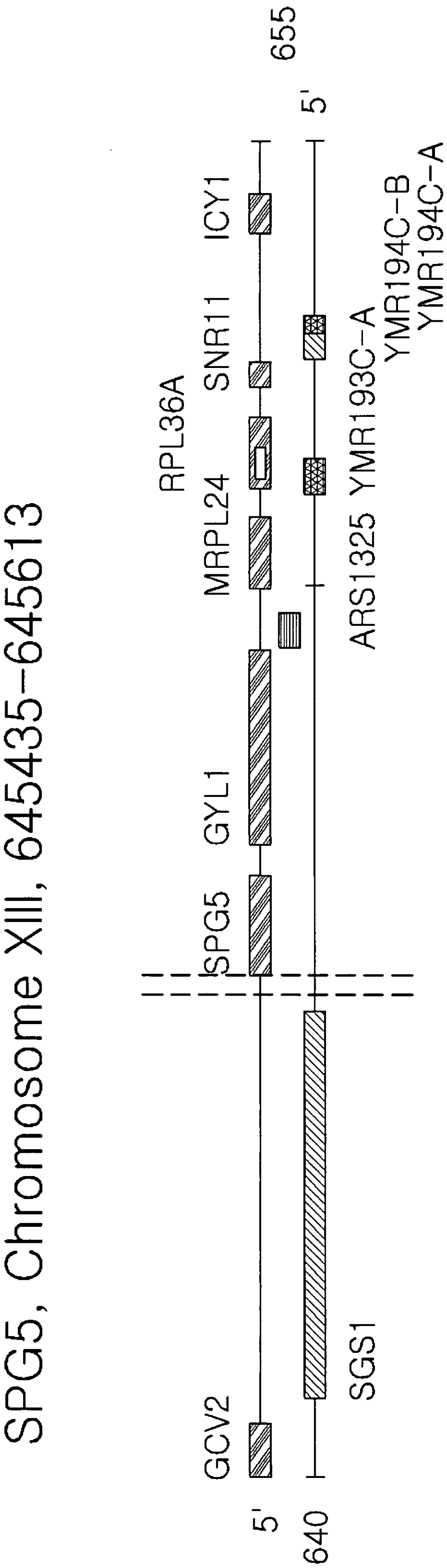
SPG5, Chromosome XIII, 645435-645613
5'
640
GCV2
SGS1
SPG5
GYL1
MRPL24
ARS1325
RPL36A
YMR193C-A
SNR11
YMR194C-B
YMR194C-A
ICY1
5'
655

FIG. 9

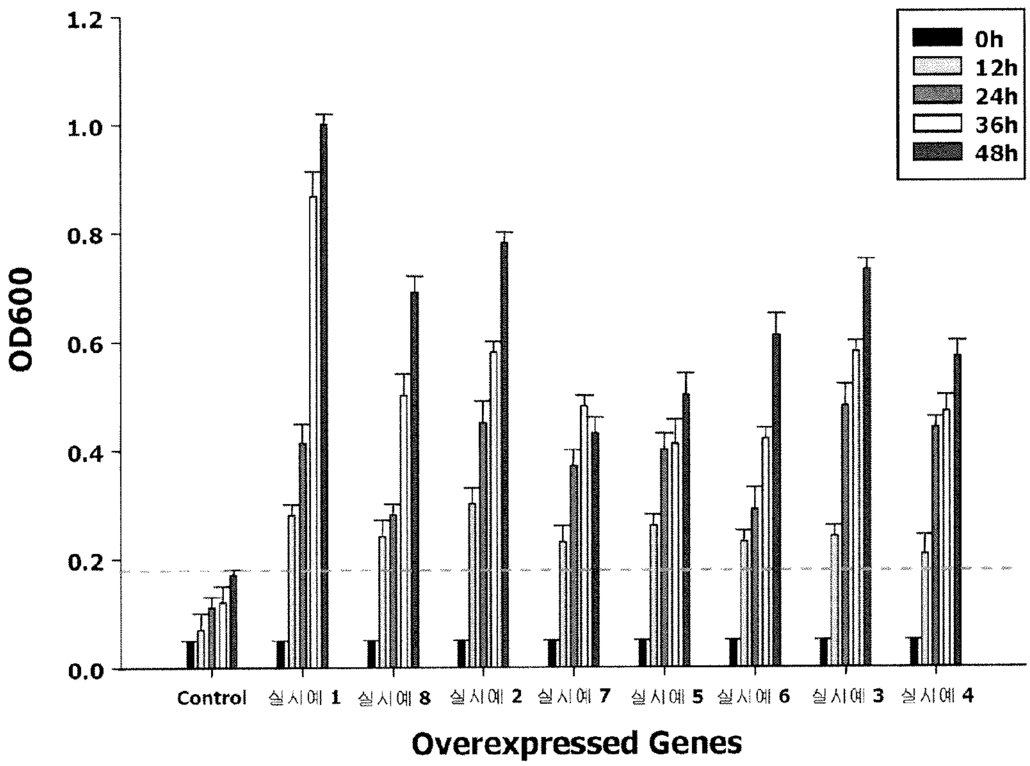
1.2
1.0
0.8
0.6
0.4
0.2
0.0
OD600
0h
12h
24h
36h
48h
Control
실시예 1
실시예 8
실시예 2
실시예 7
실시예 5
실시예 6
실시예 3
실시예 4
Overexpressed Genes

FIG. 10

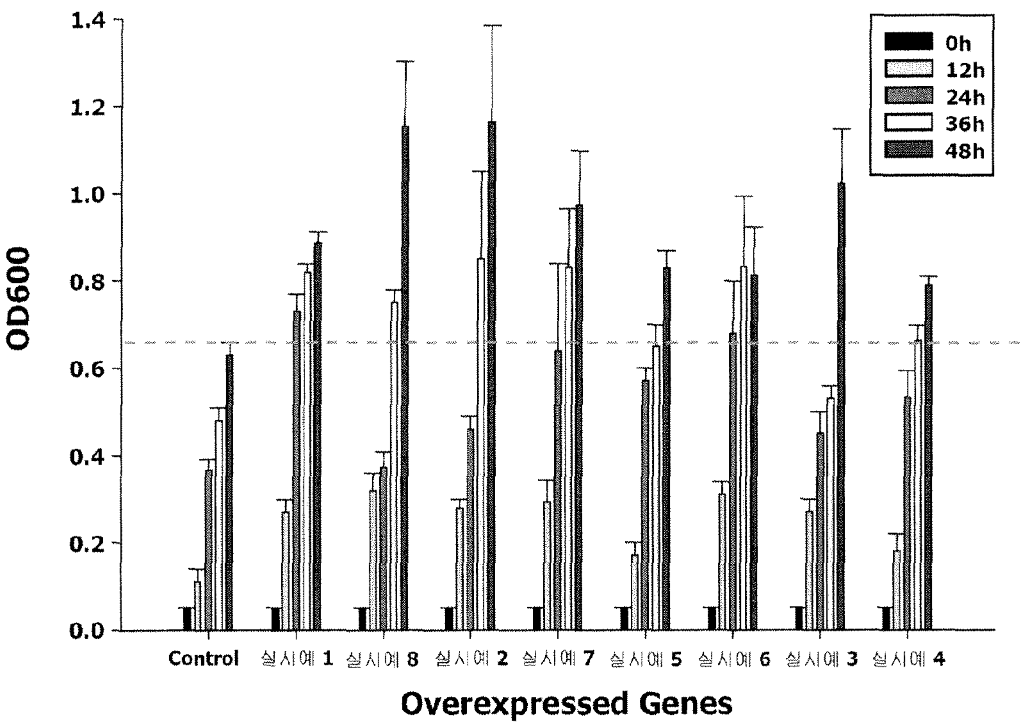

OD600
1.4
1.2
1.0
0.8
0.6
0.4
0.2
0.0
0h
12h
24h
36h
48h
Control
실시예 1
실시예 8
실시예 2
실시예 7
실시예 5
실시예 6
실시예 3
실시예 4
Overexpressed Genes

FIG. 11

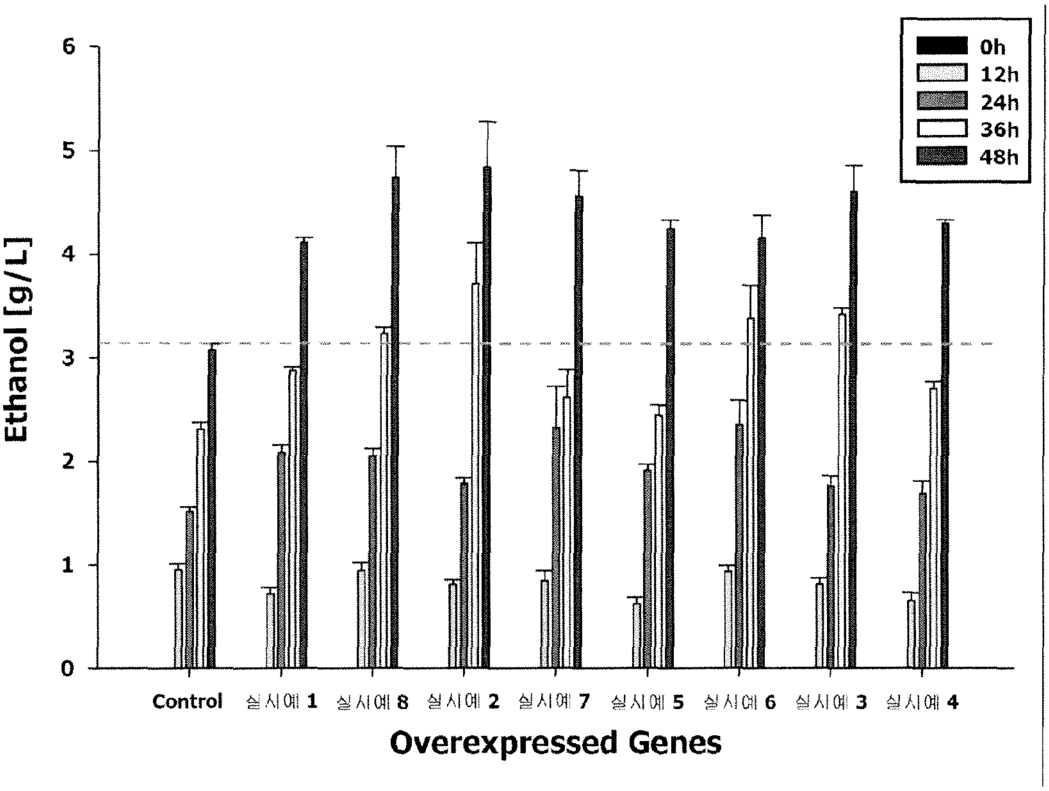
6
5
4
3
2
1
0
Ethanol [g/L]
0h
12h
24h
36h
48h
Control
실시예 1
실시예 8
실시예 2
실시예 7
실시예 5
실시예 6
실시예 3
실시예 4
Overexpressed Genes

FIG. 12

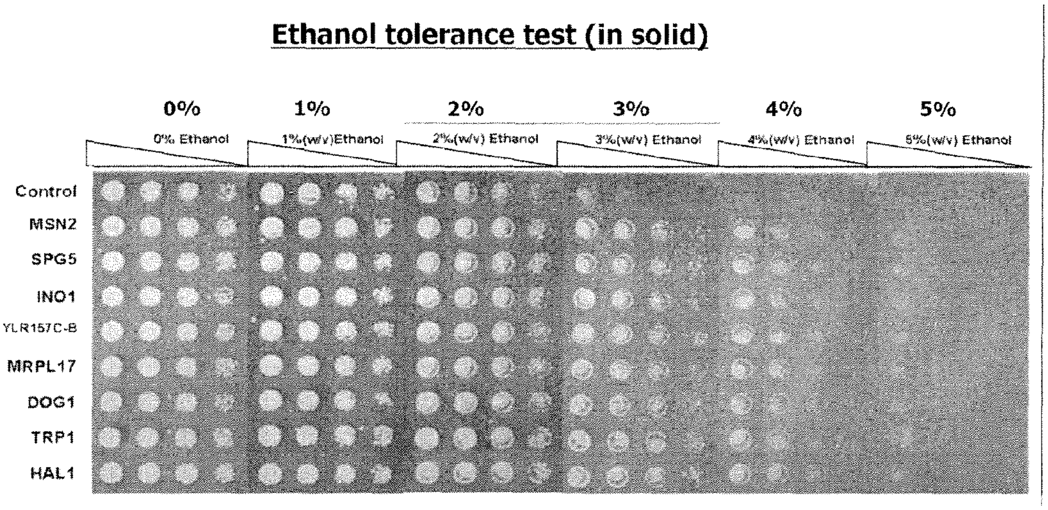
Ethanol tolerance test (in solid)
0%
1%
2%
3%
4%
5%
0% Ethanol
1%(w/v)Ethanol
2%(w/v) Ethanol
3%(w/v) Ethanol
4%(w/v) Ethanol
5%(w/v) Ethanol
Control
MSN2
SPG5
INO1
YLR157C-B
MRPL17
DOG1
TRP1
HAL1

FIG. 13

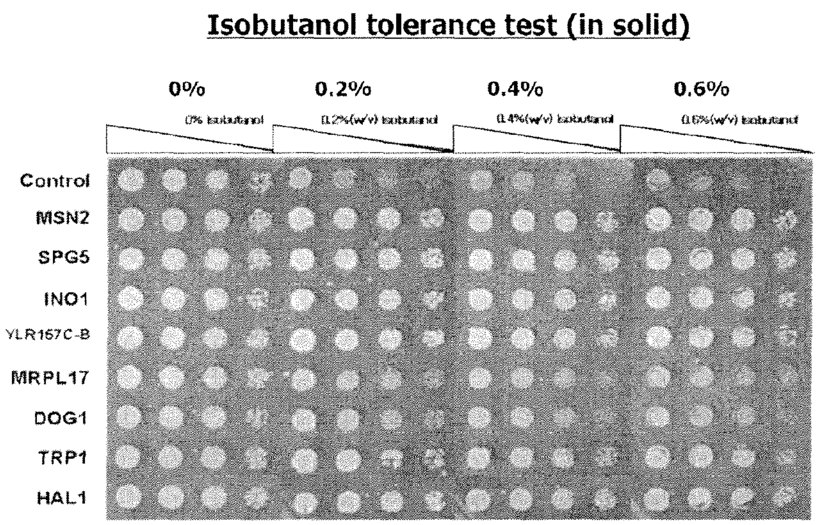
Isobutanol tolerance test (in solid)
0%
0.2%
0.4%
0.6%
0% isobutanol
0.2%(w/v) isobutanol
0.4%(w/v) isobutanol
0.6%(w/v) isobutanol
Control
MSN2
SPG5
INO1
YLR157C-B
MRPL17
DOG1
TRP1
HAL1

FIG. 14

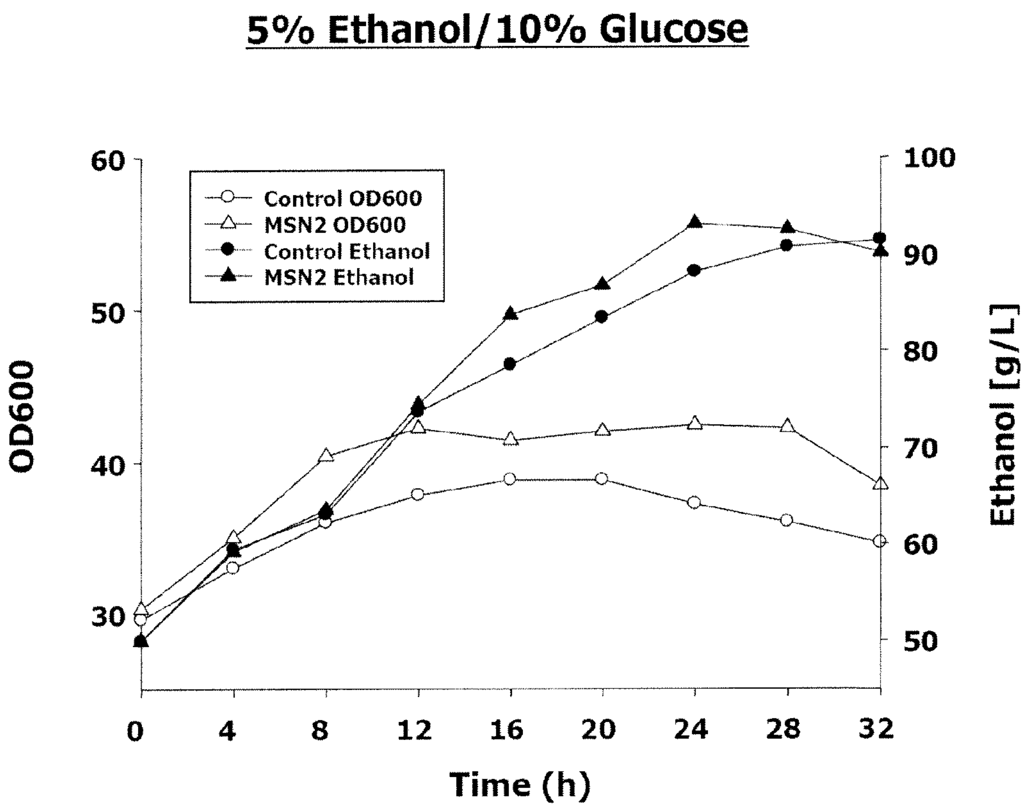
5% Ethanol/10% Glucose
Control OD600
MSN2 OD600
Control Ethanol
MSN2 Ethanol
OD600
Ethanol [g/L]
Time (h)
60
50
40
30
100
90
80
70
60
50
0
4
8
12
16
20
24
28
32

FIG. 15

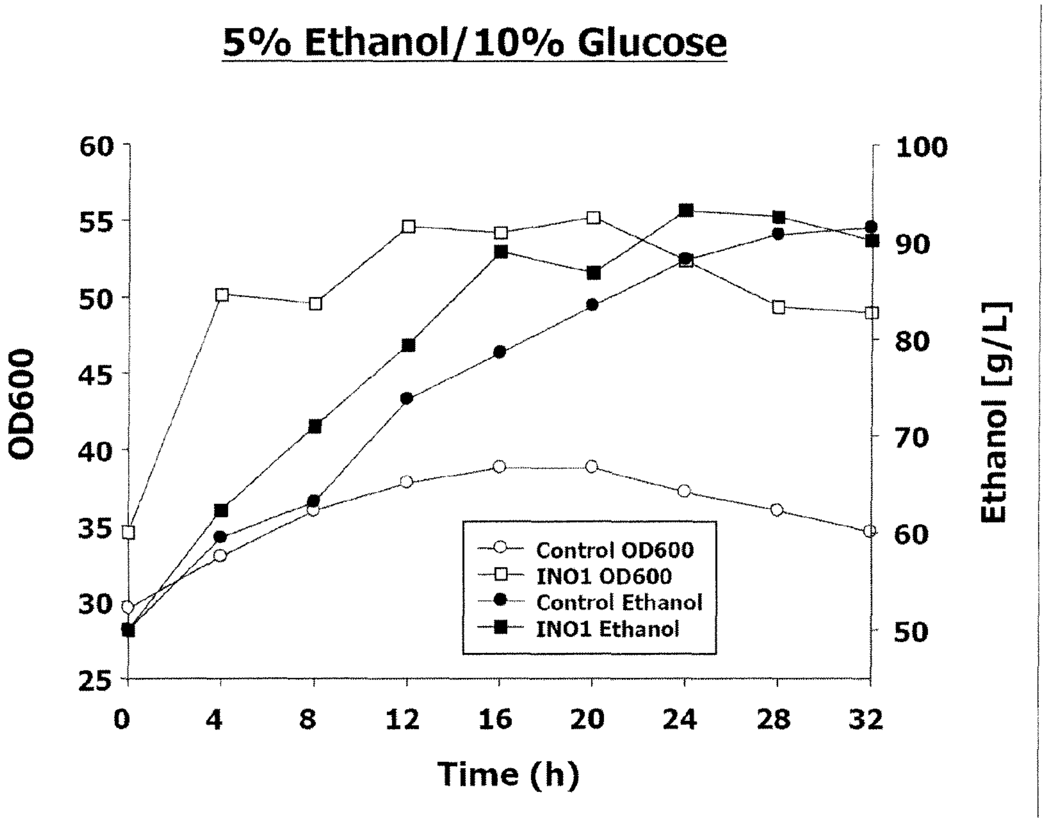
5% Ethanol/10% Glucose
60
55
50
45
40
35
30
25
OD600
100
90
80
70
60
50
Ethanol [g/L]
0
4
8
12
16
20
24
28
32
Time (h)
Control OD600
INO1 OD600
Control Ethanol
INO1 Ethanol

FIG. 16

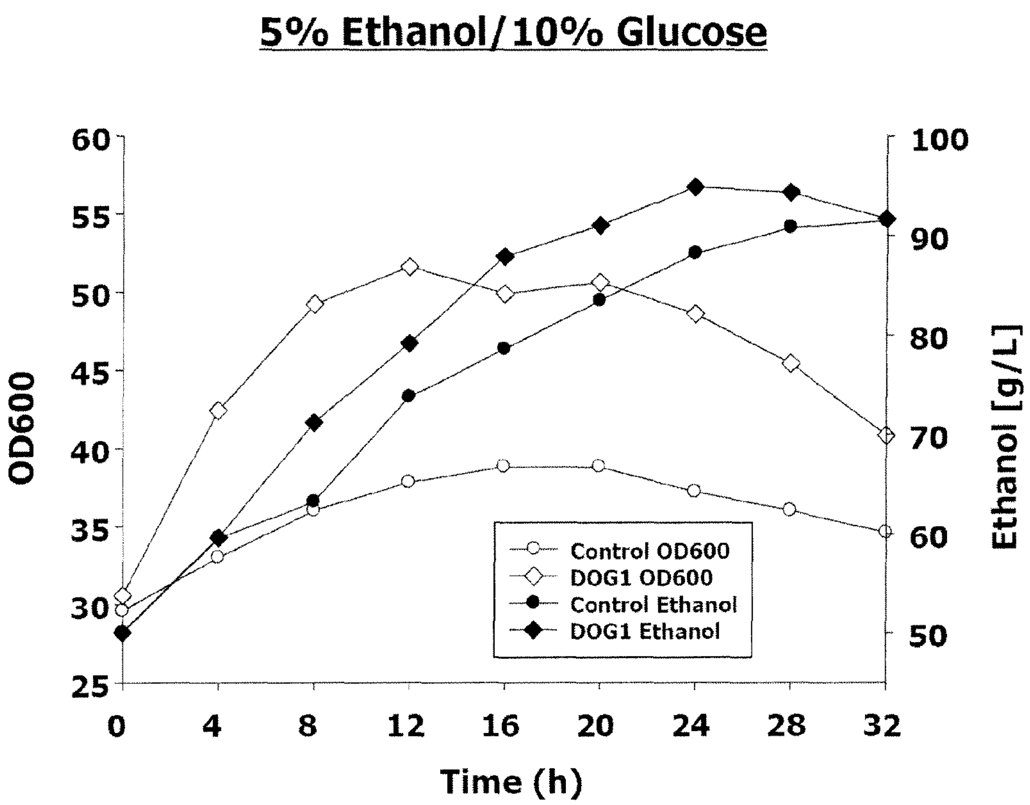
5% Ethanol/10% Glucose
OD600
Ethanol [g/L]
Time (h)
Control OD600
DOG1 OD600
Control Ethanol
DOG1 Ethanol
60
55
50
45
40
35
30
25
100
90
80
70
60
50
0
4
8
12
16
20
24
28
32

FIG. 17

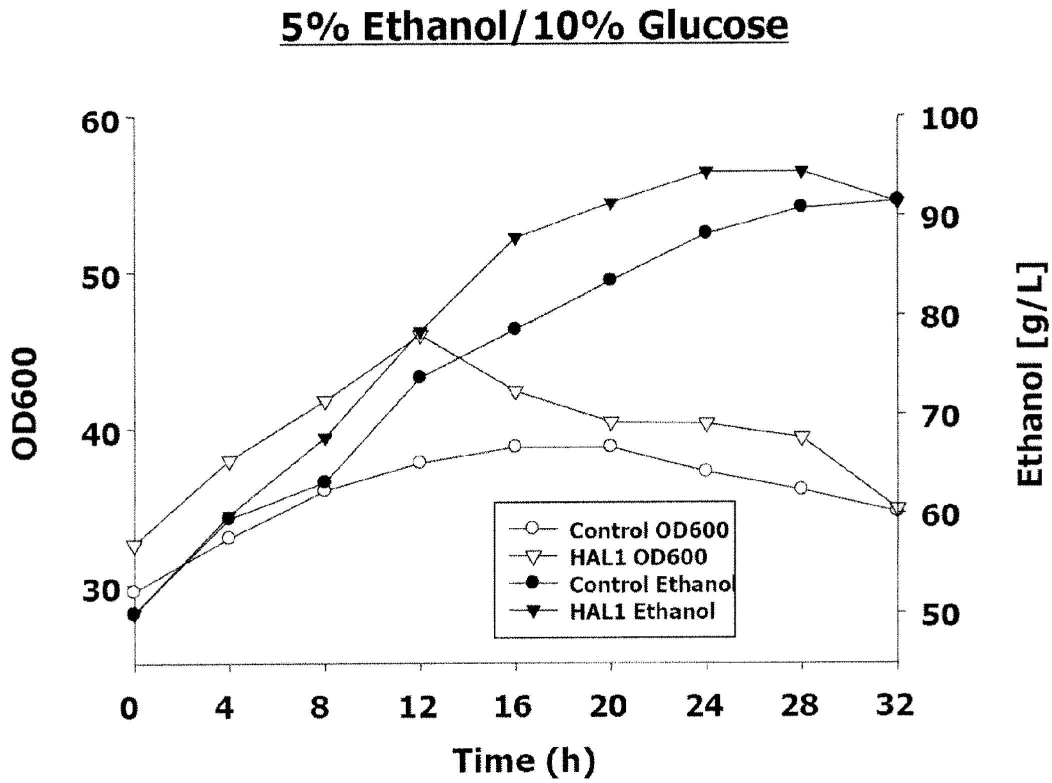
5% Ethanol/10% Glucose
60
50
40
30
OD600
100
90
80
70
60
50
Ethanol [g/L]
Control OD600
HAL1 OD600
Control Ethanol
HAL1 Ethanol
0
4
8
12
16
20
24
28
32
Time (h)

FIG. 18

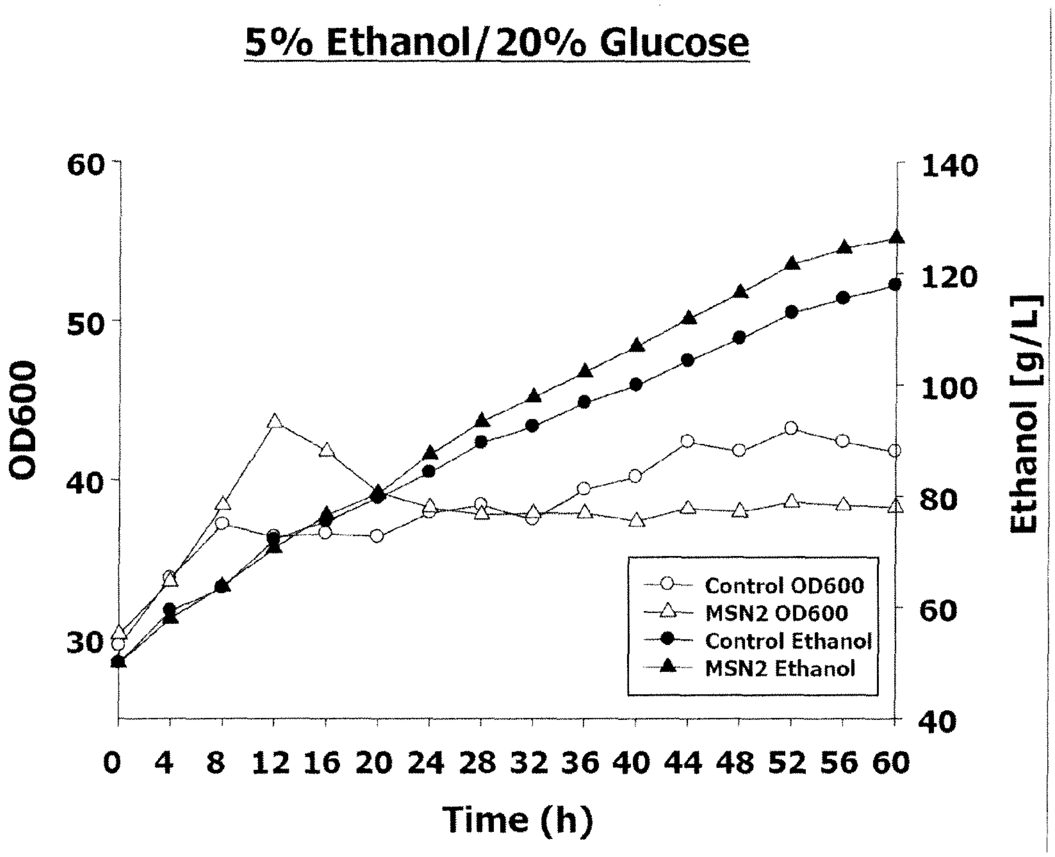
5% Ethanol/20% Glucose
OD600
60
50
40
30
Ethanol [g/L]
140
120
100
80
60
40
0 4 8 12 16 20 24 28 32 36 40 44 48 52 56 60
Time (h)
Control OD600
MSN2 OD600
Control Ethanol
MSN2 Ethanol

FIG. 19

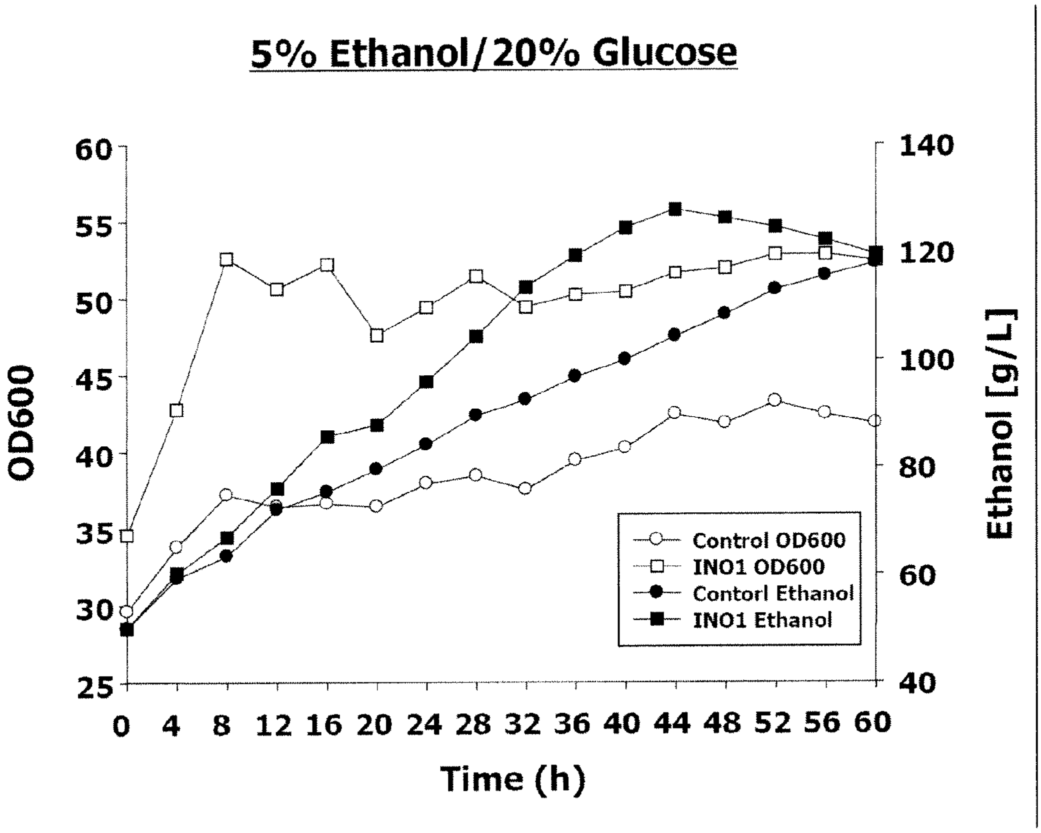
5% Ethanol/20% Glucose
OD600
60
55
50
45
40
35
30
25
140
120
100
80
60
40
Ethanol [g/L]
0 4 8 12 16 20 24 28 32 36 40 44 48 52 56 60
Time (h)
Control OD600
INO1 OD600
Contorl Ethanol
INO1 Ethanol

FIG. 20

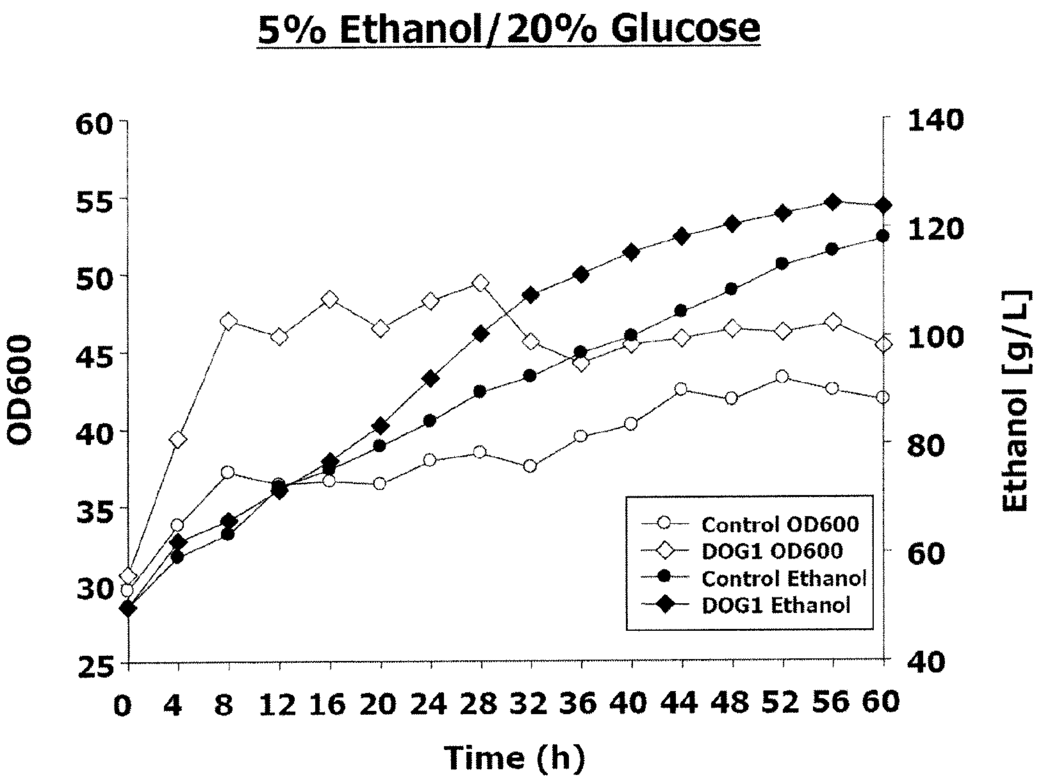
5% Ethanol/20% Glucose
OD600
Ethanol [g/L]
Time (h)
60
55
50
45
40
35
30
25
140
120
100
80
60
40
0 4 8 12 16 20 24 28 32 36 40 44 48 52 56 60
Control OD600
DOG1 OD600
Control Ethanol
DOG1 Ethanol

FIG. 21

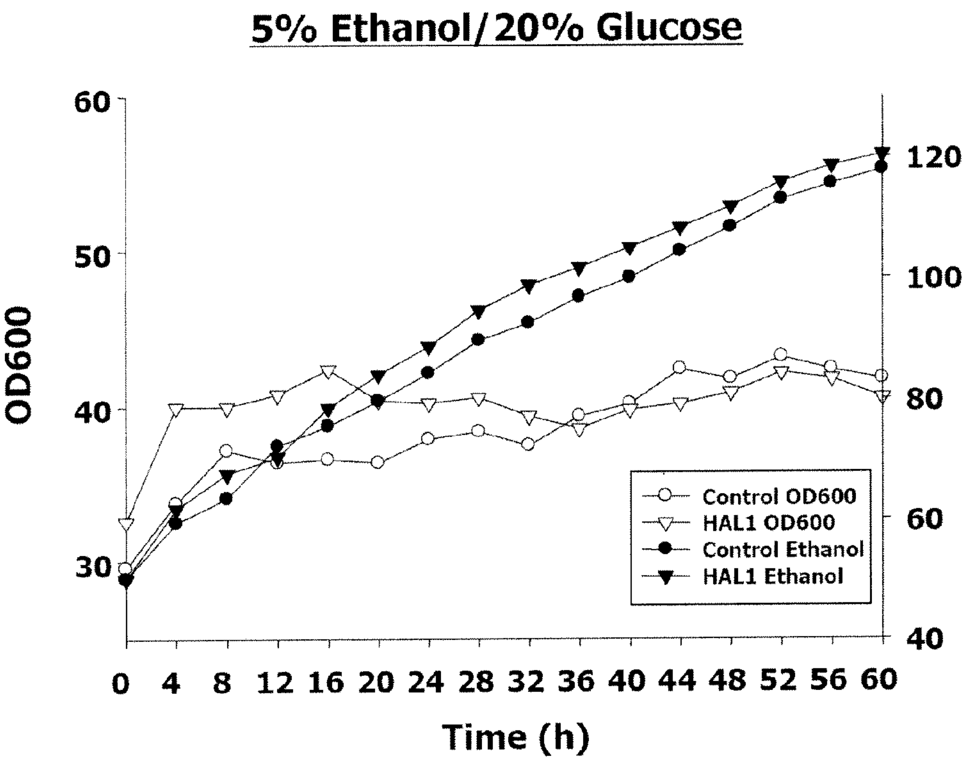
5% Ethanol/20% Glucose
OD600
Ethanol [g/L]
Time (h)
Control OD600
HAL1 OD600
Control Ethanol
HAL1 Ethanol
60
50
40
30
120
100
80
60
40
0 4 8 12 16 20 24 28 32 36 40 44 48 52 56 60

FIG. 22

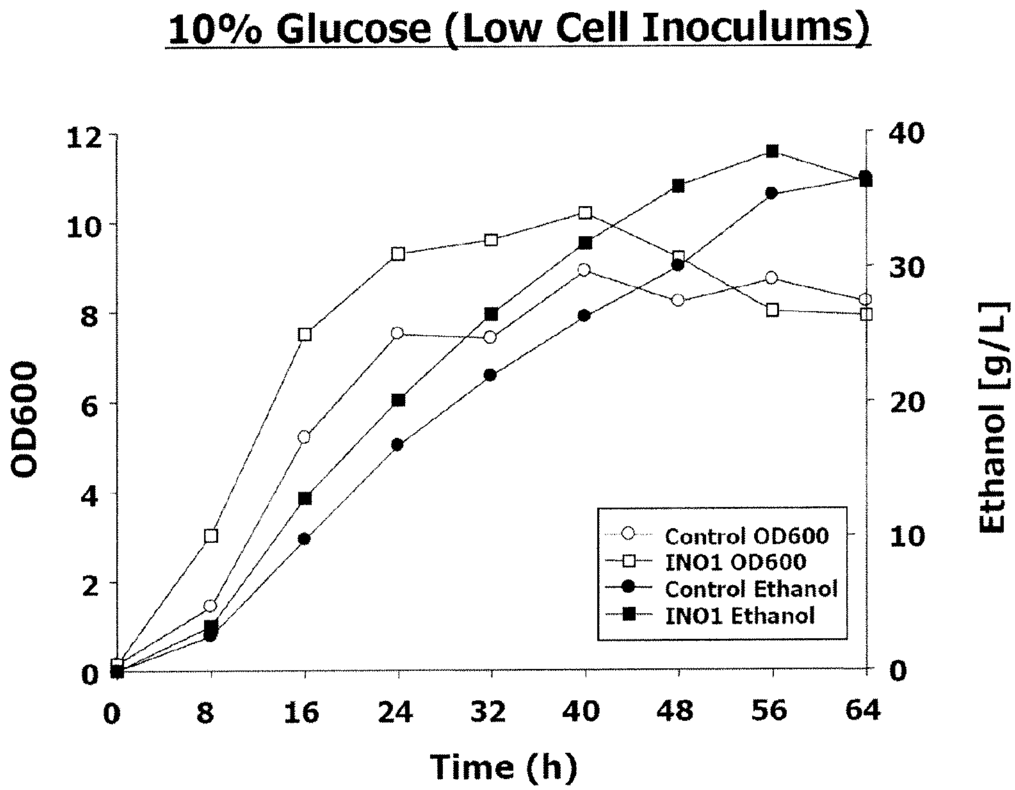
10% Glucose (Low Cell Inoculums)
OD600
Ethanol [g/L]
Time (h)
0
2
4
6
8
10
12
0
10
20
30
40
0
8
16
24
32
40
48
56
64
Control OD600
INO1 OD600
Control Ethanol
INO1 Ethanol

FIG. 23

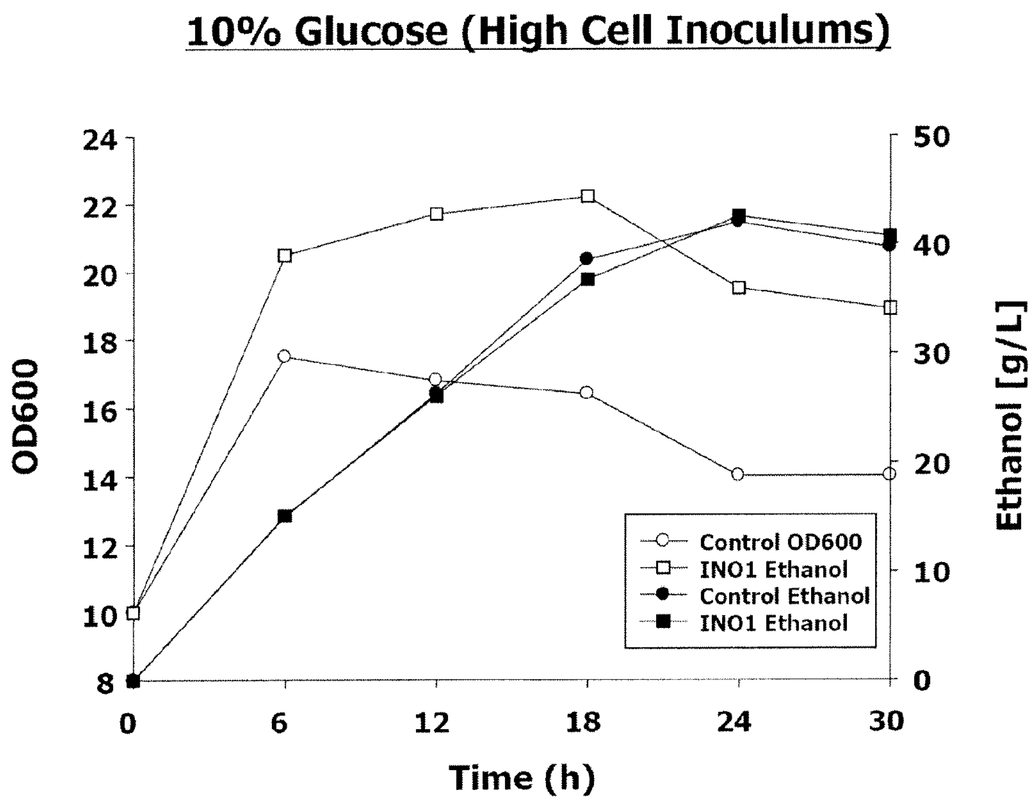
10% Glucose (High Cell Inoculums)
OD600
Ethanol [g/L]
Time (h)
Control OD600
INO1 Ethanol
Control Ethanol
INO1 Ethanol
24
22
20
18
16
14
12
10
8
50
40
30
20
10
0
0
6
12
18
24
30

FIG. 24

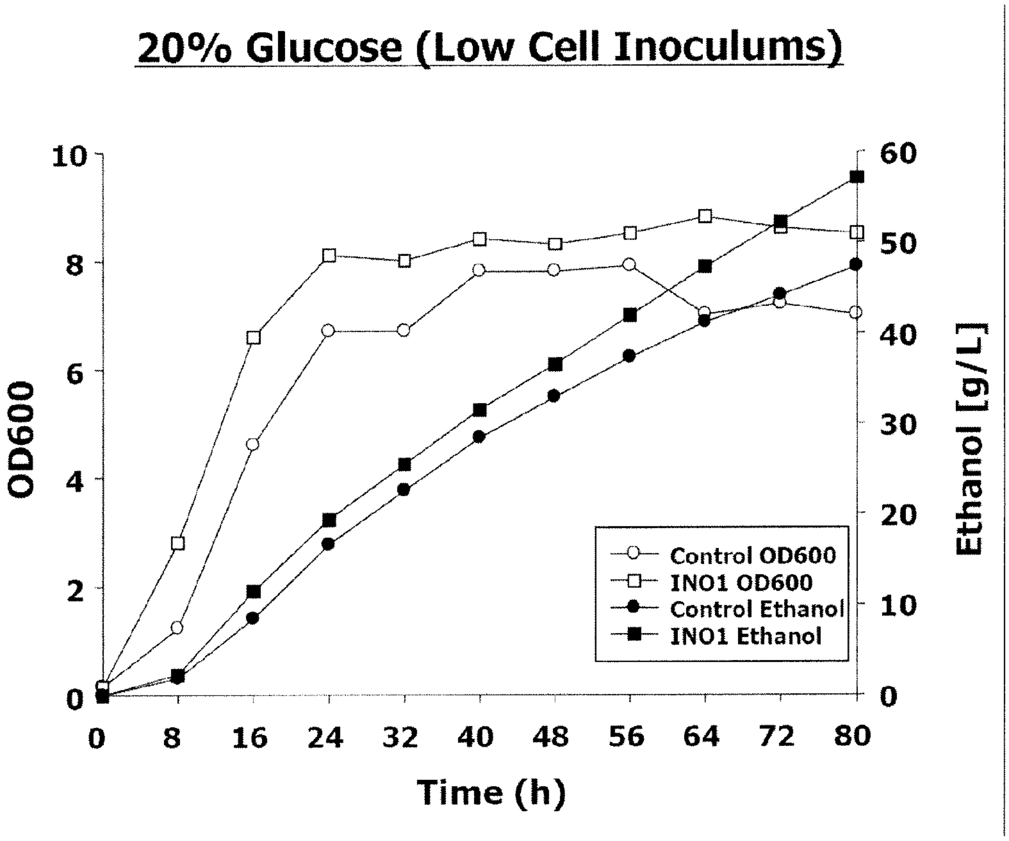
20% Glucose (Low Cell Inoculums)
OD600
Ethanol [g/L]
Time (h)
0
8
16
24
32
40
48
56
64
72
80
0
2
4
6
8
10
0
10
20
30
40
50
60
Control OD600
INO1 OD600
Control Ethanol
INO1 Ethanol

FIG. 25

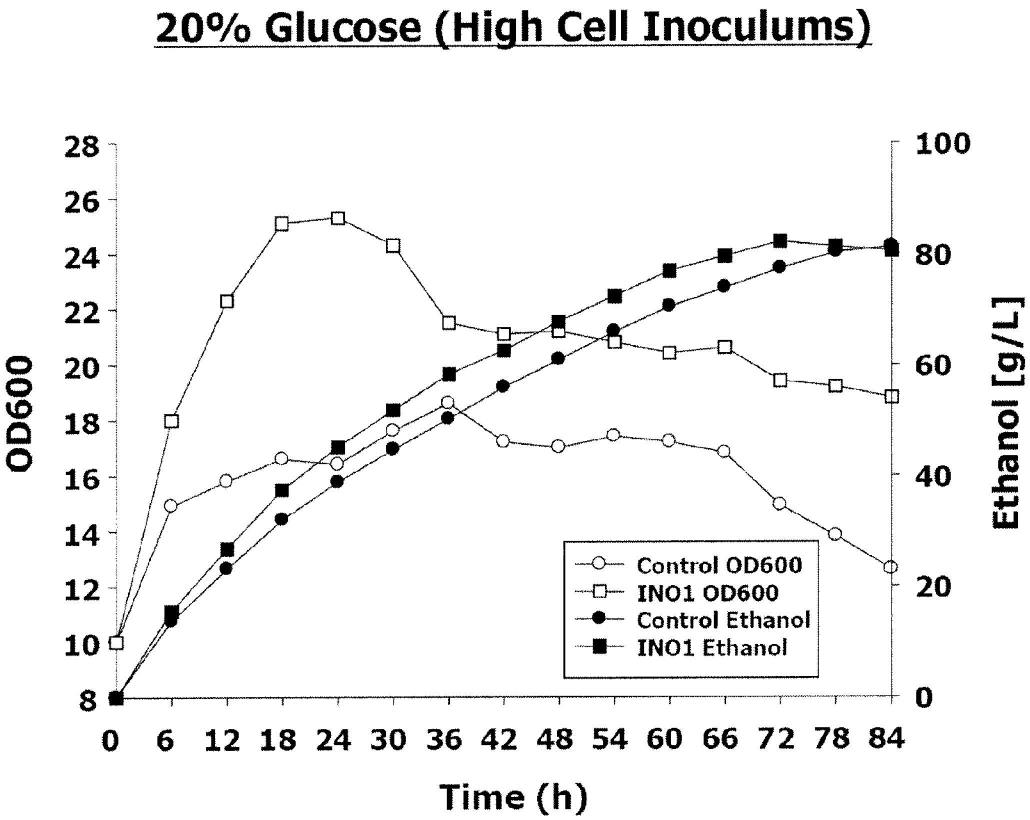
20% Glucose (High Cell Inoculums)
OD600
Ethanol [g/L]
Time (h)
28
26
24
22
20
18
16
14
12
10
8
100
80
60
40
20
0
0 6 12 18 24 30 36 42 48 54 60 66 72 78 84
Control OD600
INO1 OD600
Control Ethanol
INO1 Ethanol

FIG. 26

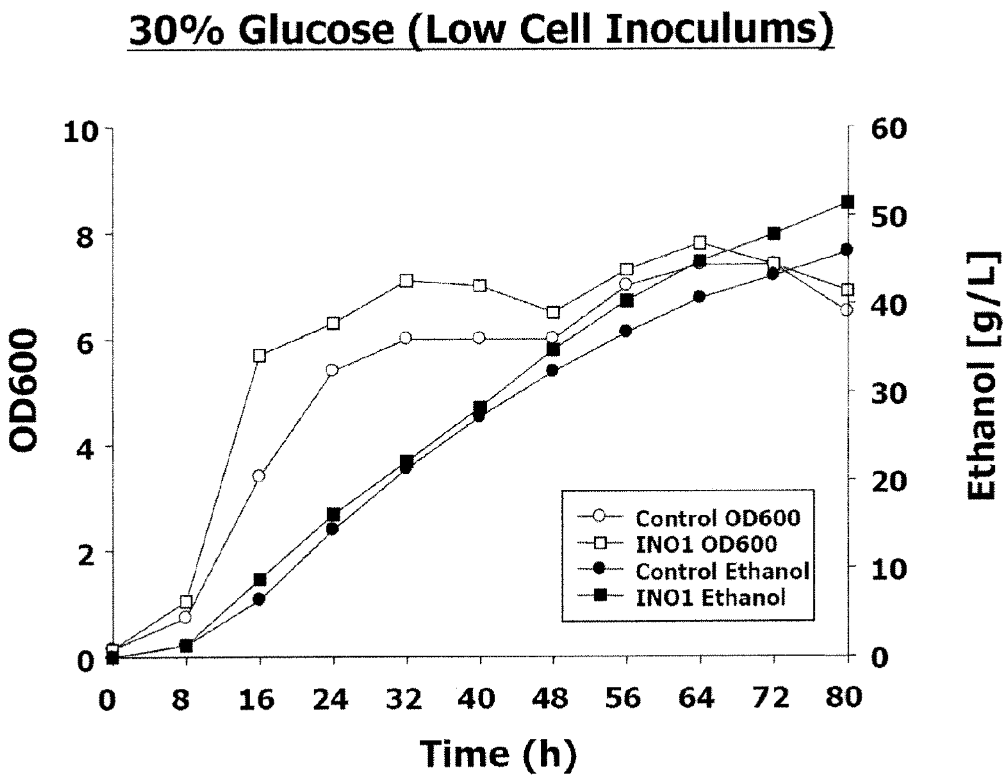
30% Glucose (Low Cell Inoculums)
OD600
Ethanol [g/L]
Time (h)
0
2
4
6
8
10
10
20
30
40
50
60
8
16
24
32
40
48
56
64
72
80
Control OD600
INO1 OD600
Control Ethanol
INO1 Ethanol

FIG. 27

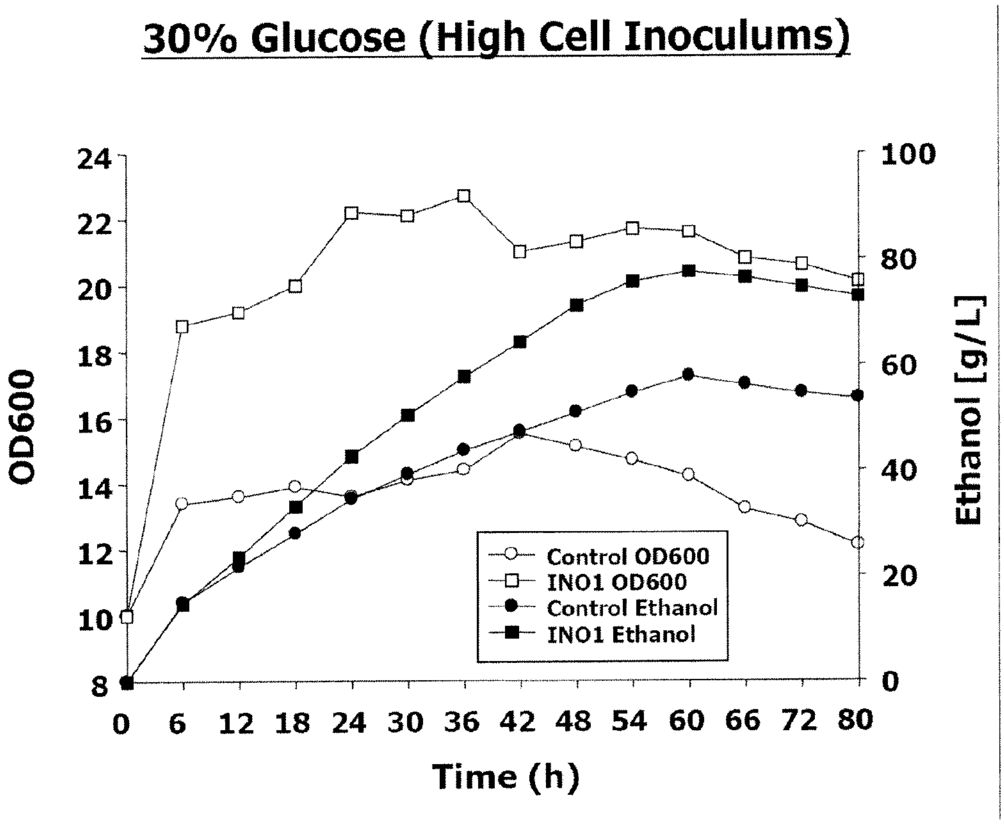
30% Glucose (High Cell Inoculums)
OD600
Ethanol [g/L]
Time (h)
24
22
20
18
16
14
12
10
8
100
80
60
40
20
0
0 6 12 18 24 30 36 42 48 54 60 66 72 80
Control OD600
INO1 OD600
Control Ethanol
INO1 Ethanol

FIG. 28

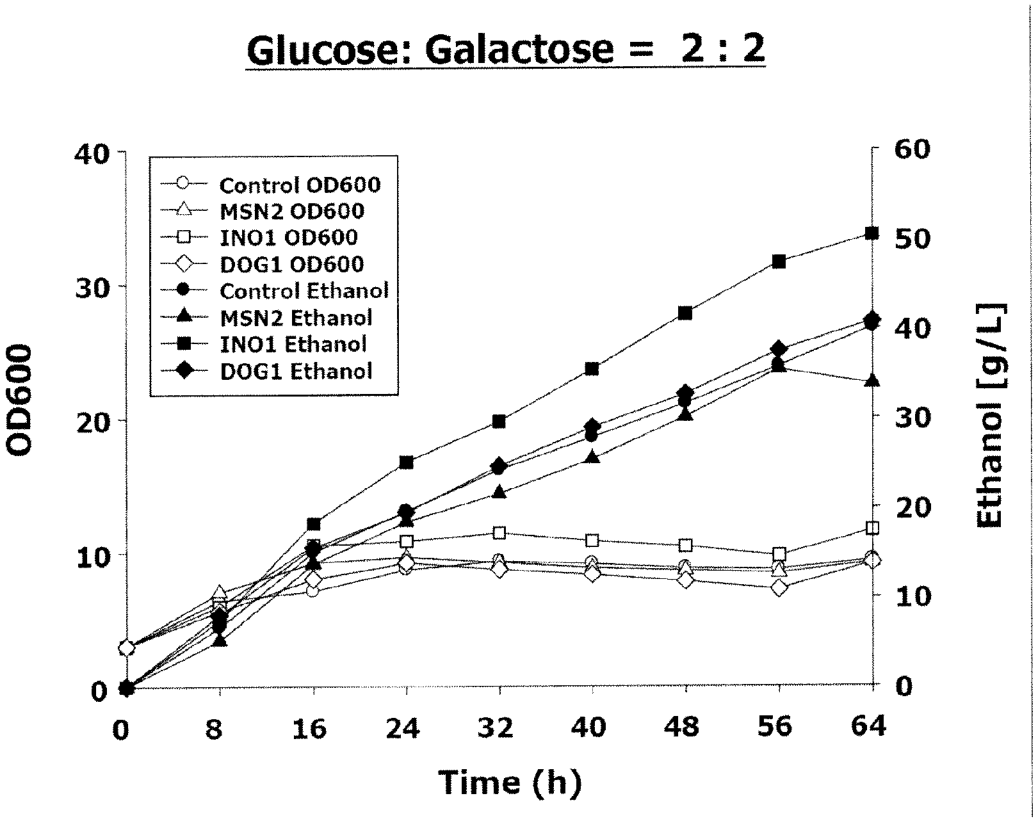
Glucose: Galactose = 2 : 2
OD600
Ethanol [g/L]
Time (h)
Control OD600
MSN2 OD600
INO1 OD600
DOG1 OD600
Control Ethanol
MSN2 Ethanol
INO1 Ethanol
DOG1 Ethanol
0
10
20
30
40
0
10
20
30
40
50
60
0
8
16
24
32
40
48
56
64

FIG. 29

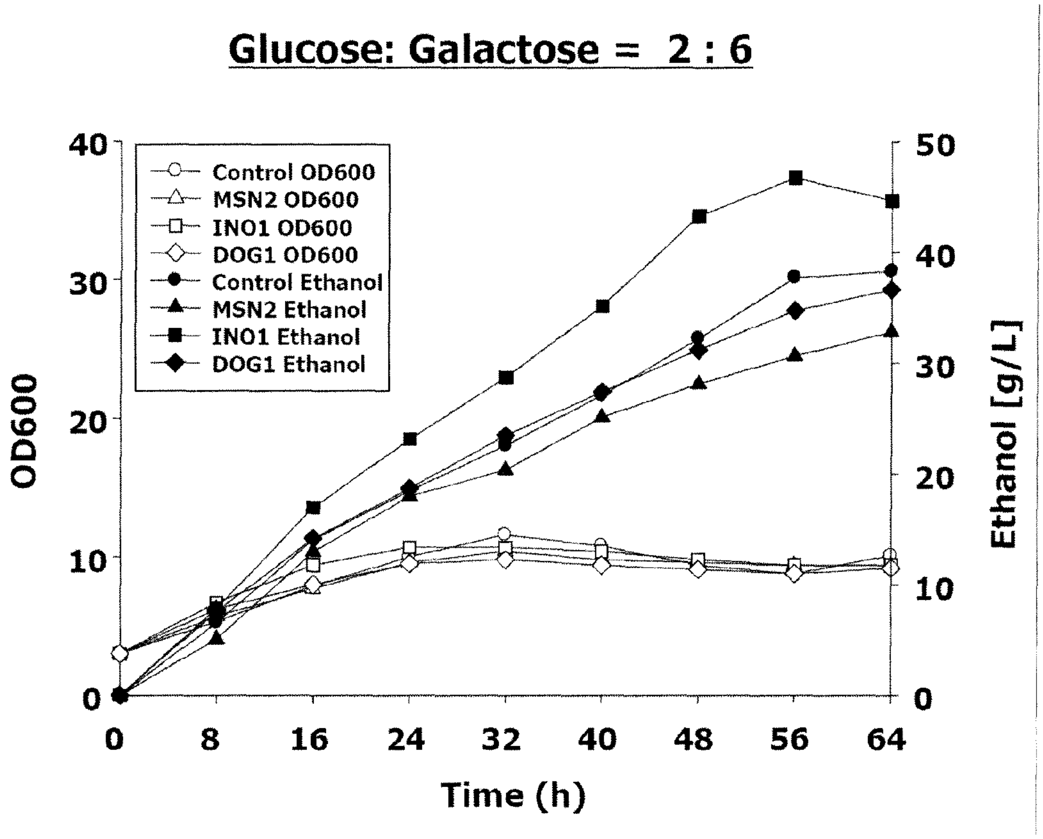
Glucose: Galactose = 2 : 6
Control OD600
MSN2 OD600
INO1 OD600
DOG1 OD600
Control Ethanol
MSN2 Ethanol
INO1 Ethanol
DOG1 Ethanol
OD600
Ethanol [g/L]
Time (h)
0
8
16
24
32
40
48
56
64
0
10
20
30
40
50

FIG. 30

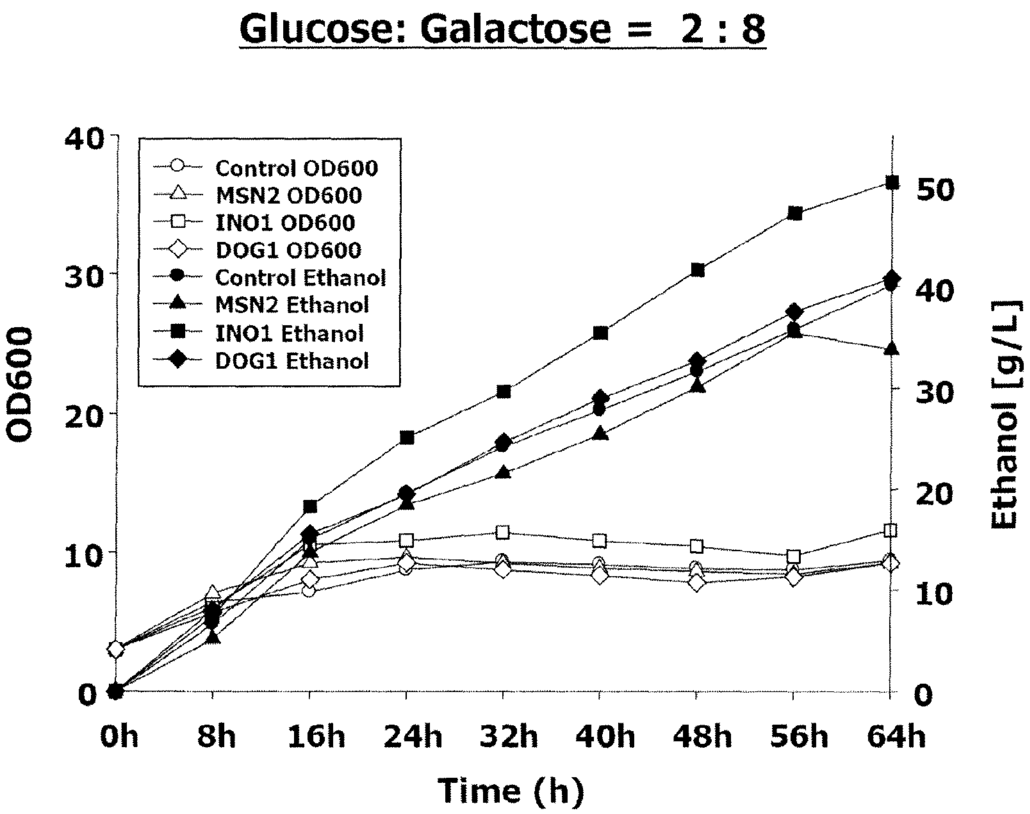
Glucose: Galactose = 2 : 8
Control OD600
MSN2 OD600
INO1 OD600
DOG1 OD600
Control Ethanol
MSN2 Ethanol
INO1 Ethanol
DOG1 Ethanol
OD600
Ethanol [g/L]
Time (h)
0h
8h
16h
24h
32h
40h
48h
56h
64h
0
10
20
30
40
50

FIG. 31

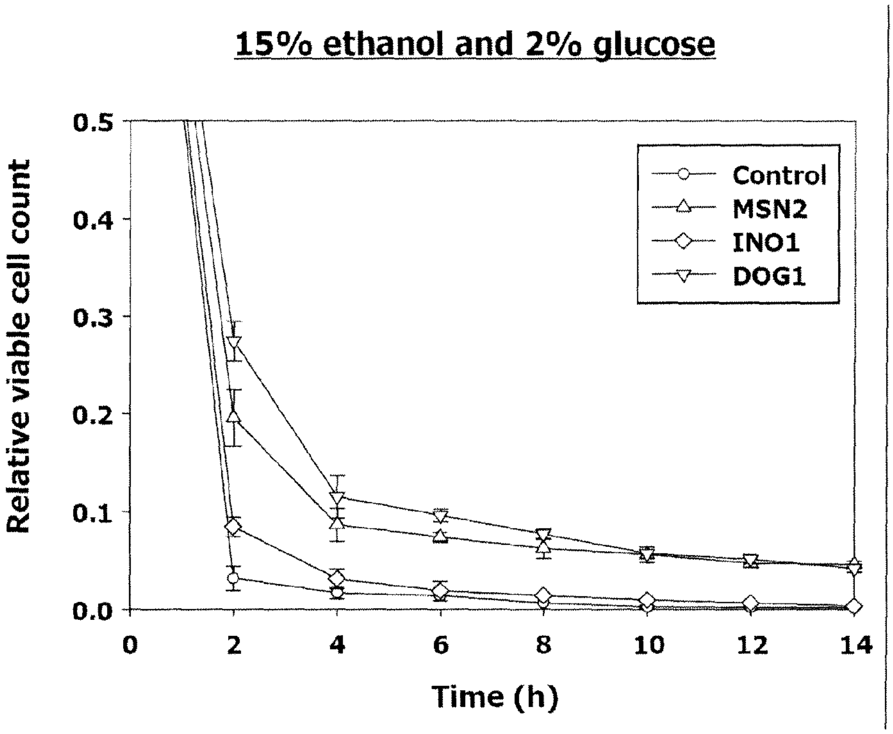
15% ethanol and 2% glucose
Relative viable cell count
0.5
0.4
0.3
0.2
0.1
0.0
0
2
4
6
8
10
12
14
Time (h)
Control
MSN2
INO1
DOG1

FIG. 32

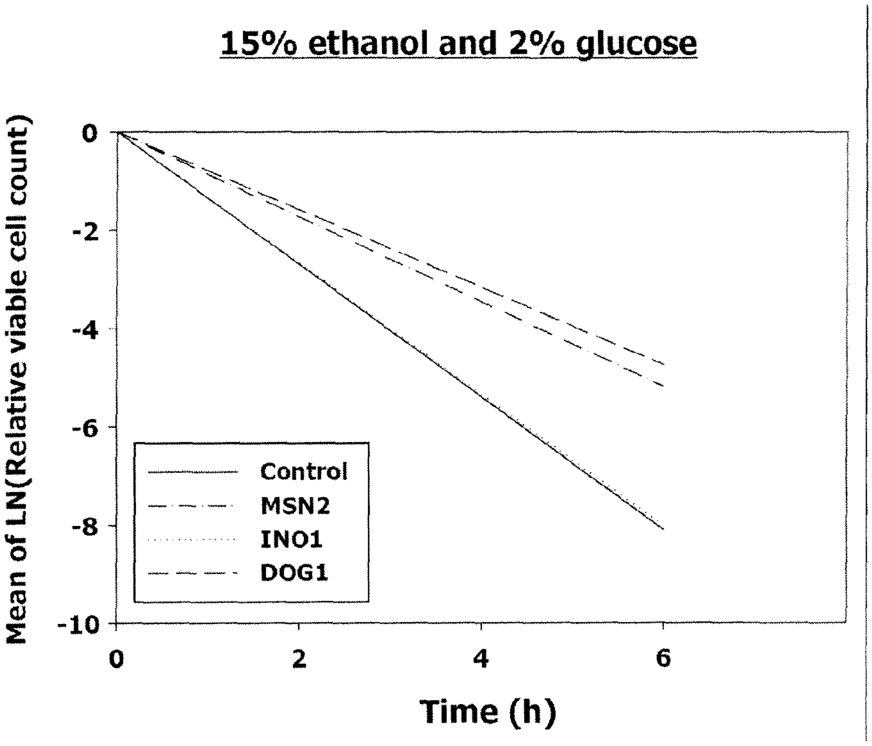
15% ethanol and 2% glucose
Mean of LN(Relative viable cell count)
0
-2
-4
-6
-8
-10
0
2
4
6
Time (h)
Control
MSN2
INO1
DOG1

Europäisches Patentamt
European Patent Office
Office européen des brevets

# EUROPEAN SEARCH REPORT

Application Number
EP 09 16 1944

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FUJITA KATSUHIDE ET AL: "The genome-wide screening of yeast deletion mutants to identify the genes required for tolerance to ethanol and other alcohols" FEMS YEAST RESEARCH, vol. 6, no. 5, August 2006 (2006-08), pages 744-750, XP002550324 ISSN: 1567-1356 * the whole document * ----- | 1-25 | INV. C07K14/395 |
| X | DATABASE EMBL [Online] 23 November 1989 (1989-11-23), "Saccharomyces cerevisiae mitotic inducer protein (MIH1) gene, complete cds." XP002550327 retrieved from EBI accession no. EMBL:J04846 Database accession no. J04846 * comprising a fragment 100% identical to SEQ ID NO:1 * | 1-25 | |
| A | -& RUSSELL P ET AL: "Conservation of mitotic controls in fission and budding yeasts" CELL, CELL PRESS, CAMBRIDGE, NA, US, vol. 57, no. 2, 21 April 1989 (1989-04-21), pages 295-303, XP024246192 ISSN: 0092-8674 [retrieved on 1989-04-21] ----- | 1-25 | TECHNICAL FIELDS SEARCHED (IPC) C07K |
| X | DATABASE EMBL [Online] 29 October 2006 (2006-10-29), "Synthetic construct Saccharomyces cerevisiae clone FLH238345.01X MIH1, complete sequence." XP002550328 retrieved from EBI accession no. EMBL:EF059121 Database accession no. EF059121 * comprising a fragment 100% identical to SEQ ID NO:1 * -/-- | 1-25 | |

~~The present search report has been drawn up for all claims~~

| Place of search | Date of completion of the search | Examiner |
|---|---|---|
| Munich | 11 February 2010 | Luo, Xinmei |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

7

EPO FORM 1503 03.82 (P04C01)

Europäisches Patentamt
European Patent Office
Office européen des brevets

# EUROPEAN SEARCH REPORT

Application Number
EP 09 16 1944

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | -& HU YANHUI ET AL: "Approaching a complete repository of sequence-verified protein-encoding clones for Saccharomyces cerevisiae" GENOME RESEARCH, vol. 17, no. 4, April 2007 (2007-04), pages 536-543, XP002550325 ISSN: 1088-9051 ----- | 1-25 | |
| X | DATABASE EMBL [Online] 26 March 2008 (2008-03-26), "Method for identification of useful proteins derived from brewery yeast." XP002550329 retrieved from EBI accession no. EMBL:DJ207335 Database accession no. DJ207335 * comprising a fragment 99.5% identical to SEQ ID NO:1 * ----- | 1-25 | |
| X | DATABASE EMBL [Online] 8 December 2006 (2006-12-08), "Saccharomyces cerevisiae mRNA, clone: S03052-75_M15, 5'-end sequence." XP002550330 retrieved from EBI accession no. EMBL:DB650171 Database accession no. DB650171 * comprising a fragment 99.5% identical to SEQ ID NO:1 * | 1-25 | TECHNICAL FIELDS SEARCHED (IPC) |
| A | -& MIURA FUMIHITO ET AL: "A large-scale full-length cDNA analysis to explore the budding yeast transcriptome" PROCEEDINGS OF THE NATIONAL ACADEMY OF SCIENCES OF THE UNITED STATES OF AMERICA, vol. 103, no. 47, November 2006 (2006-11), pages 17846-17851, XP002550326 ISSN: 0027-8424 ----- -/-- | 1-25 | |

~~The present search report has been drawn up for all claims~~

| Place of search | Date of completion of the search | Examiner |
|---|---|---|
| Munich | 11 February 2010 | Luo, Xinmei |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

7

EPO FORM 1503 03.82 (P04C01)

Europäisches Patentamt
European Patent Office
Office européen des brevets

# EUROPEAN SEARCH REPORT

Application Number
EP 09 16 1944

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE EMBL [Online] 28 January 1995 (1995-01-28), "EST103570 S. cerevisiae strain X2180-1A Saccharomyces cerevisiae cDNA 3' end, mRNA sequence." XP002550331 retrieved from EBI accession no. EMBL:T38177 Database accession no. T38177 * comprising a fragment 99.5% identical to SEQ ID NO:1 * | 1-25 | |
| X | US 2003/064432 A1 (ROZZELL J DAVID [US] ET AL ROZZELL JR J DAVID [US] ET AL) 3 April 2003 (2003-04-03) * SEQ ID NO:72 is 100% identical to SEQ ID NO:2 of the application * | 1-25 | |
| X | WO 01/55342 A2 (BIOCATALYTICS INC [US]) 2 August 2001 (2001-08-02) * SEQ ID NO:72 is 100% identical to SEQ ID NO:2 of the application * | 1-25 | TECHNICAL FIELDS SEARCHED (IPC) |
| X | WO 2009/029644 A1 (CORNELL RES FOUNDATION INC [US]; VILLA-GARCIA MANUEL J [US]; KRAUSE ER) 5 March 2009 (2009-03-05) * SEQ ID NO:1 (pages 7-8) is 100% identical to SEQ ID NO:2 of the application * | 1-25 | |

-/--

~~The present search report has been drawn up for all claims~~

| Place of search | Date of completion of the search | Examiner |
|---|---|---|
| Munich | 11 February 2010 | Luo, Xinmei |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

7

EPO FORM 1503 03.82 (P04C01)

Europäisches Patentamt
European Patent Office
Office européen des brevets

# EUROPEAN SEARCH REPORT

Application Number
EP 09 16 1944

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE EMBL [Online]<br>6 October 1995 (1995-10-06), "S.cerevisiae chromosome X reading frame ORF YJL153c"<br>XP002567955<br>retrieved from EBI accession no. EMBL:Z49428<br>Database accession no. Z49428<br>* this sequence comprises a fragment which is 100% identical to SEQ ID NO:2 of the applicationcompound *<br>----- | 1-25 | |
| X | DATABASE EMBL [Online]<br>29 October 2006 (2006-10-29), "Synthetic construct Saccharomyces cerevisiae clone FLH200985.01X INO1, complete sequence."<br>XP002567956<br>retrieved from EBI accession no. EMBL:EF059009<br>Database accession no. EF059009<br>* this sequence is 100% identical to SEQ ID NO:2 of the application *<br>----- | 1-25 | TECHNICAL FIELDS SEARCHED (IPC) |

~~The present search report has been drawn up for all claims~~

| Place of search | Date of completion of the search | Examiner |
|---|---|---|
| Munich | 11 February 2010 | Luo, Xinmei |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........
& : member of the same patent family, corresponding document

7

EPO FORM 1503 03.82 (P04C01)

Europäisches Patentamt
European Patent Office
Office européen des brevets

Application Number
EP 09 16 1944

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☒ Only part of the further search fees have been paid within the fixed time limit. The present European search report has been drawn up for those parts of the European patent application which relate to the inventions in respect of which search fees have been paid, namely claims:

1-25(all partially)

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches Patentamt
European Patent Office
Office européen des brevets

**LACK OF UNITY OF INVENTION**
**SHEET B**

Application Number
EP 09 16 1944

The Search Division considers that the present European patent application does not comply with the requirements of unity of invention and relates to several inventions or groups of inventions, namely:

1. claims: 1-25(all partially)

relates to an islolated polynucleotide comprising a polynucleotide consisting of a base sequence having at least 90% identity to a base sequence according to SEQ ID NO:1;

---

2-17. claims: 1-25(all partially)

relates to an islolated polynucleotide comprising a polynucleotide consisting of a base sequence having at least 90% identity to a base sequence according to SEQ ID NO:2-11,26,27,28-31, respectively.

---

## ANNEX TO THE EUROPEAN SEARCH REPORT ON EUROPEAN PATENT APPLICATION NO. EP 09 16 1944

This annex lists the patent family members relating to the patent documents cited in the above-mentioned European search report.
The members are as contained in the European Patent Office EDP file on
The European Patent Office is in no way liable for these particulars which are merely given for the purpose of information.

11-02-2010

| Patent document cited in search report | Publication date | Patent family member(s) | Publication date |
|---|---|---|---|
| US 2003064432 A1 | 03-04-2003 | US 6366860 B1 | 02-04-2002 |
| WO 0155342 A2 | 02-08-2001 | EP 1189921 A2 | 27-03-2002 |
| | | US 2005196774 A1 | 08-09-2005 |
| WO 2009029644 A1 | 05-03-2009 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0472869 A
- US 4601893 A
- WO 9615246 A


### Non-patent literature cited in the description


- **Devereux J. et al.** *Nucleic Acids Res,* 1984, vol. 12, 387-395
- **Altschul S. F. et al.** *Nucleic Acids Res.,* 1997, vol. 25, 389-3402
- **Pearson W.R.** *Methods in Enzymology,* 1990, vol. 183, 63-99
- **Huh WK et al.** Global analysis of protein localization in budding yeast. *Mature,* 2003, vol. 425 (6959), 686-91
- **Marquez JA.** The Ssn6-Tup1 repressor complex of Saccharomyces cerevisiae is involved in the osmotic induction of HOG-dependent and -independent genes. *EMBO J,* 1998, vol. 17 (9), 2543-53
- **Pascual-Ahuir A et al.** The Sko1p repressor and Gcn4p activator antagonistically modulate stress-regulated transcription in Saccharomyces cerevisiae. *Mol Cell Biol,* 2001, vol. 21 (1), 16-25
- **Kim JM et al.** Transposable elements and genome organization: a comprehensive survey of retrotransposons revealed by the complete Saccharomyces cerevisiae genome sequence. *Genome Res,* 1998, vol. 8 (5), 464-78
- **Eikmanns et al.** *Gene,* 1991, vol. 102, 93-98
- **LaBarre et al.** *Journal of Bacteriology,* 1993, vol. 175, 1001-1007
- **Sambrook et al.** Molecular Cloning: A Laboratory Manual. Cold Spring Harbor Laboratory Press, 1989
- **Ito, H. ; Y. Fukuoka ; K. Murata ; A. Kimura.** Transformation of intact yeast cells treated with alkali cations. *J. Bacteriol.,* 1983, vol. 153, 163-168
- Engineering Yeast Transcription Machinery for Improved Ethanol Tolerance and Production. **Hal Alper et al.** Science. 08 December 2006, vol. 314, 1565